# EUROPEAN PATENT APPLICATION

(11) **EP 4 837 965 A1**
(43) Date of publication of application: **11.11.2026**
(21) Application number: 25752250.8
(22) Date of filing: 07.02.2025
(51) Int. Cl.: C12N 15/113, A61K 31/7105, A61K 31/712, A61K 31/7125, A61K 31/713, A61M 5/14, A61M 25/06, A61P 25/28

(54) **OLIGONUCLEOTIDE**

(30) Priority: 08.02.2024 JP 2024017851
(71) Applicant: Terumo Kabushiki Kaisha, Tokyo 151-0072 (JP)
(72) Inventor: ARINOBE, Manabu, Ashigarakami-gun, Kanagawa 259-0151 (JP); SATO, Eri, Ashigarakami-gun, Kanagawa 259-0151 (JP); NAKAOKA, Mayu, Ashigarakami-gun, Kanagawa 259-0151 (JP); IWASE, Yoichiro, Ashigarakami-gun, Kanagawa 259-0151 (JP); KODA, Yasuo, Tokyo 100-0004 (JP); HARUMOTO, Toshimasa, Tokyo 100-0004 (JP); YAMAMOTO, Junichiro, Tokyo 100-0004 (JP); SHIRAISHI, Yasuhisa, Tokyo 100-0004 (JP)
(74) Representative: Prüfer & Partner mbB Patentanwälte · Rechtsanwälte
(86) International application number: PCT/JP2025/004074
(87) International publication number: WO 2025/170027

(57) **Abstract**

An oligonucleotide administered intranasally into the brain of a mammal is provided.

The oligonucleotide is administered intranasally into the brain of a mammal by being injected from within a cribriform foramen of a cribriform plate. Further, the oligonucleotide may be administered intranasally into the brain of a mammal using an administration device including a needle portion having a puncture portion, and may be administered through an opening portion of the puncture portion in a state where the puncture portion is disposed within a cribriform foramen of the cribriform plate.

## Description

### Technical Field

The present invention relates to an oligonucleotide that is administered intranasally into the brain of a mammal using an administration device.

### Background Art

Since central nervous system (CNS) diseases are often caused by pathogenic proteins, methods that approach the pathogenic proteins themselves or genes involved in expression of such proteins are considered effective for treatment, prevention, or improvement thereof. For example, a nucleic acid drug has long been expected to be applicable to the treatment of a CNS disease because it can control the expression of a protein through controlling the expression of a specific gene.

On the other hand, in the brain, transport of substances between circulating blood and brain tissue is strictly controlled by the blood-brain barrier (BBB), and when drugs (such as nucleic acids, proteins, and antibodies) are administered orally or intravenously, the amount of drug delivered to the brain is extremely limited.

Accordingly, for the purpose of administering a drug into the brain more effectively than oral administration or intravenous administration, research has been conducted on administration methods that bypass the blood-brain barrier and can directly access the central nervous system. At present, methods such as an intranasal spray method in which a drug is sprayed into the nasal cavity, an intraventricular administration method in which a drug is directly administered into a cerebral ventricle, and an intrathecal administration method in which a drug is injected into the spinal canal are known (Biopharm. Drug Dispos. 44(1), 26-47 (2023)).

### Summary of Invention

### Technical Problem

However, although the intranasal spray method is minimally invasive, there is a problem that the drug transfer rate is extremely low. In addition, in the intraventricular administration method, because the skull is perforated and the drug is directly administered into a cerebral ventricle, there is a risk of injury caused by cerebral puncture. The intrathecal administration method is generally used as a method for administering a nucleic acid drug for treating a central nervous system disease, but has many problems, including insufficient drug delivery to deep brain regions such as the striatum, high invasiveness, and difficulty in administering the drug itself to patients having scoliosis, which is common in intractable neurological diseases.

The present invention has been made to solve the above problems, and an object thereof is to provide an oligonucleotide that, in treatment of a central nervous system disease using a nucleic acid drug, is minimally invasive, can be efficiently administered into the brain, and provides high efficacy.

### Solution to Problem

The present inventors conducted intensive research to solve the above problems. As a result, the present inventors found that the above problems are solved by an oligonucleotide administered intranasally using a predetermined administration device, and have completed the present invention.

That is, the above object can be achieved by the present invention having the following configuration, and the present invention includes the following aspects and forms.

According to one aspect of the present invention,
1. there is provided an oligonucleotide that is administered intranasally into the brain of a mammal by being injected from within a cribriform foramen of a cribriform plate.
2. The oligonucleotide according to the above 1. is preferably
   administered intranasally into the brain of a mammal using an administration device including a needle portion having a puncture portion, and
   injected through an opening portion of the puncture portion in a state where the puncture portion is disposed within a cribriform foramen of the cribriform plate.
3. The oligonucleotide according to the above 1. or 2. is preferably any one selected from the group consisting of an aptamer, an antisense oligonucleotide, a decoy nucleic acid, a ribozyme, siRNA, miRNA, and mRNA.
4. The oligonucleotide according to any one of the above 1. to 3. is preferably siRNA or antisense oligonucleotide.
5. The oligonucleotide according to any one of the above 1. to 4. is preferably siRNA.
6. In the oligonucleotide according to the above 5., the proportion of 2'-modified nucleotides among nucleotides constituting the siRNA is preferably 80% or more.
7. In the oligonucleotide according to the above 5. or 6., all nucleotides constituting the siRNA are preferably 2'-modified nucleotides.
8. The oligonucleotide according to any one of the above 5. to 7. preferably has one or more lipophilic moieties.
9. In the oligonucleotide according to the above 8., the lipophilic moiety is preferably at least one selected from the group consisting of a substituted or unsubstituted alkyl chain having 14 to 24 carbon atoms, eicosapentaenoic acid (EPA), arachidonic acid (ARA), and cholic acid.
10. In the oligonucleotide according to any one of the above 5. to 9., a target gene of the siRNA is preferably SNCA or HTT.
11. In the oligonucleotide according to any one of the above 5. to 10., all phosphodiester bonds linking the first and second nucleotides counted from both ends of the sense strand and antisense strand of the siRNA are preferably replaced with phosphorothioate bonds.
12. In the oligonucleotide according to the above 5. to 11., the siRNA preferably includes any one pair of nucleic acid sequences selected from the group consisting of (S1) to (S11) below.
   (S1) SEQ ID NO: 46 (sense strand) and SEQ ID NO: 213 (antisense strand)
   (S2) SEQ ID NO: 83 (sense strand) and SEQ ID NO: 250 (antisense strand)
   (S3) SEQ ID NO: 93 (sense strand) and SEQ ID NO: 260 (antisense strand)
   (S4) SEQ ID NO: 169 (sense strand) and SEQ ID NO: 336 (antisense strand)
   (S5) SEQ ID NO: 180 (sense strand) and SEQ ID NO: 347 (antisense strand)
   (S6) SEQ ID NO: 1360 (sense strand) and SEQ ID NO: 1389 (antisense strand)
   (S7) SEQ ID NO: 1366 (sense strand) and SEQ ID NO: 1395 (antisense strand)
   (S8) SEQ ID NO: 1368 (sense strand) and SEQ ID NO: 1397 (antisense strand)
   (S9) SEQ ID NO: 1372 (sense strand) and SEQ ID NO: 1401 (antisense strand)
   (S10) SEQ ID NO: 1374 (sense strand) and SEQ ID NO: 1403 (antisense strand)
   (S11) SEQ ID NO: 1756 (sense strand) and SEQ ID NO: 1759 (antisense strand)
13. In the oligonucleotide according to the above 5., the siRNA preferably includes the sequence of any one pair of oligonucleotides selected from the group consisting of ds2, ds9, ds17 to ds249, and ds254 to ds273 described in any of Tables 1-1 to 1-6.

In addition, in the tables, "[L1]" represents the compound represented by Formula (1) below, "^" represents a phosphorothioate bond, "mA", "mU", "mC", and "mG" represent 2'-O-methyl RNA (that is, "mA" represents 2'-O-methyladenosine, "mU" represents 2'-O-methyluridine, "mC" represents 2'-O-methylcytidine, and "mG" represents 2'-O-methylguanosine), "fA", "fU", "fC", and "fG" represent 2'-fluoro-DNA (that is, "fA" represents 2'-fluoro-2'-deoxyadenosine, "fU" represents 2'-fluoro-2'-deoxyuridine, "fC" represents 2'-fluoro-2'-deoxycytidine, and "fG" represents 2'-fluoro-2'-deoxyguanosine), "dA", "dT", "dC", and "dG" represent DNA (that is, "dA" represents deoxyadenosine, "dT" represents deoxythymidine, "dC" represents deoxycytidine, and "dG" represents deoxyguanosine), "rA", "rU", "rC", and "rG" represent RNA (that is, "rA" represents adenosine, "rU" represents uridine, "rC" represents cytidine, and "rG" represents guanosine), "(vmU)" represents 5'-vinylphosphate-2'-O-methyl-uridine, and "p" represents 5'-phosphate.

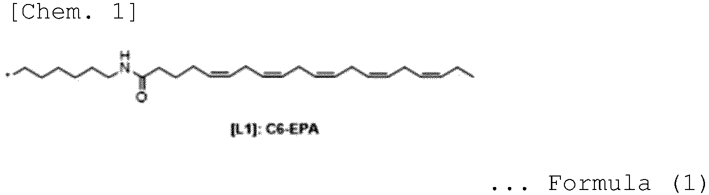

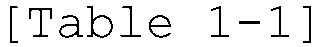

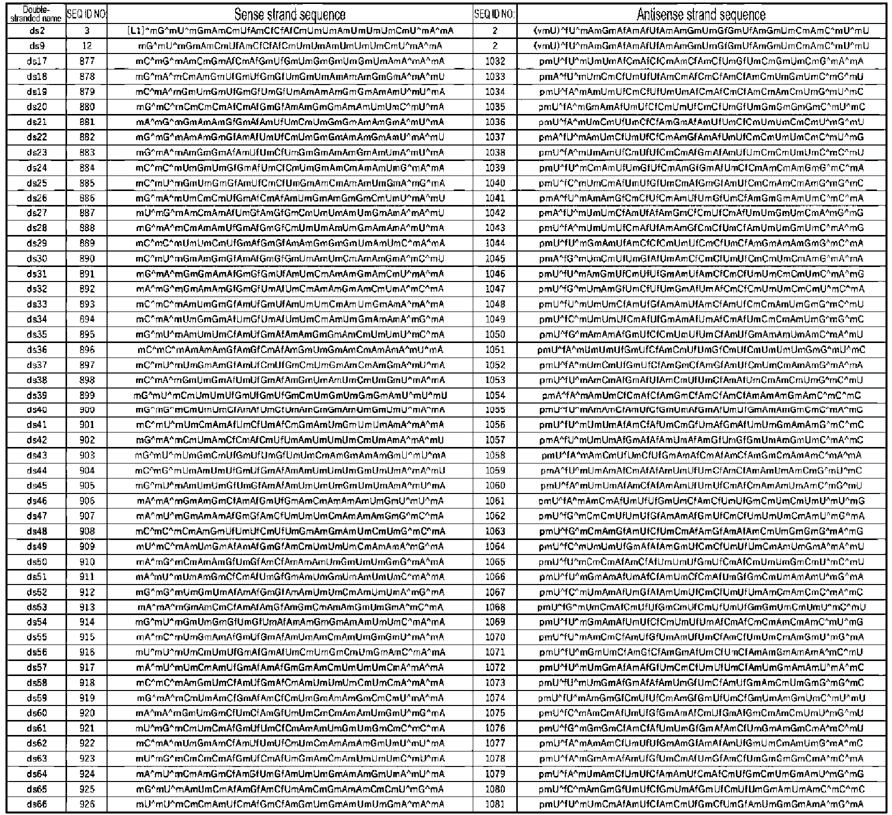

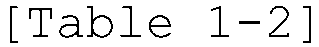

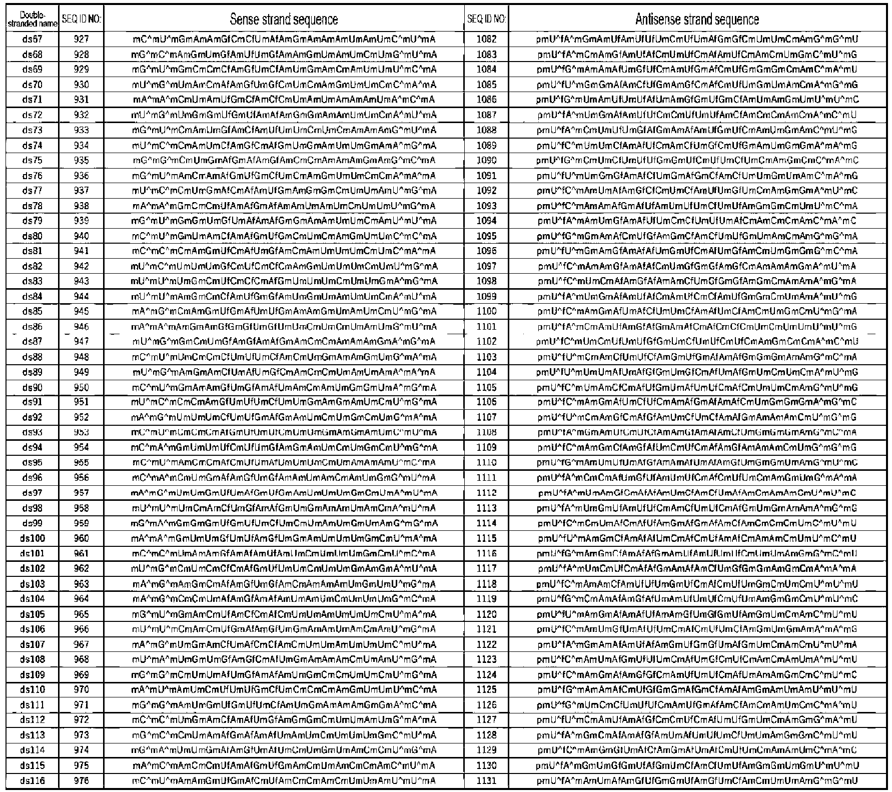

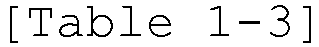

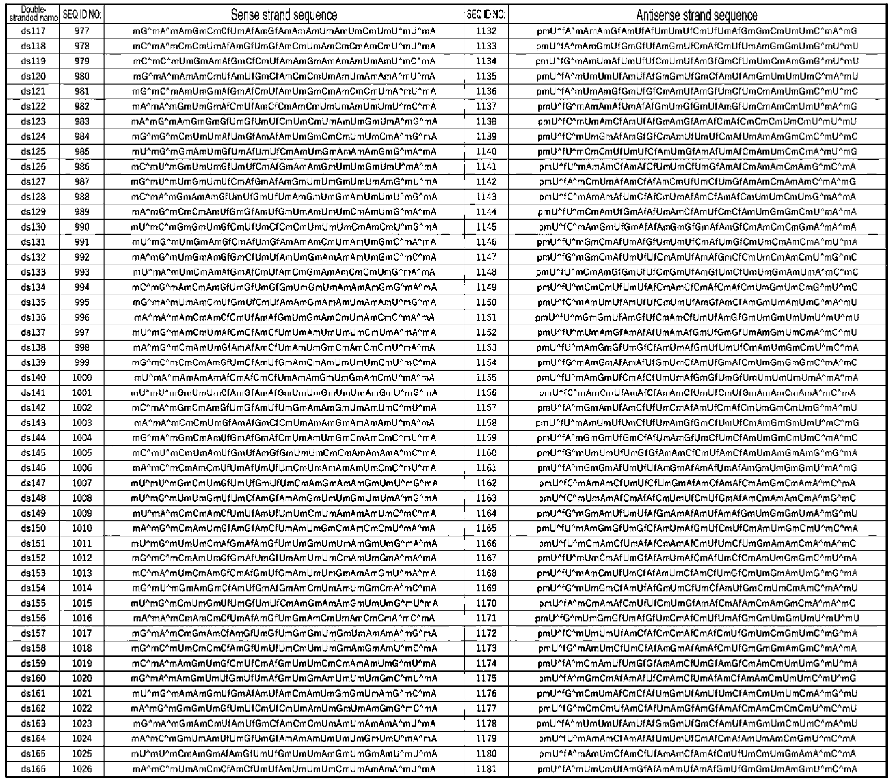

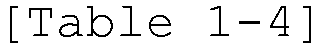

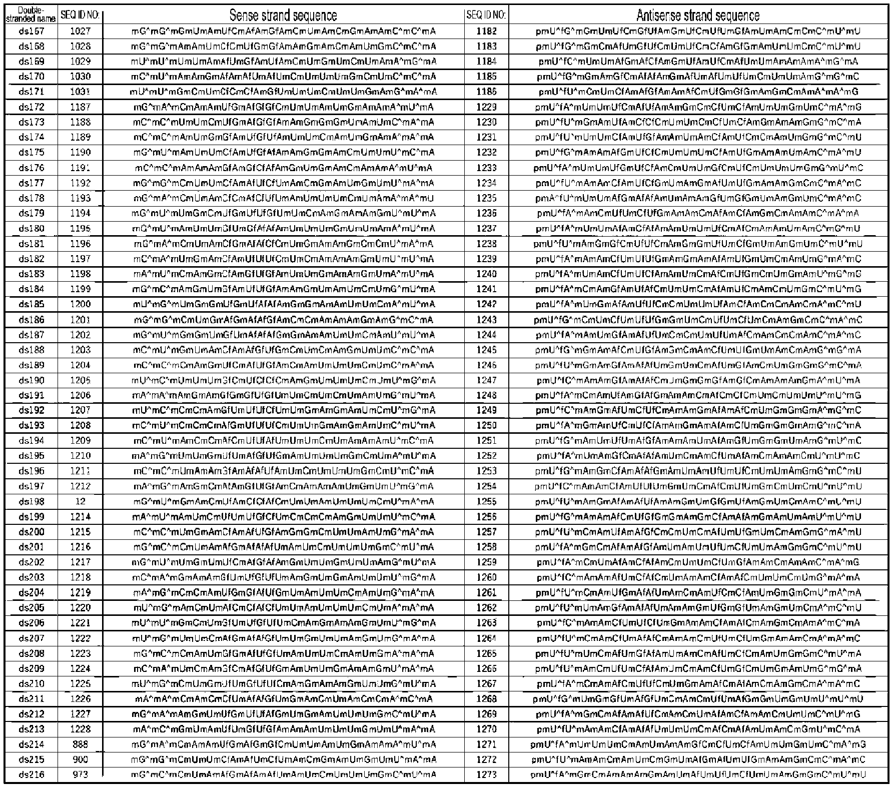

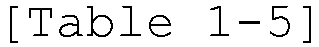

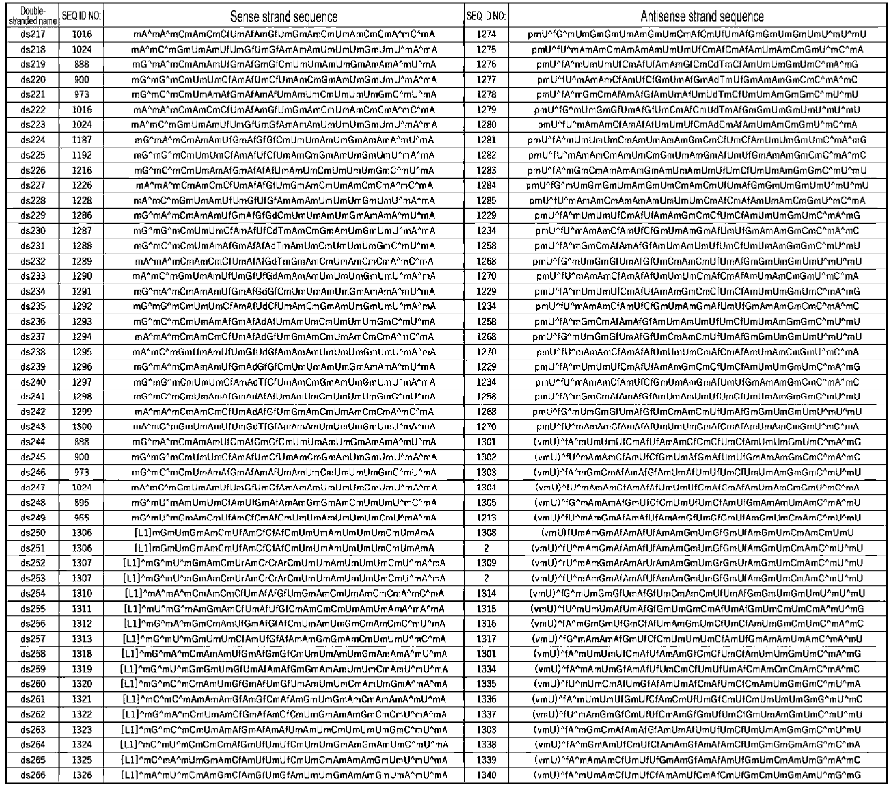

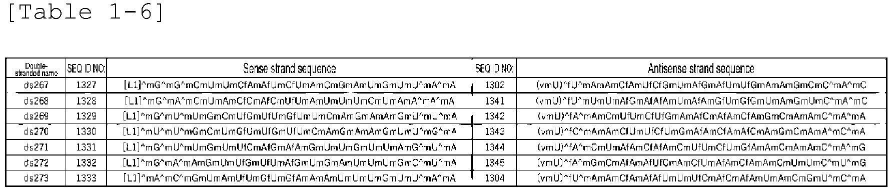

14. In the oligonucleotide according to the above 13., the siRNA preferably includes a sequence of any one pair of oligonucleotides selected from the group consisting of the ds9, the ds28, the ds35, the ds36, the ds40, the ds42, the ds79, the ds105, the ds113, the ds156, the ds164, the ds172, the ds175, the ds176, the ds177, the ds178, the ds187, the ds198, the ds201, the ds211, and the ds213 to the ds246 described in any of the Tables 1-1 to 1-6.

15. In the oligonucleotide according to the above 13., the siRNA is preferably any one pair of oligonucleotides selected from the group consisting of the ds2, the ds254, the ds257, the ds258, the ds259, the ds261, the ds263, the ds267, the ds268, and the ds273 described in any of Tables 1-1 to 1-6.

16. In the oligonucleotide according to the above 5., the siRNA preferably includes the sequence of any one pair of oligonucleotides selected from the group consisting of ds274 to ds461 described in any of Tables 2-1 to 2-4.

In addition, in the tables, "^" represents a phosphorothioate bond, "mA", "mU", "mC", and "mG" represent 2'-O-methyl RNA (that is, "mA" represents 2'-O-methyladenosine, "mU" represents 2'-O-methyluridine, "mC" represents 2'-O-methylcytidine, and "mG" represents 2'-O-methylguanosine), "fA", "fU", "fC", and "fG" represent 2'-fluoro-DNA (that is, "fA" represents 2'-fluoro-2'-deoxyadenosine, "fU" represents 2'-fluoro-2'-deoxyuridine, "fC" represents 2'-fluoro-2'-deoxycytidine, and "fG" represents 2'-fluoro-2'-deoxyguanosine), "dA", "dT", "dC", and "dG" represent DNA (that is, "dA" represents deoxyadenosine, "dT" represents deoxythymidine, "dC" represents deoxycytidine, and "dG" represents deoxyguanosine), and "p" represents 5'-phosphate.

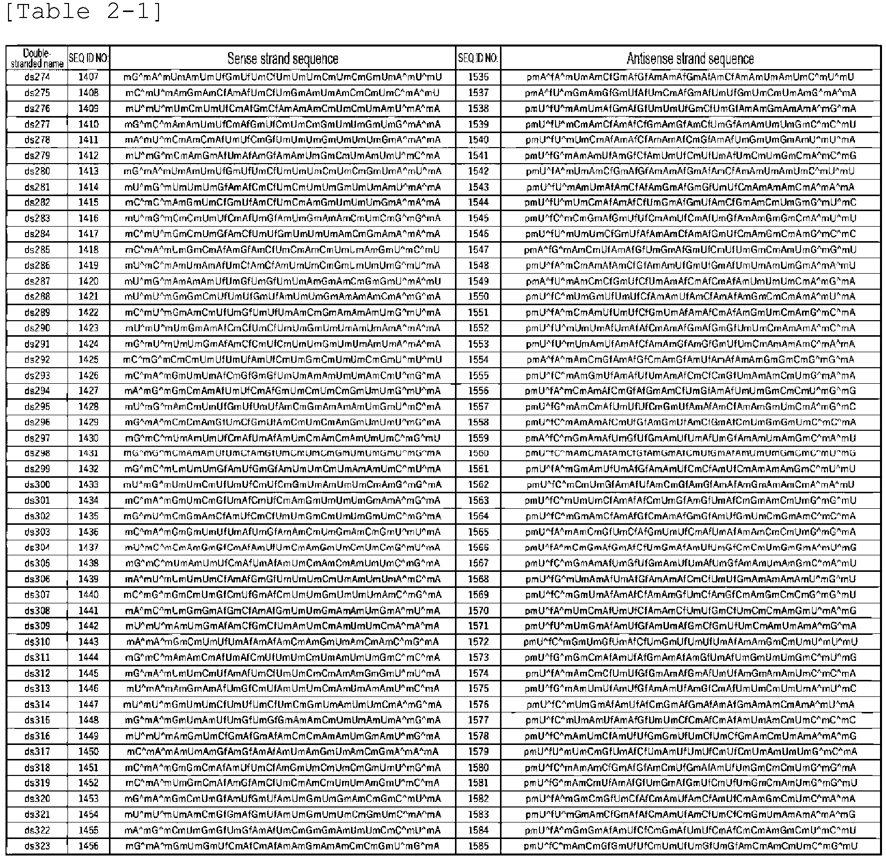

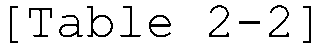

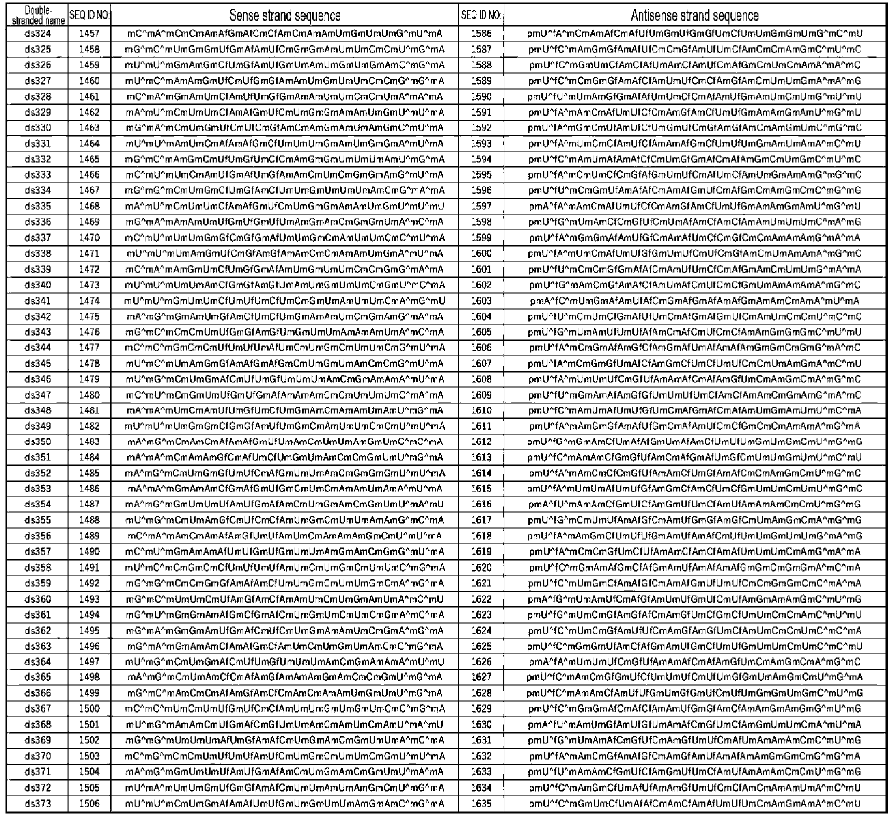

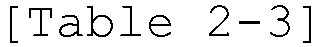

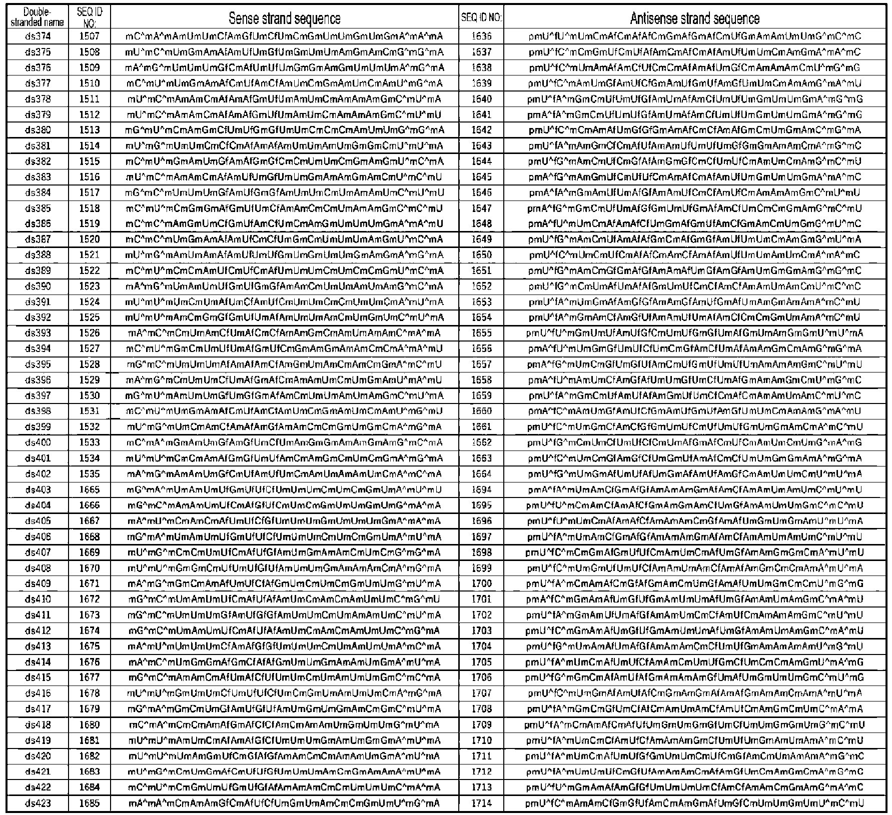

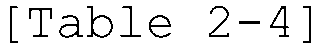

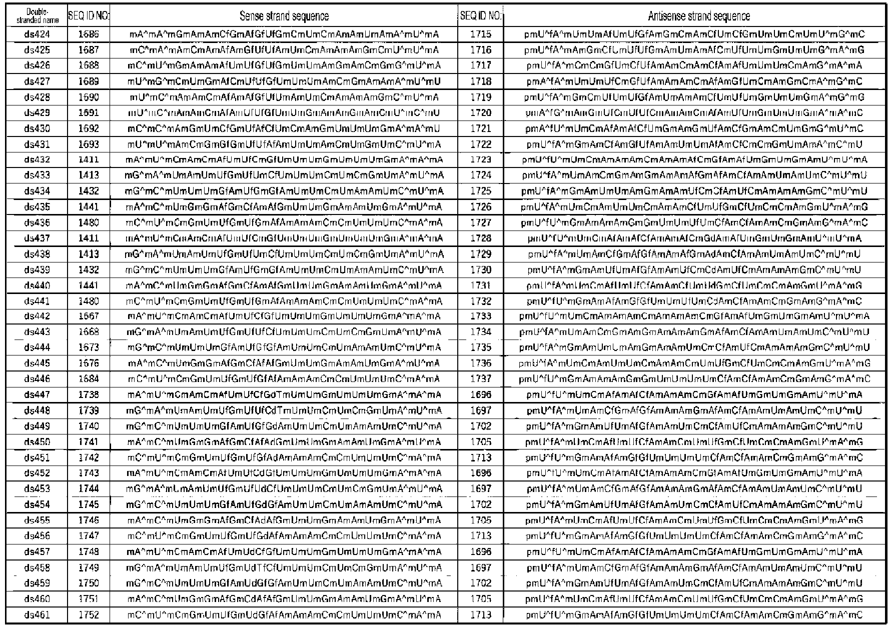

17. In the oligonucleotide according to the above 16., the siRNA preferably includes a sequence of any one pair of oligonucleotides selected from the group consisting of the ds278, the ds280, the ds299, the ds308, the ds347, the ds405, the ds406, the ds411, the ds414, the ds422, and the ds432 to ds461 described in any of the Tables 2-1 to 2-4.

According to one aspect of the present invention,
18. there is provided a pharmaceutical composition containing the oligonucleotide according to any one of the above 1. to 17.

According to one aspect of the present invention,
19. there is provided a therapeutic agent for a central nervous system disease containing the oligonucleotide according to any one of the above 1. to 17.
20. In the oligonucleotide according to the above 2.,
   the administration device preferably has a cannula portion that is formed of a tubular member and is disposed to cover the needle portion such that the puncture portion is exposed,
   the opening portion is preferably provided on a distal end side of the puncture portion,
   a stopper portion having an abutment portion that abuts against an olfactory epithelium of an olfactory mucosa is preferably formed at a distal end portion of the cannula portion, and
   the opening portion is preferably disposed within the cribriform plate in a state where the abutment portion of the stopper portion is brought into contact with the olfactory epithelium.
21. In the oligonucleotide according to the above 20.,
   the administration device preferably has a hub portion to which an accommodation portion accommodating the oligonucleotide is attachable, and
   the hub portion preferably holds a proximal end of the cannula portion and/or a proximal end of a needle shaft portion of the needle portion inserted through a lumen of the cannula portion.
22. In the oligonucleotide according to the above 20. or 21., a cross-sectional shape of the abutment portion of the stopper portion is preferably circular or elliptical.

According to one aspect of the present invention,
23. there is provided an administration system including an accommodation portion accommodating the oligonucleotide according to any one of the above 1. to 17. and an administration device including a needle portion having a puncture portion, in which the administration system is configured such that the oligonucleotide accommodated in the accommodation portion is dischargeable through an opening portion of the puncture portion.
24. In the administration system according to the above 23., the administration device preferably has a cannula portion that is formed of a tubular member and is disposed to cover the needle portion such that the puncture portion is exposed,
   the opening portion is preferably provided on a distal end side of the puncture portion,
   a stopper portion having an abutment portion that abuts against an olfactory epithelium of an olfactory mucosa is preferably formed at a distal end portion of the cannula portion, and
   the opening portion is preferably disposed within the cribriform plate in a state where the abutment portion of the stopper portion is brought into contact with the olfactory epithelium.
25. In the administration system according to the above 24., the administration device preferably has a hub portion to which an accommodation portion accommodating the oligonucleotide is attachable, and
   the hub portion preferably holds a proximal end of the cannula portion and/or a proximal end of a needle shaft portion of the needle portion inserted through a lumen of the cannula portion.
26. In the administration system according to the above 24. or 25., a cross-sectional shape of the abutment portion of the stopper portion is preferably circular or elliptical.

In addition, the above-described objects can also be achieved by the present invention having the following configurations, and the present invention also encompasses the following aspects and embodiments.

According to another aspect of the present invention,
1. there is provided an oligonucleotide for use in the treatment of a central nervous system disease, in which the oligonucleotide is administered intranasally into the brain of a mammal by being injected from within a cribriform foramen of a cribriform plate.
2. The oligonucleotide according to the above 1. is preferably
   administered intranasally into the brain of a mammal using an administration device including a needle portion having a puncture portion, and
   injected through an opening portion of the puncture portion in a state where the puncture portion is disposed within a cribriform foramen of the cribriform plate.
3. The oligonucleotide according to the above 1. or 2. is preferably any one selected from the group consisting of an aptamer, an antisense oligonucleotide, a decoy nucleic acid, a ribozyme, siRNA, miRNA, and mRNA.
4. The oligonucleotide according to any one of the above 1. to 3. is preferably siRNA or antisense oligonucleotide.
5. The oligonucleotide according to any one of the above 1. to 4. is preferably siRNA.
6. In the oligonucleotide according to the above 5., the proportion of 2'-modified nucleotides among nucleotides constituting the siRNA is preferably 80% or more.
7. In the oligonucleotide according to the above 5. or 6., all nucleotides constituting the siRNA are preferably 2'-modified nucleotides.
8. The oligonucleotide according to any one of the above 5. to 7. preferably has one or more lipophilic moieties.
9. In the oligonucleotide according to the above 8., the lipophilic moiety is preferably at least one selected from the group consisting of a substituted or unsubstituted alkyl chain having 14 to 24 carbon atoms, eicosapentaenoic acid (EPA), arachidonic acid (ARA), and cholic acid.
10. In the oligonucleotide according to any one of the above 5. to 9., a target gene of the siRNA is preferably SNCA or HTT.
11. In the oligonucleotide according to any one of the above 5. to 10., all phosphodiester bonds linking the first and second nucleotides counted from both ends of the sense strand and antisense strand of the siRNA are preferably replaced with phosphorothioate bonds.
12. In the oligonucleotide according to any one of the above 5. to 11., the siRNA preferably includes any one pair of nucleic acid sequences selected from the group consisting of (S1) to (S11) below.
   (S1) SEQ ID NO: 46 (sense strand) and SEQ ID NO: 213 (antisense strand)
   (S2) SEQ ID NO: 83 (sense strand) and SEQ ID NO: 250 (antisense strand)
   (S3) SEQ ID NO: 93 (sense strand) and SEQ ID NO: 260 (antisense strand)
   (S4) SEQ ID NO: 169 (sense strand) and SEQ ID NO: 336 (antisense strand)
   (S5) SEQ ID NO: 180 (sense strand) and SEQ ID NO: 347 (antisense strand)
   (S6) SEQ ID NO: 1360 (sense strand) and SEQ ID NO: 1389 (antisense strand)
   (S7) SEQ ID NO: 1366 (sense strand) and SEQ ID NO: 1395 (antisense strand)
   (S8) SEQ ID NO: 1368 (sense strand) and SEQ ID NO: 1397 (antisense strand)
   (S9) SEQ ID NO: 1372 (sense strand) and SEQ ID NO: 1401 (antisense strand)
   (S10) SEQ ID NO: 1374 (sense strand) and SEQ ID NO: 1403 (antisense strand)
   (S11) SEQ ID NO: 1756 (sense strand) and SEQ ID NO: 1759 (antisense strand)
13. In the oligonucleotide according to the above 5., the siRNA preferably includes the sequence of any one pair of oligonucleotides selected from the group consisting of ds2, ds9, ds17 to ds249, and ds254 to ds273 described in any of Tables 1-1 to 1-6.
14. In the oligonucleotide according to the above 13., the siRNA preferably includes a sequence of any one pair of oligonucleotides selected from the group consisting of the ds9, the ds28, the ds35, the ds36, the ds40, the ds42, the ds79, the ds105, the ds113, the ds156, the ds164, the ds172, the ds175, the ds176, the ds177, the ds178, the ds187, the ds198, the ds201, the ds211, and the ds213 to the ds246 described in any of the Tables 1-1 to 1-6.
15. In the oligonucleotide according to the above 13., the siRNA is preferably any one pair of oligonucleotides selected from the group consisting of the ds2, the ds254, the ds257, the ds259, the ds261, the ds263, the ds267, and the ds268 described in any of Tables 1-1 to 1-6.
16. In the oligonucleotide according to the above 5., the siRNA preferably includes the sequence of any one pair of oligonucleotides selected from the group consisting of ds274 to ds461 described in any of Tables 2-1 to 2-4.
17. In the oligonucleotide according to the above 16., the siRNA preferably includes a sequence of any one pair of oligonucleotides selected from the group consisting of the ds278, the ds280, the ds299, the ds308, the ds347, the ds405, the ds406, the ds411, the ds414, the ds422, and the ds432 to ds461 described in any of the Tables 2-1 to 2-4.

According to one aspect of the present invention,
18. there is provided a pharmaceutical composition containing the oligonucleotide according to any one of the above 1. to 17.

According to one aspect of the present invention,
19. there is provided a therapeutic agent for a central nervous system disease containing the oligonucleotide according to any one of the above 1. to 17.
20. In the oligonucleotide according to the above 2., the administration device preferably has a cannula portion that is formed of a tubular member and is disposed to cover the needle portion such that the puncture portion is exposed,
   the opening portion is preferably provided on a distal end side of the puncture portion,
   a stopper portion having an abutment portion that abuts against an olfactory epithelium of an olfactory mucosa is preferably formed at a distal end portion of the cannula portion, and
   the opening portion is preferably disposed within the cribriform plate in a state where the abutment portion of the stopper portion is brought into contact with the olfactory epithelium.
21. In the oligonucleotide according to the above 20., the administration device preferably has a hub portion to which an accommodation portion accommodating the oligonucleotide is attachable, and
   the hub portion preferably holds a proximal end of the cannula portion and/or a proximal end of a needle shaft portion of the needle portion inserted through a lumen of the cannula portion.
22. In the oligonucleotide according to the above 20. or 21., a cross-sectional shape of the abutment portion of the stopper portion is preferably circular or elliptical.

According to one aspect of the present invention,
23. there is provided an administration system including an accommodation portion accommodating the oligonucleotide according to any one of the above 1. to 17. and an administration device including a needle portion having a puncture portion, in which the administration system is configured such that the oligonucleotide accommodated in the accommodation portion is dischargeable through an opening portion of the puncture portion.
24. In the administration system according to the above 23., the administration device preferably has a cannula portion that is formed of a tubular member and is disposed to cover the needle portion such that the puncture portion is exposed,
   the opening portion is preferably provided on a distal end side of the puncture portion,
   a stopper portion having an abutment portion that abuts against an olfactory epithelium of an olfactory mucosa is preferably formed at a distal end portion of the cannula portion, and
   the opening portion is preferably disposed within the cribriform plate in a state where the abutment portion of the stopper portion is brought into contact with the olfactory epithelium.
25. In the administration system according to the above 24., the administration device preferably has a hub portion to which an accommodation portion accommodating the oligonucleotide is attachable, and
   the hub portion preferably holds a proximal end of the cannula portion and/or a proximal end of a needle shaft portion of the needle portion inserted through a lumen of the cannula portion.
26. In the administration system according to the above 24. or 25., a cross-sectional shape of the abutment portion of the stopper portion is preferably circular or elliptical.

In addition, the above-described objects can also be achieved by the present invention having the following configurations, and the present invention also encompasses the following aspects and embodiments.

According to another aspect of the present invention,
1. there is provided a use of an oligonucleotide administered intranasally into the brain of a mammal by being injected from within a cribriform foramen of a cribriform plate for the treatment of a central nervous system disease.
2. In the use according to the above 1.,
   the oligonucleotide is preferably administered intranasally into the brain of a mammal using an administration device including a needle portion having a puncture portion, and
   injected through an opening portion of the puncture portion in a state where the puncture portion is disposed within a cribriform foramen of the cribriform plate.
3. In the use according to the above 1. or 2., the oligonucleotide is preferably any one selected from the group consisting of an aptamer, an antisense oligonucleotide, a decoy nucleic acid, a ribozyme, siRNA, miRNA, and mRNA.
4. In the use according to any one of the above 1. to 3., the oligonucleotide is preferably siRNA or antisense oligonucleotide.
5. In the use according to any one of the above 1. to 4., the oligonucleotide is preferably siRNA.
6. In the use according to the above 5., the proportion of 2'-modified nucleotides among nucleotides constituting the siRNA is preferably 80% or more.
7. In the use according to the above 5. or 6., all nucleotides constituting the siRNA are preferably 2'-modified nucleotides.
8. The oligonucleotide according to any one of the above 5. to 7. preferably has one or more lipophilic moieties.
9. In the use according to the above 8., in the oligonucleotide, the lipophilic moiety is preferably at least one selected from the group consisting of a substituted or unsubstituted alkyl chain having 14 to 24 carbon atoms, eicosapentaenoic acid (EPA), arachidonic acid (ARA), and cholic acid.
10. In the use according to any one of the above 5. to 9., a target gene of the siRNA is preferably SNCA or HTT.
11. In the use according to any one of the above 5. to 10., in the oligonucleotide, all phosphodiester bonds linking the first and second nucleotides counted from both ends of the sense strand and antisense strand of the siRNA are preferably replaced with phosphorothioate bonds.
12. In the use according to any one of the above 5. to 11., the siRNA preferably includes any one pair of nucleic acid sequences selected from the group consisting of the following (S1) to (S11):
   (S1) SEQ ID NO: 46 (sense strand) and SEQ ID NO: 213 (antisense strand);
   (S2) SEQ ID NO: 83 (sense strand) and SEQ ID NO: 250 (antisense strand);
   (S3) SEQ ID NO: 93 (sense strand) and SEQ ID NO: 260 (antisense strand);
   (S4) SEQ ID NO: 169 (sense strand) and SEQ ID NO: 336 (antisense strand);
   (S5) SEQ ID NO: 180 (sense strand) and SEQ ID NO: 347 (antisense strand);
   (S6) SEQ ID NO: 1360 (sense strand) and SEQ ID NO: 1389 (antisense strand);
   (S7) SEQ ID NO: 1366 (sense strand) and SEQ ID NO: 1395 (antisense strand);
   (S8) SEQ ID NO: 1368 (sense strand) and SEQ ID NO: 1397 (antisense strand);
   (S9) SEQ ID NO: 1372 (sense strand) and SEQ ID NO: 1401 (antisense strand);
   (S10) SEQ ID NO: 1374 (sense strand) and SEQ ID NO: 1403 (antisense strand); and
   (S11) SEQ ID NO: 1756 (sense strand) and SEQ ID NO: 1759 (antisense strand).
13. In the use according to the above 5., the siRNA preferably includes the sequence of any one pair of oligonucleotides selected from the group consisting of ds2, ds9, ds17 to ds249, and ds254 to ds273 described in any of Tables 1-1 to 1-6.
14. In the use according to the above 13., the siRNA preferably includes a sequence of any one pair of oligonucleotides selected from the group consisting of the ds9, the ds28, the ds35, the ds36, the ds40, the ds42, the ds79, the ds105, the ds113, the ds156, the ds164, the ds172, the ds175, the ds176, the ds177, the ds178, the ds187, the ds198, the ds201, the ds211, and the ds213 to the ds246 described in any of the Tables 1-1 to 1-6.
15. In the use according to the above 13., the siRNA is preferably any one pair of oligonucleotides selected from the group consisting of the ds2, the ds254, the ds257, the ds258, the ds259, the ds261, the ds263, the ds267, the ds268, and the ds273 described in any of Tables 1-1 to 1-6.
16. In the use according to the above 5., the siRNA preferably includes the sequence of any one pair of oligonucleotides selected from the group consisting of ds274 to ds461 described in any of Tables 2-1 to 2-4.
17. In the use according to the above 16., the siRNA preferably includes a sequence of any one pair of oligonucleotides selected from the group consisting of the ds278, the ds280, the ds299, the ds308, the ds347, the ds405, the ds406, the ds411, the ds414, the ds422, and the ds432 to ds461 described in any of the Tables 2-1 to 2-4.
18. In the use according to the above 2.,
   the administration device preferably has a cannula portion that is formed of a tubular member and is disposed to cover the needle portion such that the puncture portion is exposed,
   the opening portion is preferably provided on a distal end side of the puncture portion,
   a stopper portion having an abutment portion that abuts against an olfactory epithelium of an olfactory mucosa is preferably formed at a distal end portion of the cannula portion, and
   the opening portion is preferably disposed within the cribriform plate in a state where the abutment portion of the stopper portion is brought into contact with the olfactory epithelium.
19. In the use according to the above 18., the administration device preferably has a hub portion to which an accommodation portion accommodating the oligonucleotide is attachable, and
   the hub portion preferably holds a proximal end of the cannula portion and/or a proximal end of a needle shaft portion of the needle portion inserted through a lumen of the cannula portion.
20. In the use according to the above 18. or 19., a cross-sectional shape of the abutment portion of the stopper portion is preferably circular or elliptical.

### Brief Description of Drawings

[Fig. 1] Fig. 1 is a view illustrating a structure of a phosphate group-modified nucleotide contained in the oligonucleotide used in Examples.
[Fig. 2] Fig. 2 is a view illustrating a structure of a lipophilic moiety contained in the oligonucleotide used in Examples.
[Fig. 3] Fig. 3 is a view illustrating an example of an administration device and an administration system used for intranasal administration of an oligonucleotide according to one embodiment of the present invention.
[Fig. 4-1] Fig. 4-1 is a view illustrating a state where a puncture portion of the administration device is punctured into a cribriform foramen.
[Fig. 4-2A] Fig. 4-2A is a first view for explaining a method of using the administration device.

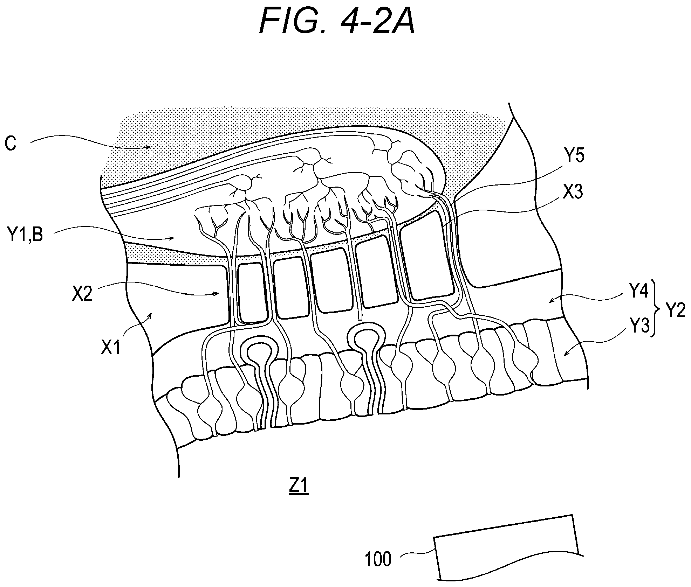

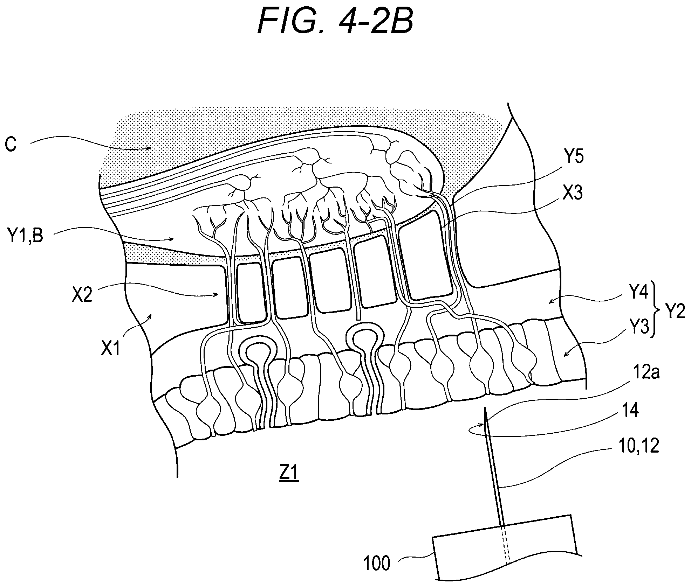

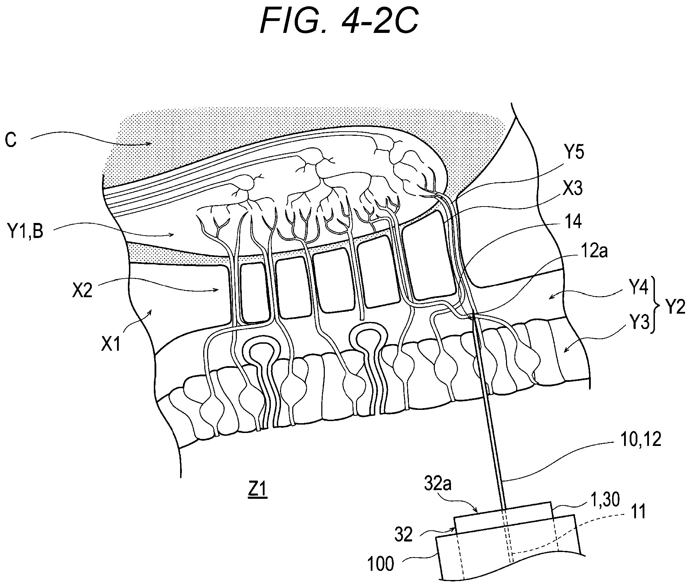

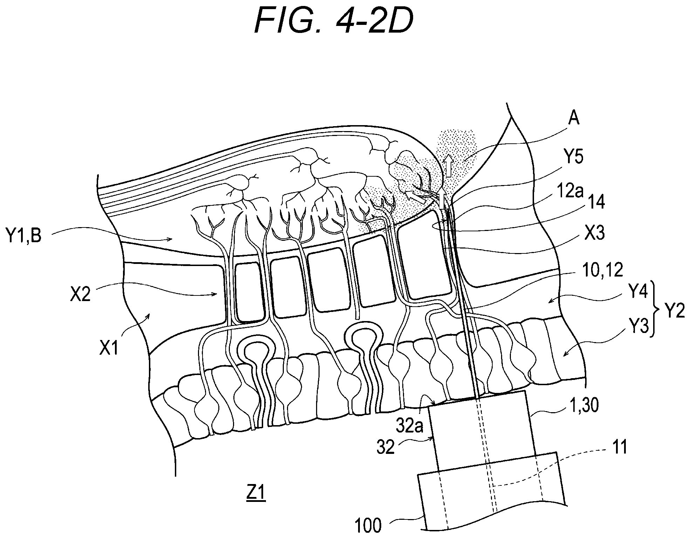

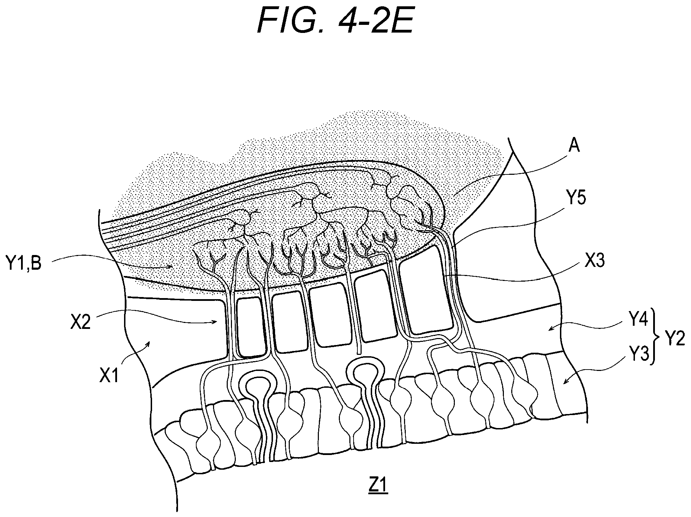

[Fig. 4-2B] Fig. 4-2B is a second view for explaining a method of using the administration device.
[Fig. 4-2C] Fig. 4-2C is a third view for explaining a method of using the administration device.
[Fig. 4-2D] Fig. 4-2D is a fourth view for explaining a method of using the administration device.
[Fig. 4-2E] Fig. 4-2E is a fifth view for explaining a method of using the administration device.

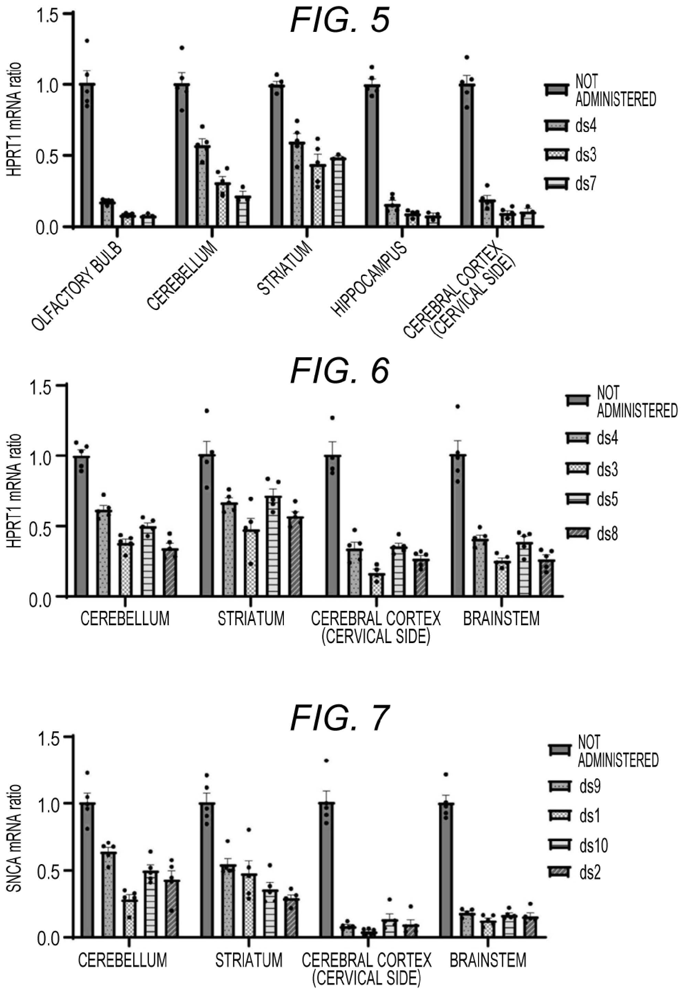

[Fig. 5] Fig. 5 is a view illustrating measurement results of Reference Example 1-1.
[Fig. 6] Fig. 6 is a view illustrating measurement results of Reference Example 1-2.
[Fig. 7] Fig. 7 is a view illustrating measurement results of Reference Example 1-3.

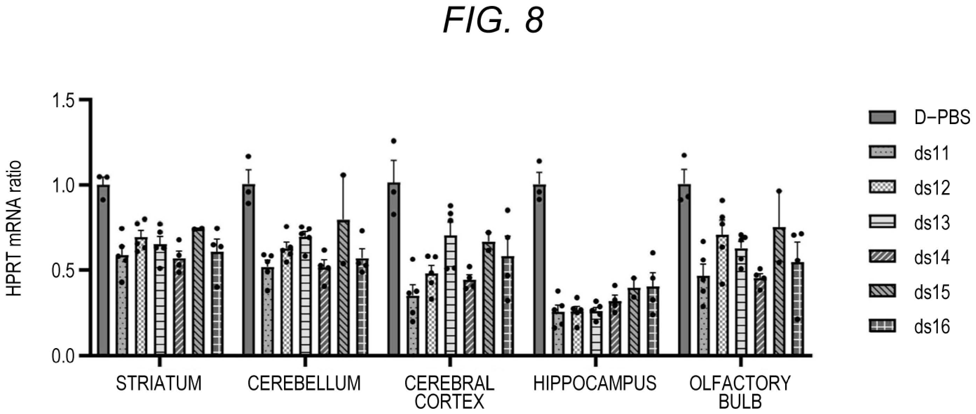

[Fig. 8] Fig. 8 is a view illustrating measurement results of Reference Example 2.

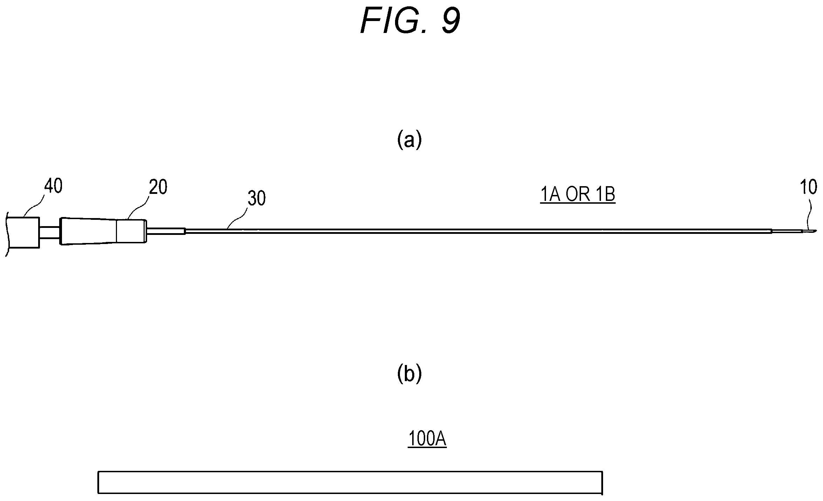

[Fig. 9] Fig. 9 is a view illustrating configurations of administration devices 1A and 1B and a guide catheter 100A used in Examples.

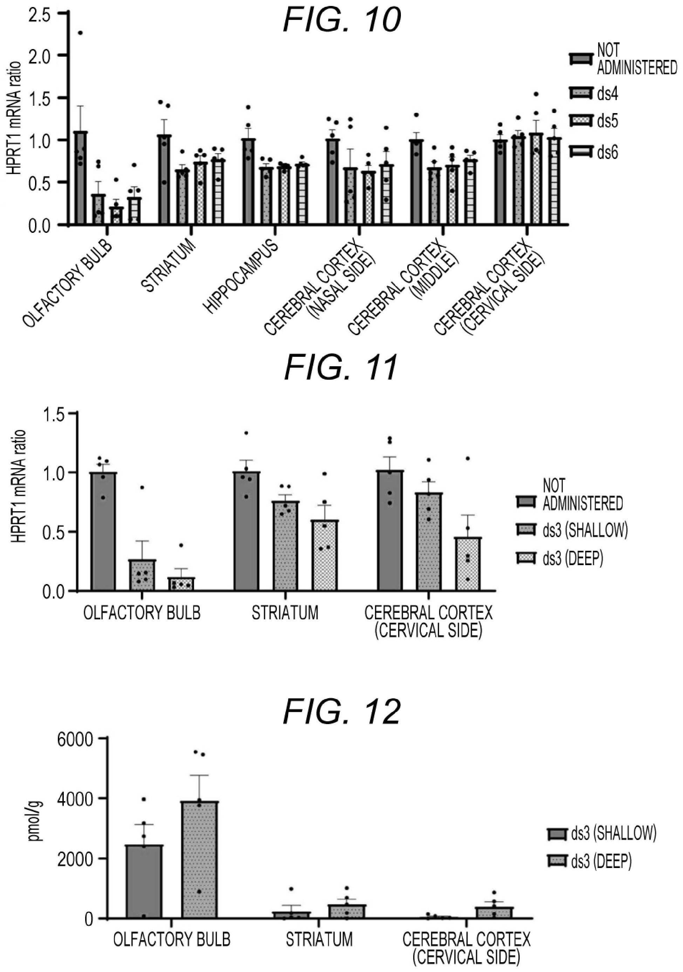

[Fig. 10] Fig. 10 is a view illustrating measurement results of knockdown activity in Example 1.
[Fig. 11] Fig. 11 is a view illustrating measurement results of knockdown activity in Example 2.
[Fig. 12] Fig. 12 is a view illustrating measurement results of the transferred amount of nucleic acid in Example 2.

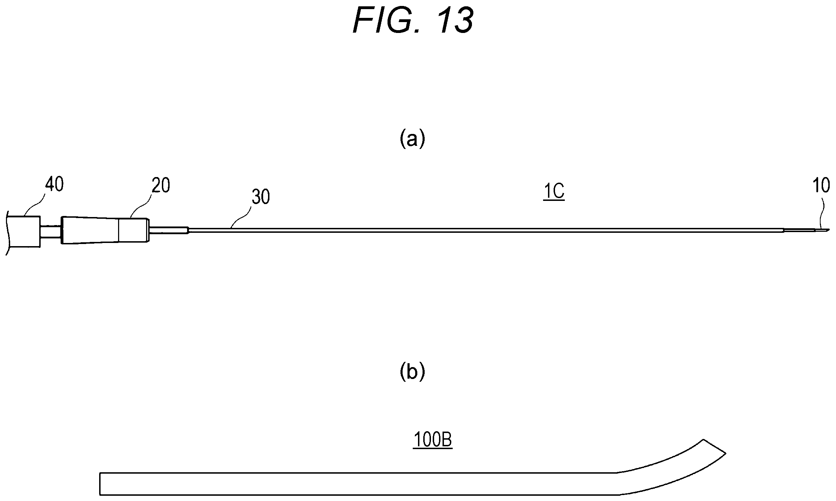

[Fig. 13] Fig. 13 is a view illustrating configurations of an administration device 1C and a guide catheter 100B used in Examples.

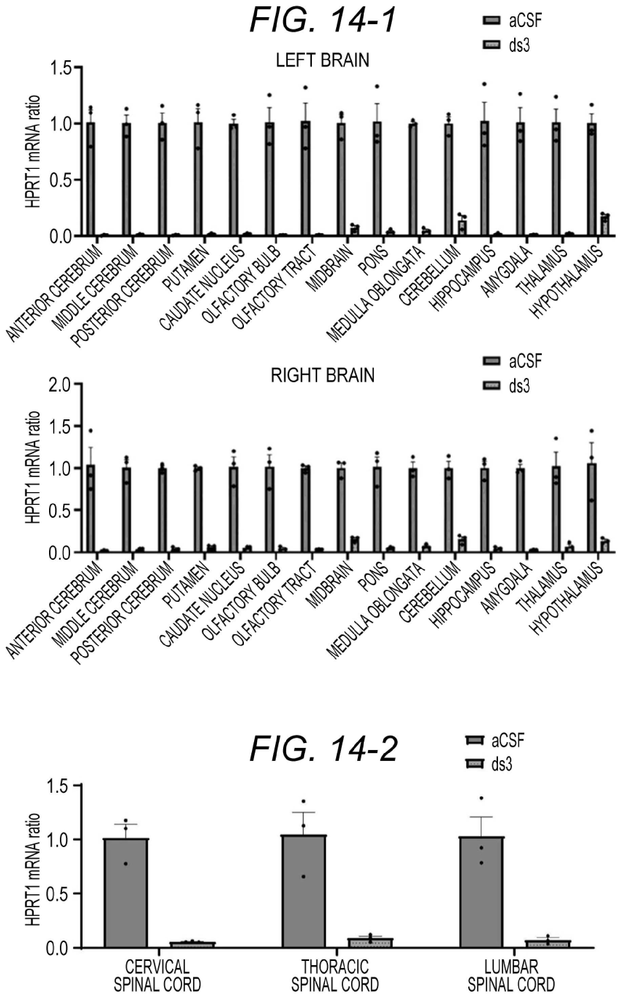

[Fig. 14-1] Fig. 14-1 is a view illustrating measurement results of knockdown activity in Example 3 (respective brain regions).
[Fig. 14-2] Fig. 14-2 is a view illustrating measurement results of knockdown activity in Example 3 (cervical spinal cord, thoracic spinal cord, and lumbar spinal cord).

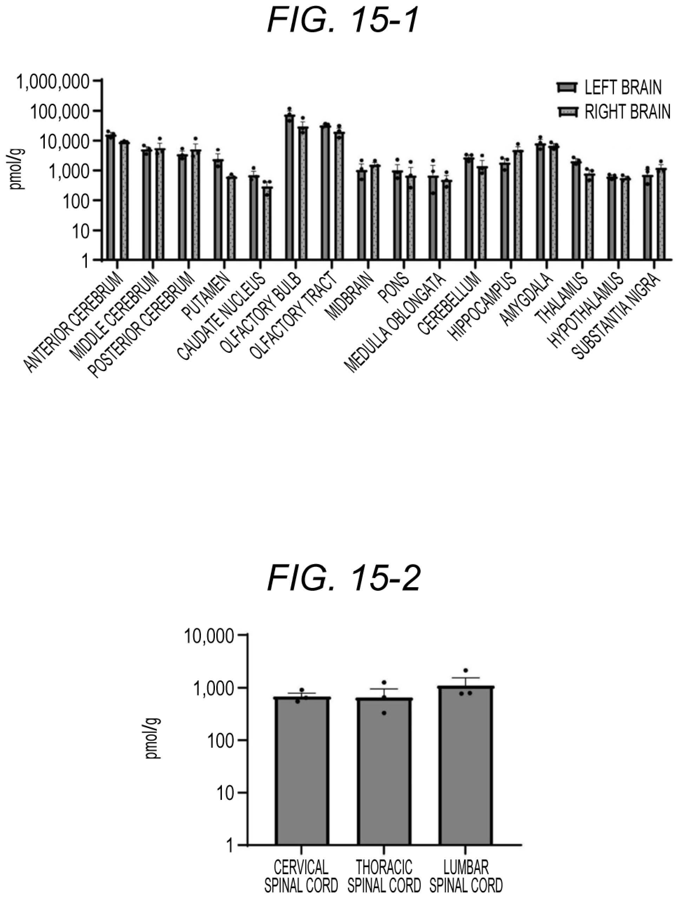

[Fig. 15-1] Fig. 15-1 is a view illustrating measurement results of the transferred amount of nucleic acid in Example 3 (respective brain regions).
[Fig. 15-2] Fig. 15-2 is a view illustrating measurement results of the transferred amount of nucleic acid in Example 3 (cervical spinal cord, thoracic spinal cord, and lumbar spinal cord).

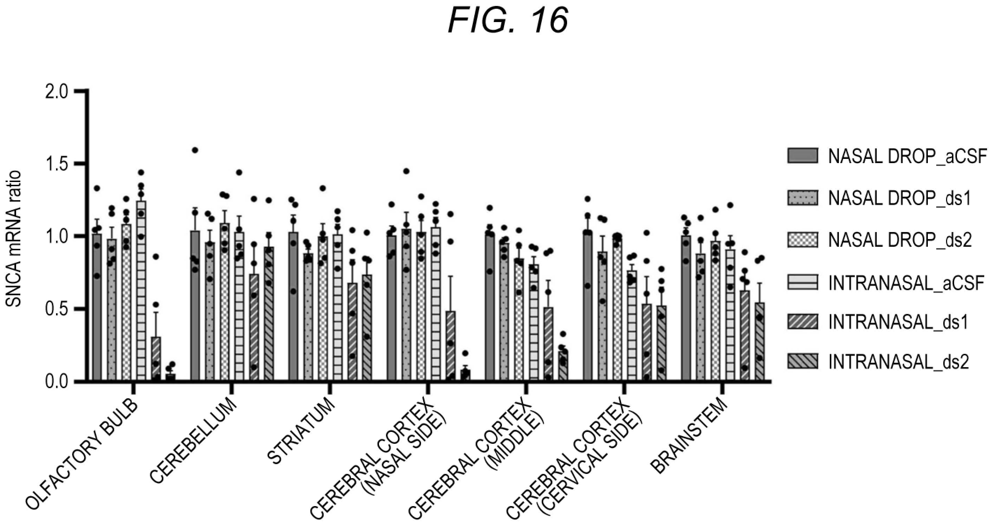

[Fig. 16] Fig. 16 is a view illustrating measurement results of knockdown activity in Example 4.

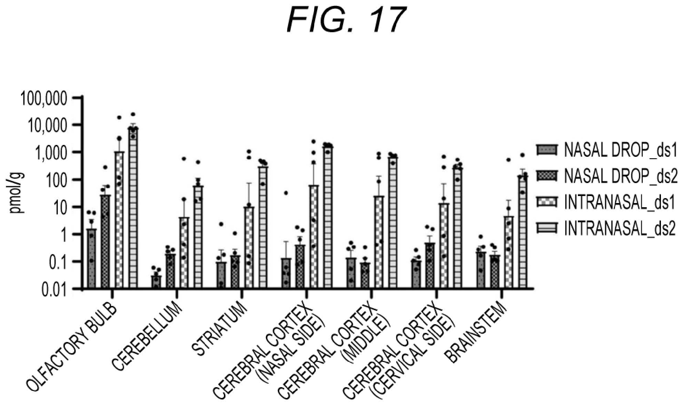

[Fig. 17] Fig. 17 is a view illustrating measurement results of the transferred amount of nucleic acid in Example 4.

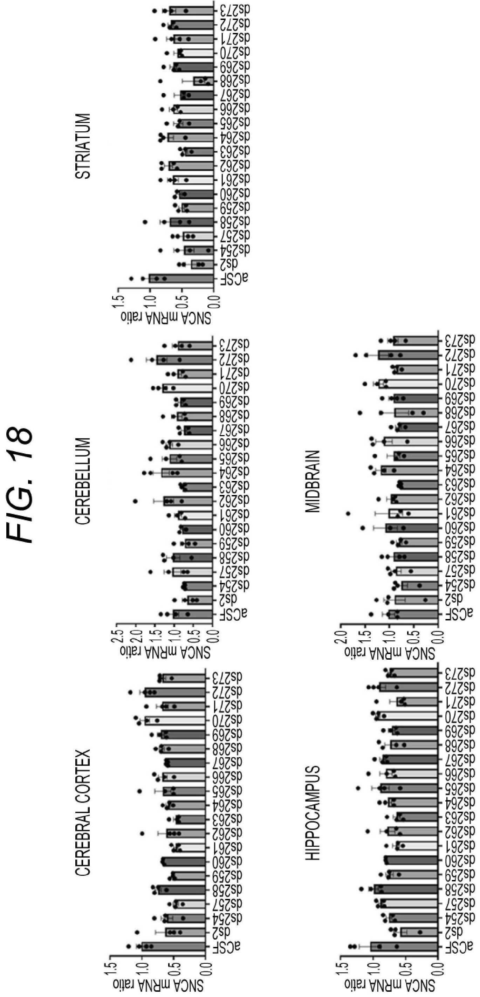

[Fig. 18] Fig. 18 is a view illustrating measurement results of knockdown activity in Example 7.

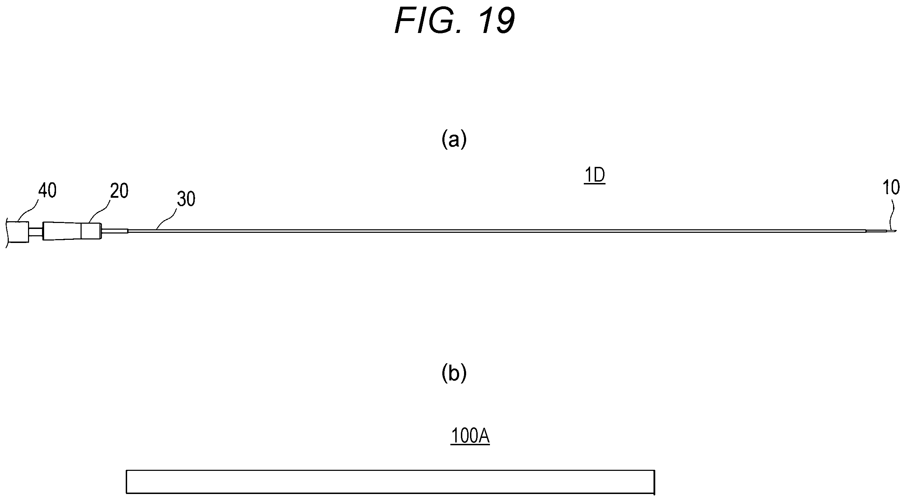

[Fig. 19] Fig. 19 is a view illustrating configurations of an administration device 1D and the guide catheter 100A used in Example 8.

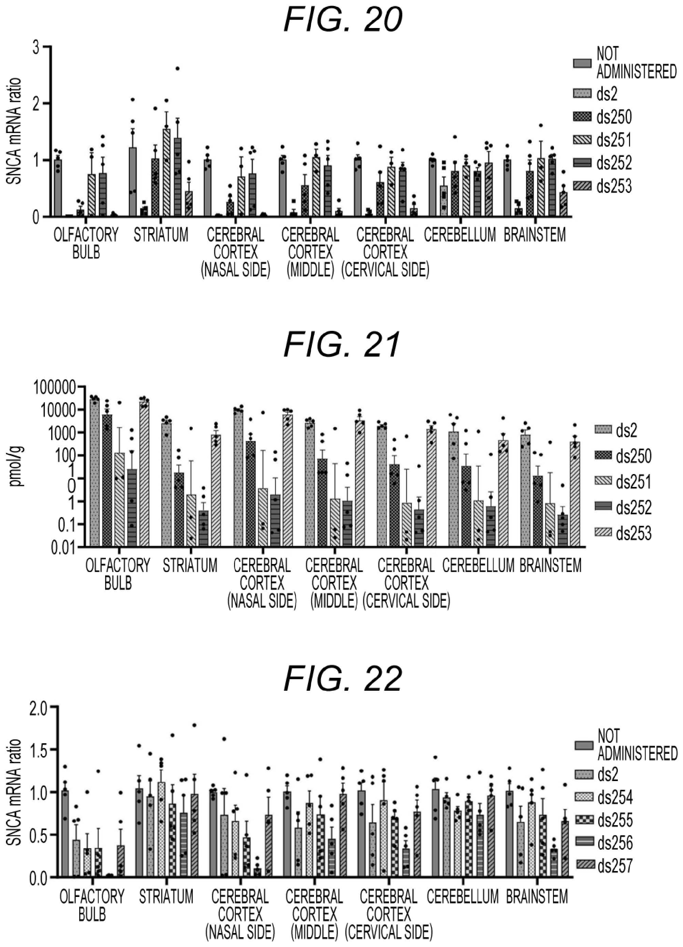

[Fig. 20] Fig. 20 is a view illustrating measurement results of knockdown activity in Example 8.
[Fig. 21] Fig. 21 is a view illustrating measurement results of the transferred amount of nucleic acid in Example 8.
[Fig. 22] Fig. 22 is a view illustrating measurement results of knockdown activity in Example 9.

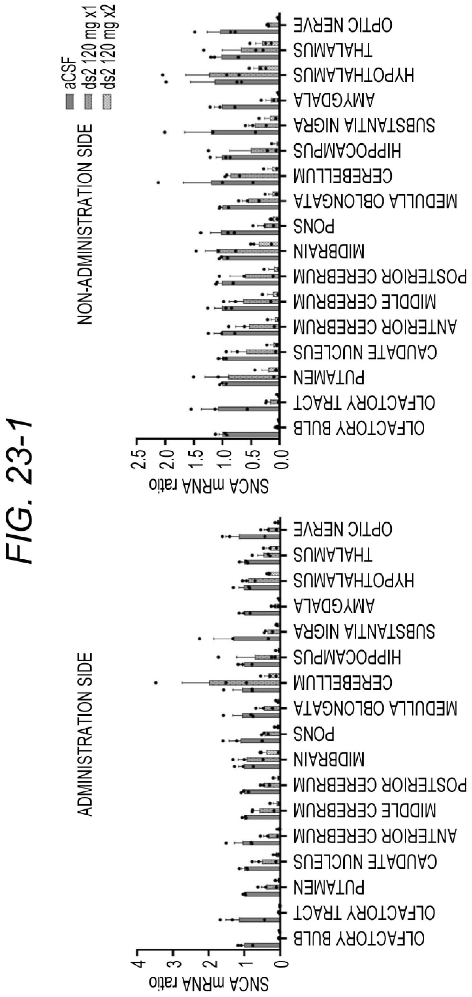

[Fig. 23-1] Fig. 23-1 is a view illustrating measurement results of knockdown activity by quantitative PCR in Example 10 (respective brain regions).

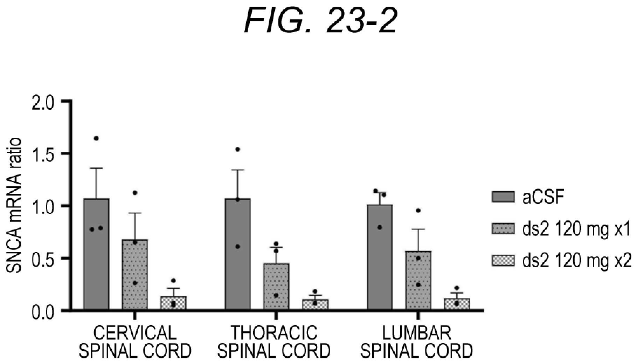

[Fig. 23-2] Fig. 23-2 is a view illustrating measurement results of knockdown activity by quantitative PCR in Example 10 (spinal cord).

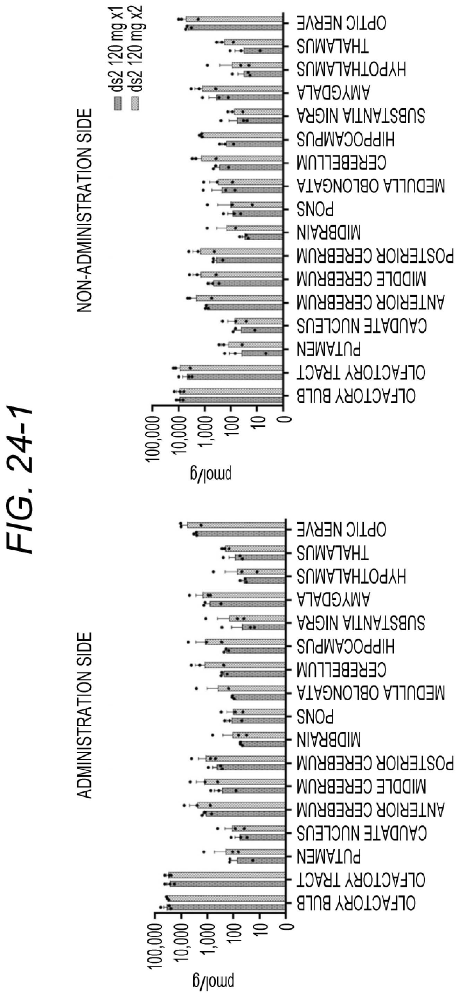

[Fig. 24-1] Fig. 24-1 is a view illustrating measurement results of the transferred amount of nucleic acid in Example 10 (respective brain regions).

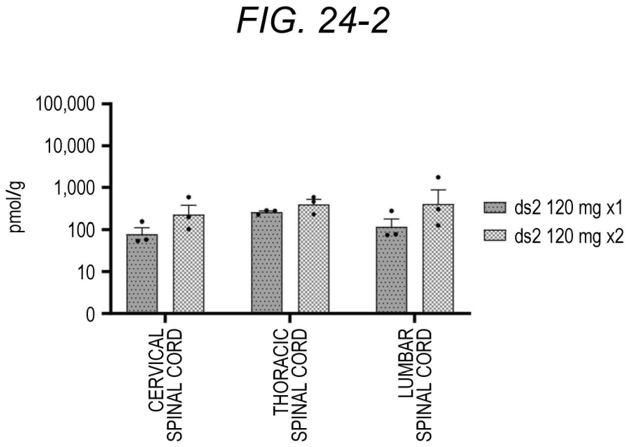

[Fig. 24-2] Fig. 24-2 is a view illustrating measurement results of the transferred amount of nucleic acid in Example 10 (spinal cord).

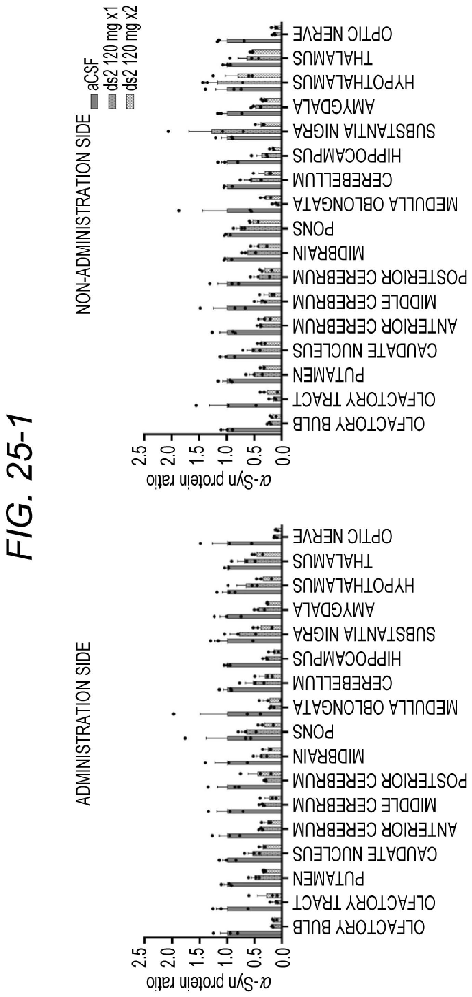

[Fig. 25-1] Fig. 25-1 is a view illustrating measurement results of knockdown activity by protein quantification in Example 10 (respective brain regions).

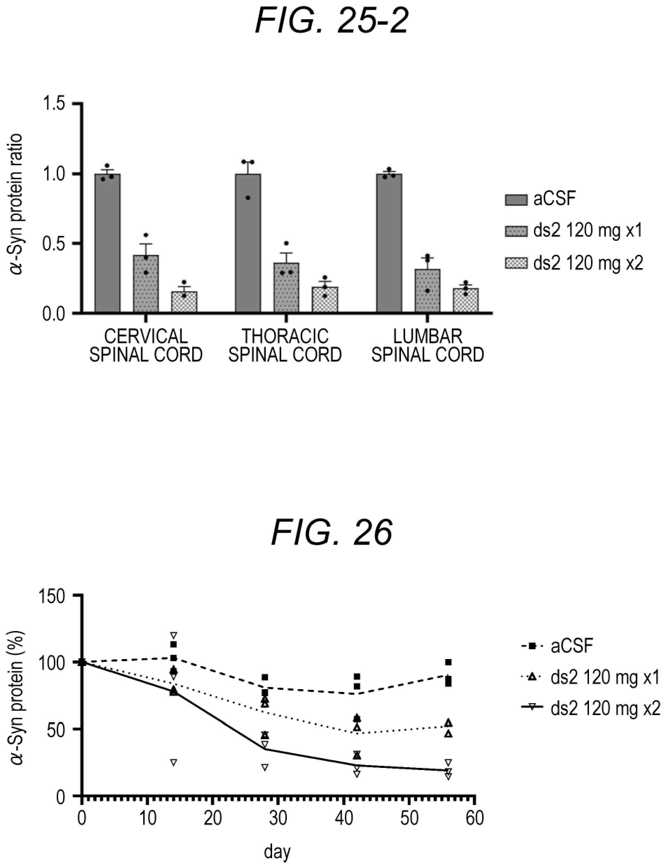

[Fig. 25-2] Fig. 25-2 is a view illustrating measurement results of knockdown activity by protein quantification in Example 10 (spinal cord).
[Fig. 26] Fig. 26 is a view illustrating measurement results of time-course change of knockdown activity (CSF) by protein quantification in Example 10.

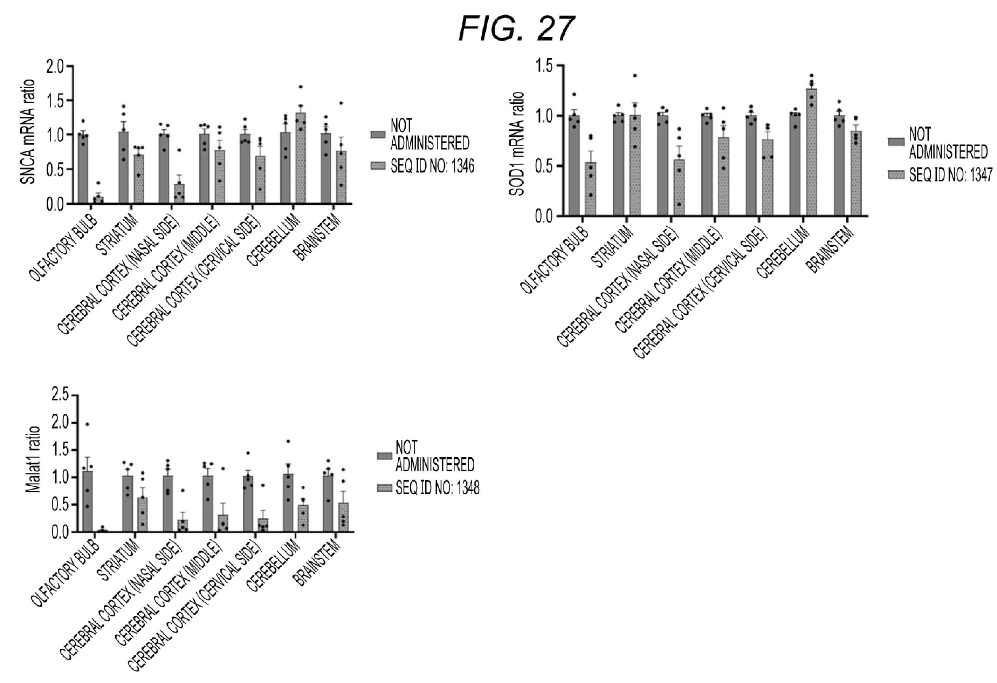

[Fig. 27] Fig. 27 is a view illustrating measurement results of knockdown activity in Example 11.

### Description of Embodiments

Hereinafter, embodiments of the present invention will be described. Note that the present invention is not limited only to the following embodiments. In addition, in the present specification, unless otherwise specified, operations and measurements of physical properties and the like are performed under conditions of room temperature (20°C or higher and 25°C or lower)/relative humidity of 40% RH or higher and 50% RH or lower.

Throughout the present specification, expression of a singular should be understood to include the concept of a plural thereof as well unless otherwise stated. Thus, an article of a singular (for example, "a", "an", "the", and the like in English) should be understood to include the concept of a plural thereof as well unless otherwise stated. Further, terms used in the present specification should be understood to be used in a sense commonly used in the art unless otherwise stated. Accordingly, unless defined otherwise, all technical and scientific terms used in the present specification have the same meaning as commonly understood by those skilled in the art to which the present invention belongs. In the case of contradiction, priority is given to the present specification (including definitions). The present invention is not limited to the following embodiments, and various modifications can be made within the scope of claims. In addition, in the present specification, "X to Y" means a range including numerical values (X and Y) described before and after "to" as a lower limit value and an upper limit value, and means "X or more and Y or less". Further, unless otherwise specified, the concentration "%" represents mass concentration "% by mass", and ratios are mass ratios unless otherwise specified.

An oligonucleotide according to one aspect of the present invention is administered intranasally into the brain of a mammal by being injected from within a cribriform foramen of a cribriform plate. Further, the oligonucleotide according to one embodiment is administered intranasally into the brain of a mammal using an administration device including a needle portion having a puncture portion, and is administered through an opening portion of the puncture portion in a state where the puncture portion is disposed within the cribriform plate. The oligonucleotide according to one embodiment of the present invention can be efficiently administered (delivered) into the brain while being minimally invasive, and can further provide high efficacy as a nucleic acid drug.

The present inventors presume that the mechanism by which the above-described problems can be solved by the present invention is as follows.

As a result of intensive studies, the present inventors arrived at the hypothesis that, in conventionally known minimally invasive intranasal administration represented by a conventional intranasal spray method, delivery of a drug to the brain is inhibited due to clearance by an epithelial barrier. The oligonucleotide according to the present invention takes the above hypothesis into consideration and is administered intranasally into the brain of a mammal by being injected from within a cribriform foramen of a cribriform plate.

Here, "injecting an oligonucleotide from within a cribriform foramen of the cribriform plate" means that the oligonucleotide is directly injected into a cribriform foramen of the cribriform plate. The injection can be performed using a medical device having a structure capable of injecting the oligonucleotide into a cribriform foramen of the cribriform plate, such as an injection needle. The oligonucleotide injected into the cribriform foramen of the cribriform plate flows from within the cribriform foramen of the cribriform plate into the brain due to injection pressure or the like, thereby enabling intranasal administration of the oligonucleotide into the brain. For example, the oligonucleotide according to one embodiment of the present invention may be administered intranasally into the brain by injecting the oligonucleotide (drug) through the opening portion of the puncture portion in a state where the puncture portion of the administration device is disposed within a cribriform foramen of the cribriform plate.

As described above, although the oligonucleotide according to the present application is for intranasal administration, the oligonucleotide enables administration from within a cribriform foramen of the cribriform plate, which is a site close to the brain. Accordingly, it is considered that the oligonucleotide according to the present invention is less susceptible to the effects of clearance by an epithelial barrier and achieves a high transfer rate into the brain. Further, since a sufficient amount of the oligonucleotide is transferred into the brain, a sufficient amount of the oligonucleotide is taken up into tissues in each region of the brain, and it is considered that the efficacy of the oligonucleotide is thereby also improved. For example, since an amount of the oligonucleotide necessary for reducing expression (knockdown) of a protein that causes a central nervous system disease (hereinafter also referred to as a central disease) or the like reaches each region of the brain, knockdown efficiency in each region is improved, and it is considered that the efficacy of the oligonucleotide as a nucleic acid drug is thereby also improved. Note that the above mechanism is based on presumption, and the present invention is not limited to the mechanism in any way.

### [Oligonucleotide]

The oligonucleotide according to one embodiment of the present invention may be any molecule as long as the molecule is a molecule in which nucleotides or molecules having functions equivalent to those of the nucleotides are polymerized, and examples thereof include RNA, which is a polymer of ribonucleotides; DNA, which is a polymer of deoxyribonucleotides; a chimeric nucleic acid, which is a polymer of ribonucleotides and deoxyribonucleotides; and nucleotide polymers in which at least one nucleotide of these nucleic acids (RNA, DNA, and chimeric nucleic acid) is replaced with a molecule having a function equivalent to that of the nucleotide. Uracil (U) in RNA is unambiguously read as thymine (T) in DNA.

Examples of the molecule having a function equivalent to that of a nucleotide include, for example, a nucleotide derivative in which a nucleotide is modified. Although not particularly limited thereto, the use of a nucleotide derivative provides advantages such as, for example, enabling an increase in the transferred amount to each region of the brain, improving or stabilizing nuclease resistance, improving affinity for a complementary-strand nucleic acid, improving cell permeability, and/or enabling visualization, as compared with RNA or DNA.

As the oligonucleotide according to the present invention, an oligonucleotide known to be used as a nucleic acid drug can be used. Among these, the oligonucleotide according to one embodiment of the present invention is preferably selected from the group consisting of an aptamer, an antisense oligonucleotide (ASO), a decoy nucleic acid, a ribozyme, siRNA, miRNA (microRNA), and mRNA (messenger RNA), and among these, the oligonucleotide according to one embodiment of the present invention is more preferably siRNA or an antisense oligonucleotide. Because the oligonucleotide is selected from these, it becomes possible for the oligonucleotide to bind more effectively to a target molecule, and control in the transcription and translation processes of a gene, inhibition of protein function, and the like can be more effectively achieved.

Here, an aptamer is a single-stranded nucleic acid molecule that binds to a specific target molecule (for example, a protein or the like). The aptamer can bind to a disease-related target protein or the like and inhibit the function thereof.

An antisense oligonucleotide is DNA, RNA, or a chimeric molecule of DNA and RNA that is at least partially complementary to a target molecule, which is a base sequence (for example, mRNA or miRNA), and may be single-stranded. For example, an antisense oligonucleotide inhibits translation of a complementary RNA strand by binding to the complementary RNA strand. It is also possible to control splicing by binding to a specific complementary pre-mRNA. The antisense oligonucleotide may be a heteroduplex oligonucleotide (HDO) that forms a double strand with a complementary strand.

A ribozyme, also referred to as an RNA enzyme, is a generic term for RNA having catalytic function. A ribozyme can be designed to cut itself or another target RNA molecular strand, and is thereby considered useful as an inhibitor of gene expression.

A decoy nucleic acid is a double-stranded oligonucleotide having a nucleic acid-recognizing protein such as a transcription factor as a target molecule. The decoy nucleic acid mimics a nucleic acid sequence recognized by a nucleic acid-recognizing protein, and inhibits binding of the protein to the target nucleic acid.

siRNA is an abbreviation for small interfering RNA (short chain interfering RNA), and is a double-stranded oligonucleotide involved in RNA interference (hereinafter RNAi). The oligonucleotides constituting the siRNA may be composed of RNA, or may be composed of RNA and DNA. More specifically, siRNA has a function as a guide for suppressing expression of a target gene, which is a target molecule, and can selectively suppress (knock down) expression of the protein whose expression is mediated by the mRNA through cleavage of messenger RNA (mRNA). In this manner, from the viewpoint of more efficiently suppressing expression of a target protein and more effectively treating a central nervous system disease, the oligonucleotide according to one embodiment of the present invention is further preferably siRNA.

When the oligonucleotide according to one embodiment is siRNA, it is preferable that all phosphodiester bonds linking the first and second nucleotides counted from both ends of the sense strand and the antisense strand constituting the siRNA are replaced with phosphorothioate bonds. Further, it is more preferable that all phosphodiester bonds linking the first and second nucleotides and the second and third nucleotides counted from both ends of the sense strand and the antisense strand are all replaced with phosphorothioate bonds. By having the above configuration, the knockdown effect by siRNA can be improved.

In one embodiment of the present invention, siRNA having a hairpin structure that includes a sense strand and an antisense strand in a single-stranded oligonucleotide and forms complementary base pairs intramolecularly can also be used.

miRNA (microRNA) is a small molecule RNA of about 22 bases encoded in the genome, and controls gene expression by suppressing translation from mRNA, which is a target molecule having a sequence partially complementary to the miRNA.

mRNA (messenger RNA) is an oligonucleotide encoding information of a target protein, and can be used for treatment or prevention of a disease by causing the target protein to be produced in cells.

When the oligonucleotide according to one embodiment of the present invention is single-stranded and is other than mRNA, the number of nucleotides forming the single strand (that is, the number of bases) may be 10 or more and 100 or less, 10 or more and 70 or less, 10 or more and 60 or less, 10 or more and 45 or less, 10 or more and 30 or less, 10 or more and 20 or less, 12 or more and 100 or less, 12 or more and 70 or less, 12 or more and 60 or less, 12 or more and 45 or less, 12 or more and 30 or less, 12 or more and 20 or less, 15 or more and 100 or less, 15 or more and 70 or less, 15 or more and 60 or less, 15 or more and 45 or less, 15 or more and 30 or less, 15 or more and 20 or less, 30 or more and 100 or less, 30 or more and 70 or less, 30 or more and 60 or less, 30 or more and 45 or less, 40 or more and 100 or less, 40 or more and 70 or less, 40 or more and 60 or less, or 40 or more and 45 or less. For example, when the oligonucleotide according to one embodiment of the present invention is a single-stranded antisense oligonucleotide, the number of nucleotides forming the single strand (that is, the number of bases) is preferably 10 or more and 30 or less, more preferably 11 or more and 25 or less, and still more preferably 12 or more and 20 or less. Further, for example, when the oligonucleotide according to one embodiment of the present invention is siRNA composed of a single strand and having a loop structure such as a hairpin, the number of nucleotides forming the single strand is preferably 30 or more and 70 or less, and more preferably 40 or more and 60 or less. Further, when the oligonucleotide according to one embodiment of the present invention is a single-stranded aptamer, the number of nucleotides forming the single strand (that is, the number of bases) is preferably 10 or more and 100 or less, and more preferably 15 or more and 45 or less.

Further, when the oligonucleotide according to one embodiment of the present invention is a single-stranded mRNA, the number of nucleotides forming the single strand (that is, the number of bases) is preferably 50 or more and 15000 or less, and more preferably 100 or more and 10000 or less.

Further, when the oligonucleotide according to one embodiment of the present invention is double-stranded, each single strand forming the double strand is preferably 10 or more and 30 or less, and more preferably 15 or more and 25 or less. That is, the number of nucleotides constituting the sense strand is preferably 10 or more and 30 or less, and more preferably 15 or more and 25 or less. Similarly, the number of nucleotides constituting the antisense strand is preferably 10 or more and 30 or less, and more preferably 15 or more and 25 or less. For example, when the oligonucleotide according to one embodiment of the present invention is siRNA composed of a double strand, the number of nucleotides constituting the sense strand is preferably 10 or more and 30 or less, and more preferably 15 or more and 25 or less. Similarly, the number of nucleotides constituting the antisense strand is preferably 10 or more and 30 or less, and more preferably 15 or more and 25 or less. When the number of nucleotides constituting the oligonucleotide is within the above range, the oligonucleotide is more readily taken up into cells with higher efficiency and has higher binding ability to the target molecule, thereby further improving the efficacy as an oligonucleotide.

Further, when the oligonucleotide according to one embodiment of the present invention is single-stranded and is other than mRNA, the molecular weight (Da) may be 3000 or more and 30000 or less, 3000 or more and 21000 or less, 3000 or more and 18000 or less, 3000 or more and 13500 or less, 3000 or more and 9000 or less, 3000 or more and 6000 or less, 3600 or more and 30000 or less, 3600 or more and 21000 or less, 3600 or more and 18000 or less, 3600 or more and 13500 or less, 3600 or more and 9000 or less, 3600 or more and 6000 or less, 4500 or more and 30000 or less, 4500 or more and 21000 or less, 4500 or more and 18000 or less, 4500 or more and 13500 or less, 4500 or more and 9000 or less, 4500 or more and 6000 or less, 9000 or more and 30000 or less, 9000 or more and 21000 or less, 9000 or more and 18000 or less, 9000 or more and 13500 or less, 12000 or more and 30000 or less, 12000 or more and 21000 or less, 12000 or more and 18000 or less, or 12000 or more and 13500 or less. For example, when the oligonucleotide according to one embodiment of the present invention is a single-stranded antisense oligonucleotide, the molecular weight is preferably 3000 or more and 9000 or less, more preferably 3200 or more and 8000 or less, still more preferably 3400 or more and 7000 or less, and particularly preferably 3600 or more and 6000 or less. Further, for example, when the oligonucleotide according to one embodiment of the present invention is siRNA composed of a single strand and having a loop structure such as a hairpin, the molecular weight is preferably 9000 or more and 21000 or less, and more preferably 12000 or more and 18000 or less. Further, when the oligonucleotide according to one embodiment of the present invention is a single-stranded aptamer, the molecular weight is preferably 3000 or more and 30000 or less, and more preferably 4500 or more and 13500 or less.

Further, when the oligonucleotide according to one embodiment of the present invention is single-stranded mRNA, the molecular weight is preferably 15000 or more and 4500000 or less, and more preferably 30000 or more and 3000000 or less.

Further, when the oligonucleotide according to one embodiment of the present invention is composed of a double strand, the molecular weight is preferably 6000 or more and 20000 or less, more preferably 9000 or more and 18000 or less, and still more preferably 13000 or more and 16000 or less. For example, when the oligonucleotide according to one embodiment of the present invention is siRNA composed of a double strand, the molecular weight is preferably 6000 or more and 20000 or less, more preferably 9000 or more and 18000 or less, and still more preferably 13000 or more and 16000 or less. When the molecular weight of the oligonucleotide is within the above range, the oligonucleotide is more readily taken up into cells with higher efficiency and has higher binding ability to the target molecule, thereby further improving the efficacy as an oligonucleotide.

### (Target Molecule)

The target molecule targeted by the oligonucleotide according to one embodiment of the present invention is not particularly limited, but may be a molecule capable of providing a therapeutic, prophylactic, or ameliorative benefit for a disease of the central nervous system, particularly a cranial nervous system disease. For example, when the target molecule is a target gene, the gene may be one for which knockdown of the target gene can provide a therapeutic, prophylactic, or ameliorative benefit for a disease of the central nervous system, particularly a cranial nervous system disease.

In the present specification, a central nervous system disease (also referred to as a central disease) means a disease of the central nervous system. Examples of central nervous system diseases include Alzheimer's disease, Parkinson's disease, Huntington's disease, amyotrophic lateral sclerosis (ALS), frontotemporal dementia (FTD), dementia with Lewy bodies, multiple system atrophy (MSA), spinal muscular atrophy (SMA), Dravet syndrome, Angelman syndrome, spinocerebellar ataxia, Alexander disease, progressive supranuclear palsy (PSP), multiple sclerosis, prion disease, lysosomal disease, peroxisomal disease, mitochondrial disease, glioblastoma, and cerebral infarction.

Examples of target molecules include Cu/Zn superoxide dismutase (SOD1), fused in sarcoma (FUS), C9ORF72, or ataxin 2 (ATXN2), which are involved in amyotrophic lateral sclerosis (ALS). In addition, other examples of target molecules include amyloid precursor protein (APP) and tau, which are involved in Alzheimer's disease. Further, other examples of target molecules include leucine-rich repeat kinase 2 (LRRK2) and α-synuclein (SNCA), which are involved in Parkinson's disease. In addition, another example of a target molecule is huntingtin (HTT), which is involved in Huntington's disease. Further, another example of a target molecule is ATXN3, which is involved in spinocerebellar ataxia. In addition, another example of a target molecule is a UBE3A antisense transcript, which is involved in Angelman syndrome. Further, another example of a target molecule is GFAP, which is involved in Alexander disease. In addition, another example of a target molecule is SMN2, which is involved in spinal muscular atrophy (SMA).

That is, the oligonucleotide according to one embodiment of the present invention can have Cu/Zn superoxide dismutase (SOD1), fused in sarcoma (FUS), C9ORF72, ATXN2 (ataxin 2), amyloid precursor protein (APP), tau, leucine-rich repeat kinase 2 (LRRK2), SNCA (α-synuclein), huntingtin (HTT), ATXN3, a UBE3A antisense transcript, GFAP, or SMN2 as a target molecule.

Further, when the oligonucleotide according to one embodiment of the present invention is siRNA, the above-described target molecules can be target genes, and among these, the target gene is preferably α-synuclein (SNCA) or huntingtin (HTT). Since the siRNA according to one embodiment of the present invention can knock down these target genes more effectively, it is expected to have a significant effect in the treatment, prevention, and amelioration of synucleinopathies such as MSA and Parkinson's disease, and Huntington's disease.

### (Nucleotide Derivative)

Examples of the nucleotide derivative include a sugar-modified nucleotide, a phosphodiester bond-modified nucleotide, a base-modified nucleotide, and a phosphate group-modified nucleotide. Details of various modified nucleotides are described below, but one nucleotide derivative may have features of two or more types of modified nucleotides. For example, a sugar-modified nucleotide may have features of a base-modified nucleotide, or may have features of a phosphate group-modified nucleotide.

In the oligonucleotide according to one embodiment of the present invention, among all nucleotides constituting the oligonucleotide, the proportion occupied by nucleotide derivatives may be 5% or more, 10% or more, 20% or more, 30% or more, 40% or more, 50%, 60% or more, 70% or more, 80% or more, 90% or more, 95% or more, or 99% or more, or may be 100%. When the proportion of nucleotide derivatives relative to all nucleotides constituting the oligonucleotide is within the above range, the efficiency of transfer of the oligonucleotide to each region of the brain is improved.

### <Sugar-Modified Nucleotide>

The sugar-modified nucleotide may be any nucleotide in which a part or all of the chemical structure of the sugar of the nucleotide is modified or substituted with any substituent, or substituted with any atom. However, a 2'-modified nucleotide is preferably used. That is, in the oligonucleotide according to one embodiment of the present invention, at least one of the nucleotides constituting the oligonucleotide is preferably a 2'-modified nucleotide.

Further, in the oligonucleotide according to one embodiment of the present invention, among all nucleotides constituting the oligonucleotide, the proportion of 2'-modified nucleotides is preferably 70% or more, more preferably 80% or more, still more preferably 90% or more, even more preferably 95% or more, and particularly preferably 99% or more (upper limit: 100%). When the proportion of 2'-modified nucleotides relative to all nucleotides constituting the oligonucleotide is within the above range, the efficiency of transfer of the oligonucleotide to each region of the brain is further improved.

Furthermore, in the oligonucleotide according to one embodiment of the present invention, among all nucleotides constituting the oligonucleotide, the proportion of 2'-modified nucleotides is most preferably 100%. That is, in the oligonucleotide according to one embodiment of the present invention, all of the nucleotides constituting the oligonucleotide are most preferably 2'-modified nucleotides (Claim 7). With this configuration, the transferred amount of the oligonucleotide to each region of the brain is more sufficiently improved.

Examples of the 2'-modified nucleotide include a 2'-modified nucleotide in which the 2'-OH group of ribose is substituted with a substituent selected from the group consisting of -H, -OR, -R, -R'OR, -SH, -SR, -NH₂, -NHR, - NR₂, -N₃, -CN, -F, -Cl, -Br, and -I (R is an alkyl group or an aryl group, preferably an alkyl group having 1 to 6 carbon atoms; R' is an alkylene group, preferably an alkylene group having 1 to 6 carbon atoms; and the two R groups in -NR₂ may be the same or different). Among these, as the 2'-modification, substitution with -H, -F, a methoxy group, a methoxyethoxy group, or an ethoxy group is suitable. That is, in one embodiment of the present invention, the 2'-modified nucleotide may be a 2'-modified nucleotide in which the 2'-OH group of ribose is substituted with a substituent selected from the group consisting of -F, a methoxy group, a methoxyethoxy group, and an ethoxy group. Examples of such 2'-modified nucleotides above include 2'-O-methyl-RNA, 2'-fluoro-DNA, and 2'-O-methoxyethyl-RNA.

Further, the 2'-modified nucleotide may be a nucleotide having two or more substituents at the 2' position, for example one having -F and a methoxy group (J. Am. Chem. Soc. 2022, 144, 14517-14534). Further, the 2'-modified nucleotide may be combined with a 4'-modified nucleotide or a 5'-modified nucleotide (for example, refer to Nucleic Acids Research, 2018, Vol. 46, No. 16, 8090-8104, or Nucleic Acids Research, 2020, Vol. 48, No. 18, 10101-10124).

As the 2'-modified nucleotide, for example, the 2'-OH group of ribose may be substituted with a substituent selected from the group consisting of a 2-(methoxy)ethoxy group, a 3-aminopropoxy group, a 2-[(N,N-dimethylamino)oxy]ethoxy group, a 3-(N,N-dimethylamino)propoxy group, a 2-[2-(N,N-dimethylamino)ethoxy]ethoxy group, a 2-(methylamino)-2-oxoethoxy group, a 2-(N-methylcarbamoyl)ethoxy group, and a 2-cyanoethoxy group.

Further, as the sugar-modified nucleotide, a bridged nucleic acid (BNA) having two cyclic structures by introducing a bridged structure into the sugar moiety can also be suitably used. Specific examples include Locked Nucleic Acid (LNA), in which the oxygen atom at the 2' position and the carbon atom at the 4' position are bridged via methylene [Tetrahedron Letters, 38, 8735 (1997) and Tetrahedron, 54, 3607 (1998)]; ethylene bridged nucleic acid (ENA) [Nucleic Acid Research, 32, e175 (2004)]; Constrained Ethyl (cEt) [The Journal of Organic Chemistry 75, 1569 (2010)]; Amido-Bridged Nucleic Acid (AmNA) [Chem Bio Chem 13, 2513 (2012)]; 2'-O,4'-C-spirocyclopropylene bridged nucleic acid (scpBNA) [Chem. Commun., 51, 9737 (2015)]; and tricycloDNA (tcDNA) [Nat. Biotechnol., 35, 238 (2017)].

As other sugar-modified nucleotides, Unlocked Nucleic Acid (UNA) [Mol. Ther. Nucleic Acids 2, e103 (2013)], GNA having a glycerol backbone [RNA. 2023 Apr; 29(4): 402-414.], ANA or FANA having an arabinose backbone [Nucleic Acids Res. 2006; 34(2): 451-461.], TNA having a threose backbone [J. Am. Chem. Soc. 2023, 145, 19691-19706], ANA having an altritol backbone [4028-4040, Nucleic Acids Research, 2020, Vol. 48, No. 8], HNA having an anhydrohexitol backbone or a cyclohexene backbone, CeNA [Chem. Commun., 2016, 52, 13467-13470], and the like are also suitably used. Among these, a bridged nucleic acid (BNA) is preferably used from the viewpoint of increasing the thermal stability of the double strand.

Furthermore, peptide nucleic acid (PNA) [Acc. Chem. Res., 32, 624 (1999)], oxy-peptide nucleic acid (OPNA) [J. Am. Chem. Soc., 123, 4653 (2001)], peptide ribonucleic acid (PRNA) [J. Am. Chem. Soc., 122, 6900 (2000)], and the like are also examples of sugar-modified nucleotides.

### <Phosphodiester Bond-Modified Nucleotide>

The phosphodiester bond-modified nucleotide may be any nucleotide in which a part or all of the chemical structure of the phosphodiester bond of the nucleotide is modified or substituted with any substituent, or substituted with any atom.

In the oligonucleotide according to one embodiment of the present invention, at least one of the nucleotides constituting the oligonucleotide is preferably a phosphodiester bond-modified nucleotide. Further, in the oligonucleotide according to one embodiment of the present invention, among all nucleotides constituting the oligonucleotide, the proportion occupied by phosphodiester bond-modified nucleotides may be 5% or more and 50% or less, 5% or more and 40% or less, 7% or more and 40% or less, 7% or more and 30% or less, 7% or more and 25% or less, 10% or more and 40% or less, 10% or more and 30% or less, 10% or more and 25% or less, 15% or more and 40% or less, 15% or more and 30% or less, or 15% or more and 25% or less. When the proportion of phosphodiester bond-modified nucleotides relative to all nucleotides constituting the oligonucleotide is within the above range, the efficiency of transfer of the oligonucleotide to each region of the brain and the efficacy of the oligonucleotide (for example, the efficiency of knocking down a target gene) are further improved.

Examples of phosphodiester bond-modified nucleotides include a nucleotide in which a phosphodiester bond is replaced with a phosphorothioate bond, a nucleotide in which a phosphodiester bond is replaced with a phosphorodithioate bond, a nucleotide in which a phosphodiester bond is replaced with an alkylphosphonate bond, a nucleotide in which a phosphodiester bond is replaced with a boranophosphate bond, a nucleotide in which a phosphodiester bond is replaced with an amide bond, a nucleotide in which a phosphodiester bond is replaced with a sulfonamide bond, a nucleotide in which a phosphodiester bond is replaced with a phosphonoacetate bond (PACE), a nucleotide in which a phosphodiester bond is replaced with a 2'-5' bond [Bioorg. Med. Chem. Lett. 16, 3238-3240], and a nucleotide in which a phosphodiester bond is replaced with a (mesyl)phosphoramidate bond [Nucleic Acids Research, 2021, Vol. 49, No. 16, 9026-9041]; preferable examples include a nucleotide in which a phosphodiester bond is replaced with a phosphorothioate bond. Replacement of phosphodiester bonds with these bonds further improves the efficiency of transfer of the oligonucleotide to each region of the brain and the efficacy of the oligonucleotide (for example, the efficiency of knocking down a target gene) .

The phosphodiester bond-modified nucleotide may be an optical isomer (Rp, Sp). Methods for selectively synthesizing optical isomers of phosphorothioate bonds are disclosed, for example, in J. Am. Chem. Soc., 124, 4962 (2002), Nucleic Acids Research, 42, 13546 (2014), and Science, 361, 1234 (2018).

### <Base-Modified Nucleotide>

The base-modified nucleotide may be any nucleotide in which a part or all of the chemical structure of the base of the nucleotide is modified or substituted with any substituent, or substituted with any atom.

Examples of the base-modified nucleotide include a nucleotide in which an oxygen atom in the base is substituted with a sulfur atom; a nucleotide in which a hydrogen atom in the base is substituted with an alkyl group having 1 to 6 carbon atoms, a halogen, or the like; a nucleotide in which a methyl group is substituted with hydrogen, a hydroxymethyl group, an alkyl group having 2 to 6 carbon atoms, or the like; and a nucleotide in which an amino group is substituted with an alkyl group having 1 to 6 carbon atoms, an alkanoyl group having 1 to 6 carbon atoms, an oxo group, a hydroxy group, or the like. Examples of base-modified nucleotides are disclosed in J. Org. Chem. 2011, 76, 7295-7300. Specifically, examples include 5-methylcytosine, in which the 5-position of cytosine is substituted with a methyl group.

### <Phosphate Group-Modified Nucleotide>

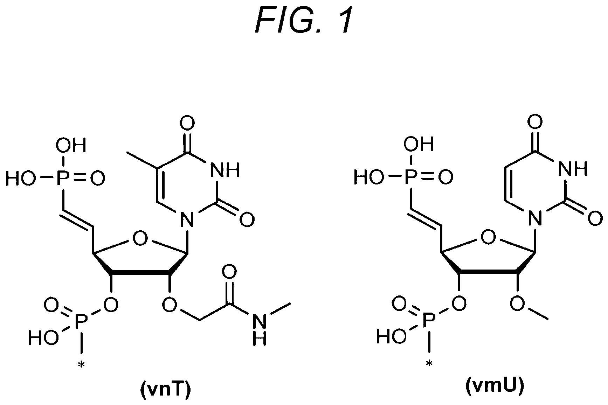

The phosphate group-modified nucleotide may be any nucleotide in which the phosphate group at the 5' position of the nucleotide is modified or substituted with any substituent, or substituted with any atom. However, substitution with a vinylphosphonate group, (PO(OH)₂(CH=CH-)), or (PO(OH)₂(CH₂CH₂)-) is more preferable. By being substituted with the modifying group, an effect of improving the knockdown efficiency of the target gene can be obtained. Specific examples of phosphate group-modified nucleotides include (vnT) and (vmU) illustrated in Fig. 1. Other phosphate group-modified nucleotides are disclosed in WO 2011/39699 and WO 2011/39702.

In the oligonucleotide according to one embodiment of the present invention, at least one of the nucleotides constituting the oligonucleotide is preferably a phosphate group-modified nucleotide. When the oligonucleotide has one or more phosphate group-modified nucleotides, the efficiency of transfer of the oligonucleotide to each region of the brain and the knockdown efficiency of the target gene are further improved.

Further, the oligonucleotide according to one embodiment of the present invention may include an oligonucleotide derivative described in WO 2018/199340. That is, the oligonucleotide according to one embodiment of the present invention may include an oligonucleotide derivative or a salt thereof that includes a cyclic oligonucleotide and a linear oligonucleotide, in which the cyclic oligonucleotide and the linear oligonucleotide have base sequences complementary to each other, and the cyclic oligonucleotide and the linear oligonucleotide form a complex via hydrogen bonds of the complementary base sequences. Further, the cyclic oligonucleotide may have a base length of 10 to 40 and may include at least one phosphorothioate bond. In addition, the base length of the cyclic oligonucleotide may be the same as the base length of the linear oligonucleotide, or may be longer than the base length of the linear oligonucleotide.

Further, the cyclic oligonucleotide may be represented by Formula 2. 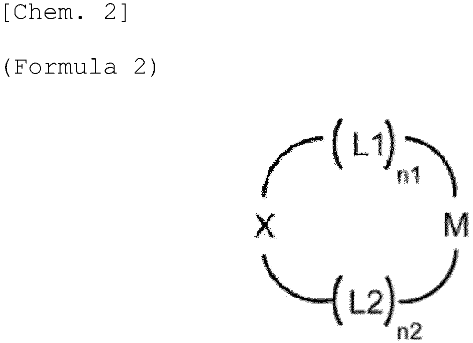 (In the formula, L1 and L2 represent linkers, n1 and n2 each independently represent an integer of 0 to 10, M represents a moiety containing a chemical structure cleaved by the intracellular environment, and X represents an oligonucleotide.)

### (Lipophilic Moiety)

The oligonucleotide according to one embodiment of the present invention preferably has one or more lipophilic moieties (also referred to as lipophilic groups). In other words, in the oligonucleotide according to one embodiment of the present invention, at least one of the nucleotides constituting the oligonucleotide is preferably a nucleotide or nucleotide derivative having a lipophilic moiety. When the oligonucleotide has one or more lipophilic moieties, it is expected that the efficiency of transfer of the oligonucleotide to each region of the brain and the knockdown efficiency of the target gene are further improved.

The lipophilic moiety is conjugated to a nucleotide or nucleotide derivative directly or via a linker. In the nucleotide or nucleotide derivative, the site to which the lipophilic moiety is conjugated may be any of the sugar moiety, base moiety, and phosphate moiety, but is preferably the sugar moiety or the phosphate moiety. Further, in the nucleotide or nucleotide derivative, when the lipophilic moiety is conjugated to the sugar moiety, the position at which the lipophilic moiety is conjugated is preferably the 2' position, the 3' position, or the 5' position.

Further, in the sequence of the oligonucleotide, the position of the conjugated nucleotide or nucleotide derivative is not particularly limited, and may be the 3' end, the 5' end, or any position from the 3' end to the 5' end of the oligonucleotide. When the oligonucleotide has a lipophilic moiety, for example, the target gene can be knocked down more effectively.

The term "lipophilic moiety" broadly refers to any compound or chemical substructure having affinity for lipids. Examples of methods for characterizing the lipophilicity of the lipophilic moiety include methods based on the octanol-water partition coefficient, logK_{ow}. Here, K_{ow} is the ratio of the concentration of a chemical substance in the octanol phase to the concentration thereof in the aqueous phase of a two-phase system at equilibrium. The octanol-water partition coefficient is a property of a substance measured in a laboratory. However, it can also be predicted by using coefficients attributed to constituent components of the chemical substance calculated using first-principles methods or empirical methods. In principle, a chemical substance is lipophilic when the logK_{ow} thereof is greater than 0. The logK_{ow} of the lipophilic moiety of the oligonucleotide according to one embodiment of the present invention is not particularly limited as long as the logK_{ow} is greater than 0, but may be greater than 1, greater than 2, or greater than 3. That is, the oligonucleotide according to one embodiment of the present invention has one or more lipophilic moieties, and the logK_{ow} of the lipophilic moiety may be greater than 0, greater than 1, greater than 2, or greater than 3.

The lipophilic moiety according to one embodiment of the present invention includes, for example, a lipid (a substituted or unsubstituted alkyl chain having 7 to 30 carbon atoms, or an alkyl chain having 7 to 30 carbon atoms substituted with an optional functional group selected from the group consisting of hydroxyl, amine, carboxylic acid, sulfonate, phosphate, thiol, azide, and alkyne), cholesterol, docosahexaenoic acid (DHA), eicosapentaenoic acid (EPA), arachidonic acid (ARA), retinoic acid, cholic acid, adamantaneacetic acid, 1-pyrenebutyric acid, dihydrotestosterone, 1,3-bis-O-(hexadecyl)glycerol, geranyloxyhexanol, hexadecylglycerol, borneol, menthol, 1,3-propanediol, a heptadecyl group, palmitic acid, myristic acid, O3-(oleoyl)lithocholic acid, O3-(oleoyl)cholenic acid, a sphingolipid, a fat-soluble vitamin, or phenoxazine. Among these, from the viewpoint of efficient uptake into cells, the lipophilic moiety according to one embodiment of the present invention is preferably at least one selected from the group consisting of a substituted or unsubstituted alkyl chain having 14 to 24 carbon atoms, eicosapentaenoic acid (EPA), arachidonic acid (ARA), and cholic acid.

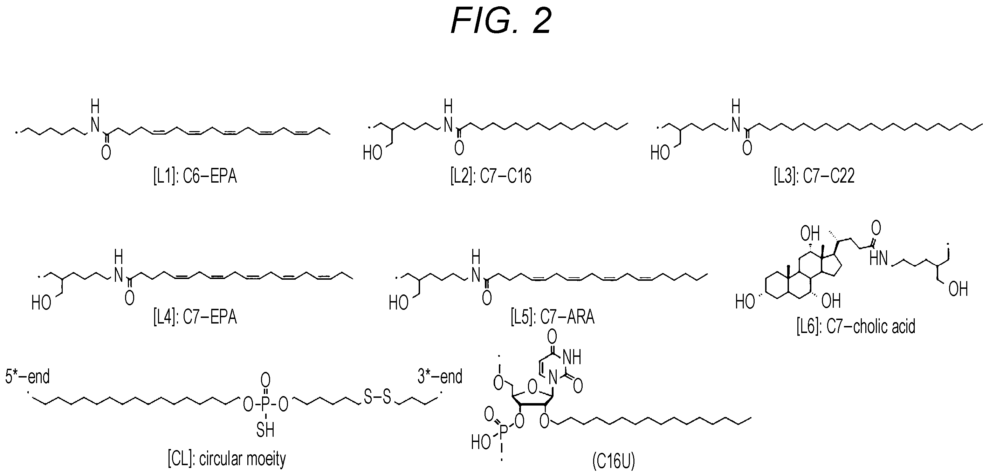

In the oligonucleotide according to one embodiment of the present invention, the lipophilic moiety may be directly bonded (conjugated) to a nucleotide. When directly conjugated, as illustrated by (C16U) in Fig. 2, the lipophilic moiety can be conjugated to the nucleotide via the OH group at the 2' position of the sugar moiety of the nucleotide. For example, a substituted or unsubstituted alkyl chain having 14 to 24 carbon atoms can be conjugated to a nucleotide via the OH group at the 2' position of the sugar moiety.

Further, in the oligonucleotide according to one embodiment of the present invention, the lipophilic moiety may be bonded via a linker. Here, the linker is not particularly limited as long as it can mediate bonding between the lipophilic moiety and the nucleotide, and examples thereof include those containing ether, thioether, urea, carbonate, amine, amide, maleimide-thioether, disulfide, phosphodiester, sulfonamide bond, a product of a click reaction (for example, triazole from azide-alkyne cycloaddition), or a carbamate. More specifically, for example, the linker can be formed using 5'-Amino-Modifier C6-PDA, 3'-PT-Amino-Modifier C6 CPG, or 3'-Amino-Modifier C7 CPG 1000 (all manufactured by Glen Research). [L1] shown in Fig. 2 is an example in which the lipophilic moiety is bonded to the nucleotide using 5'-Amino-Modifier C6-PDA, and [L2] to [L5] are examples in which the lipophilic moiety is bonded to the nucleotide using 3'-Amino-Modifier C7 CPG 1000.

### (Ligand)

The oligonucleotide according to one embodiment of the present invention may include a ligand. That is, the oligonucleotide according to one embodiment of the present invention may have at least one ligand. The ligand is not particularly limited as long as it is a molecule that binds to a biomolecule such as a protein, and examples thereof include a targeting ligand that targets a receptor mediating delivery to a specific central nervous system tissue. In addition, in the present specification, the above-described lipophilic moiety is excluded from the ligand.

### (SNCA-siRNA or HTT-siRNA)

The oligonucleotide according to one embodiment of the present invention is preferably siRNA targeting SNCA or HTT. The siRNA is not particularly limited as long as the siRNA targets SNCA or HTT, and those disclosed in the following international publications can be used.

As siRNA targeting SNCA (SNCA-siRNA), for example, those described in WO 2005/004794, WO 2006/039253, WO 2007/135426, WO 2008/086079, WO 2009/079399, WO 2012/027713, WO 2020/028816, and WO 2022/072447 can be used.

Further, as siRNA targeting HTT (HTT-siRNA), for example, those described in WO 2004/101787, WO 2005/027980, WO 2005/105995, WO 2007/051045, WO 2008/005562, WO 2016/161374, and WO 2021/087036 can be used.

Among these, the base sequences (S-0001 to 0196) of siRNAs composed of combinations of sense strands and antisense strands shown in Tables 3-1 to 3-5, and the oligonucleotides ds1, ds2, ds9, ds10, and ds17 to ds273 illustrated in Examples have high SNCA knockdown activity, and therefore can be more suitably used as the oligonucleotide (siRNA) according to one embodiment of the present invention.

From the viewpoint of higher SNCA knockdown activity, the oligonucleotide (siRNA) according to one embodiment of the present invention preferably includes any one pair of base sequences selected from the group consisting of the following (S1) to (S11), among the base sequences (S-0001 to S-0199) of siRNAs composed of combinations of sense strands and antisense strands shown in Tables 3-1 to 3-5, and more preferably includes any one pair of base sequences selected from the group consisting of (S6) to (S11). Here, "pair" means a combination of a sense strand and an antisense strand.
(S1) SEQ ID NO: 46 (sense strand) and SEQ ID NO: 213 (antisense strand) (S-0020)
(S2) SEQ ID NO: 83 (sense strand) and SEQ ID NO: 250 (antisense strand) (S-0057)
(S3) SEQ ID NO: 93 (sense strand) and SEQ ID NO: 260 (antisense strand) (S-0067)
(S4) SEQ ID NO: 169 (sense strand) and SEQ ID NO: 336 (antisense strand) (S-0143)
(S5) SEQ ID NO: 180 (sense strand) and SEQ ID NO: 347 (antisense strand) (S-0154)
(S6) SEQ ID NO: 1360 (sense strand) and SEQ ID NO: 1389 (antisense strand) (S-0179)
(S7) SEQ ID NO: 1366 (sense strand) and SEQ ID NO: 1395 (antisense strand) (S-0186)
(S8) SEQ ID NO: 1368 (sense strand) and SEQ ID NO: 1397 (antisense strand) (S-0187)
(S9) SEQ ID NO: 1372 (sense strand) and SEQ ID NO: 1401 (antisense strand) (S-0191)
(S10) SEQ ID NO: 1374 (sense strand) and SEQ ID NO: 1403 (antisense strand) (S-0193)
(S11) SEQ ID NO: 1756 (sense strand) and SEQ ID NO: 1759 (antisense strand) (S-0197)

An oligonucleotide including siRNA obtained by subjecting the base sequences of the above (S1) to (S11) to 2'-modified nucleotides can be more preferably used as the oligonucleotide according to one embodiment of the present invention. Among the oligonucleotides in Tables 1-1 to 1-6, oligonucleotides including siRNA obtained by subjecting the base sequences of (S1) to (S11) to 2'-modified nucleotides are shown below.
Modified siRNA of (S1): ds79, ds187
Modified siRNA of (S2): ds105, ds198, ds249, ds9
Modified siRNA of (S3): ds113, ds201, ds216, ds221, ds226, ds231, ds236, ds241, ds246
Modified siRNA of (S4): ds156, ds211, ds217, ds222, ds227, ds232, ds237, ds242
Modified siRNA of (S5): ds164, ds213, ds218, ds223, ds228, ds233, ds238, ds243, ds247
Modified siRNA of (S6): ds28, ds172, ds214, ds219, ds224, ds229, ds234, ds239, ds244
Modified siRNA of (S7): ds35, ds175, ds248
Modified siRNA of (S8): ds36, ds176
Modified siRNA of (S9): ds40, ds177, ds215, ds220, ds225, ds230, ds235, ds240, ds245
Modified siRNA of (S10): ds42, ds178

Furthermore, from the viewpoint of higher SNCA knockdown activity, the oligonucleotide (siRNA) according to one embodiment of the present invention more preferably includes the sequence of any one pair of oligonucleotides selected from the group consisting of ds2, ds9, ds17 to ds249, and ds254 to ds273 described in any of Tables 1-1 to 1-6.

Further, from the viewpoint of still more excellent SNCA knockdown activity, the oligonucleotide (siRNA) according to one embodiment of the present invention still more preferably includes the sequence of any one pair of oligonucleotides selected from the group consisting of ds9, ds28, ds35, ds36, ds40, ds42, ds79, ds105, ds113, ds156, ds164, ds172, ds175, ds176, ds177, ds178, ds187, ds198, ds201, ds211, and ds213 to ds246 described in any of Tables 1-1 to 1-6.

Further, from the viewpoint of having more excellent SNCA knockdown activity, the oligonucleotide (siRNA) according to one embodiment of the present invention is more preferably any one pair selected from the group consisting of ds2, ds17 to 249, and ds254 to ds273 described in any of Tables 1-1 to 1-6. Further, from the viewpoint of still more excellent SNCA knockdown activity, the oligonucleotide according to one embodiment is still more preferably any one pair selected from the group consisting of ds2 and ds250 to ds273 described in any of Tables 1-1 to 1-6, particularly preferably any one pair selected from the group consisting of ds9, ds28, ds35, ds36, ds40, ds42, ds79, ds105, ds113, ds156, ds164, ds172, ds175, ds176, ds177, ds178, ds187, ds198, ds201, ds211, and ds213 to ds246, and most preferably any one pair selected from the group consisting of ds2, ds254, ds257, ds258, ds259, ds261, ds263, ds267, ds268, and ds273.

Further, the oligonucleotide according to one embodiment of the present invention may have 80% or more sequence identity, 85% or more sequence identity, 90% or more sequence identity, or 95% or more sequence identity to any one pair of oligonucleotides selected from the group consisting of the oligonucleotides S-0001 to S-0196 shown in Tables 3-1 to 3-5, and the oligonucleotides ds1, ds2, ds9, ds10, and ds17 to ds273 illustrated in Examples.

Further, the oligonucleotide according to one embodiment of the present invention may have 80% or more sequence identity, 85% or more sequence identity, 90% or more sequence identity, or 95% or more sequence identity to any one pair of base sequences selected from the group consisting of (S1) to (S11) shown in Tables 3-1 to 3-5 described above. Further, the oligonucleotide according to one embodiment of the present invention may have 80% or more sequence identity, 85% or more sequence identity, 90% or more sequence identity, or 95% or more sequence identity to any one pair of base sequences selected from the group consisting of (S6) to (S11) shown in Tables 3-1 to 3-5 described above. Further, the oligonucleotide according to one embodiment of the present invention may have 80% or more sequence identity, 85% or more sequence identity, 90% or more sequence identity, or 95% or more sequence identity to siRNA obtained by subjecting the base sequences of (S1) to (S11) listed above to 2'-modified nucleotides.

Further, the oligonucleotide according to one embodiment may have 80% or more sequence identity, 85% or more sequence identity, 90% or more sequence identity, or 95% or more sequence identity to the sequence of any one pair of oligonucleotides selected from the group consisting of ds9, ds28, ds35, ds36, ds40, ds42, ds79, ds105, ds113, ds156, ds164, ds172, ds175, ds176, ds177, ds178, ds187, ds198, ds201, ds211, and ds213 to ds246 described in any of Tables 1-1 to 1-6.

Further, the oligonucleotide according to one embodiment may have 80% or more sequence identity, 85% or more sequence identity, 90% or more sequence identity, or 95% or more sequence identity to any one pair of sequences selected from the group consisting of ds2, ds17 to 249, and ds254 to ds273 described in any of Tables 1-1 to 1-6.

Further, the oligonucleotide according to one embodiment may have 80% or more sequence identity, 85% or more sequence identity, 90% or more sequence identity, or 95% or more sequence identity to any one pair of sequences selected from the group consisting of ds2 and ds250 to ds273 described in any of Tables 1-1 to 1-6. Further, the oligonucleotide according to one embodiment may have 80% or more sequence identity, 85% or more sequence identity, 90% or more sequence identity, or 95% or more sequence identity to any one pair of sequences selected from the group consisting of ds9, ds28, ds35, ds36, ds40, ds42, ds79, ds105, ds113, ds156, ds164, ds172, ds175, ds176, ds177, ds178, ds187, ds198, ds201, ds211, and ds213 to ds246. Further, the oligonucleotide according to one embodiment may have 80% or more sequence identity, 85% or more sequence identity, 90% or more sequence identity, or 95% or more sequence identity to any one pair of sequences selected from the group consisting of ds2, ds254, ds257, ds258, ds259, ds261, ds263, ds267, ds268, and ds273.

Here, "sequence identity" in the present specification indicates the degree to which a particular nucleic acid sequence matches another sequence at a certain ratio. Sequence identity is generally calculated using known algorithms such as Basic Local Alignment Search Tool (BLAST) or CLUSTALW. Specifically, the ratio (%) of matching bases or amino acids is calculated while taking into account gaps (insertions or deletions) between the sequences, which are comparison targets. In general, sequence identity is calculated by the following Math. 1. [Math. 1] 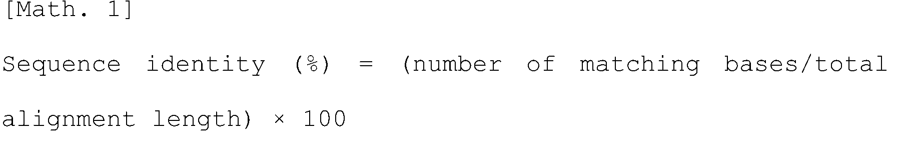 Sequence identity (%) = (number of matching bases/total alignment length) × 100

When the nucleic acid contained in the sequence is modified, the above sequence identity is calculated in the state before modification. For example, when "mN" or "fN" is present in the sequence, sequence identity is calculated with each regarded as N. Note that N is any of A, U, C, and G in the case of RNA, and any of A, T, C, and G in the case of DNA.

Further, the oligonucleotide according to one embodiment of the present invention may have a base sequence identical to a base sequence obtained by shortening the base sequence of the oligonucleotide disclosed in the present specification by 1 base, 2 bases, 3 bases, 4 bases, 5 bases, or 6 bases (hereinafter referred to as a shortened base sequence). Further, the oligonucleotide according to one embodiment of the present invention may have 85% or more sequence identity, 90% or more sequence identity, or 95% or more sequence identity to the shortened base sequence.

For example, when the number of bases of the oligonucleotide disclosed in the present specification is 21, the number of bases of the shortened base sequence is a base sequence of 20 bases, 19 bases, 18 bases, 17 bases, 16 bases, or 15 bases. Therefore, the number of bases of the oligonucleotide according to one embodiment of the present invention may be 20 bases, 19 bases, 18 bases, 17 bases, 16 bases, or 15 bases, and the base sequence of these oligonucleotides may have 85% or more sequence identity, 90% or more sequence identity, or 95% or more sequence identity to the shortened base sequence, or may be identical thereto.

Further, when the number of bases of the oligonucleotide disclosed in the present specification is 23, the number of bases of the shortened base sequence is a base sequence of 22 bases, 21 bases, 20 bases, 19 bases, 18 bases, or 17 bases. Therefore, the number of bases of the oligonucleotide according to one embodiment of the present invention may be 22 bases, 21 bases, 20 bases, 19 bases, 18 bases, or 17 bases, and the base sequence of these oligonucleotides may have 85% or more sequence identity, 90% or more sequence identity, or 95% or more sequence identity to the shortened base sequence, or may be identical thereto.

Here, the shortened base sequence may be one in which the 5' end and/or the 3' end of the oligonucleotide disclosed in the present specification is shortened, may be one in which the inside of the sequence is missing (deleted), or may be a combination thereof.

Further, the base sequences H-0001 to H-0258 of siRNAs composed of combinations of sense strands and antisense strands shown in any of Tables 4-1 to 4-6, and the sequences of any one pair of oligonucleotides selected from the group consisting of ds274 to ds461 described in any of Tables 2-1 to 2-4, have high HTT knockdown activity, and therefore can be more suitably used as the oligonucleotide according to one embodiment of the present invention.

From the viewpoint of higher HTT knockdown activity, the oligonucleotide (siRNA) according to one embodiment of the present invention preferably includes any one pair of base sequences selected from the group consisting of the following (H1) to (H5), among the base sequences (H-0001 to H-0258) of siRNAs composed of combinations of sense strands and antisense strands shown in Tables 4-1 to 4-6.
(H1) SEQ ID NO: 365 (sense strand) and SEQ ID NO: 623 (antisense strand) (H-0005)
(H2) SEQ ID NO: 370 (sense strand) and SEQ ID NO: 628 (antisense strand) (H-0010)
(H3) SEQ ID NO: 396 (sense strand) and SEQ ID NO: 654 (antisense strand) (H-0036)
(H4) SEQ ID NO: 407 (sense strand) and SEQ ID NO: 665 (antisense strand) (H-0047)
(H5) SEQ ID NO: 469 (sense strand) and SEQ ID NO: 727 (antisense strand) (H-0109)

An oligonucleotide including siRNA obtained by subjecting the base sequences of the above (H1) to (H5) to 2'-modified nucleotides can be more preferably used as the oligonucleotide according to one embodiment of the present invention. Among the oligonucleotides in Tables 2-1 to 2-4, oligonucleotides including siRNA obtained by subjecting the base sequences of (H1) to (H5) to 2'-modified nucleotides are shown below.
Modified siRNA of (H1): ds278, ds405, ds432, ds437, ds442, ds447, ds452, ds457
Modified siRNA of (H2): ds280, ds406, ds433, ds438, ds443, ds448, ds453, ds458
Modified siRNA of (H3): ds299, ds411, ds434, ds439, ds444, ds449, ds454, ds459
Modified siRNA of (H4): ds308, ds414, ds435, ds440, ds445, ds450, ds455, ds460
Modified siRNA of (H5): ds347, ds422, ds436, ds441, ds446, ds451, ds456, ds461

Further, from the viewpoint of higher HTT knockdown activity, the siRNA (oligonucleotide) targeting HTT preferably includes the sequence of any one pair of oligonucleotides selected from the group consisting of ds274 to ds461 described in any of Tables 2-1 to 2-4. Further, from the viewpoint of still more excellent HTT knockdown activity, it is still more preferable to include the sequence of any one pair of oligonucleotides selected from the group consisting of ds278, ds280, ds299, ds308, ds347, ds405, ds406, ds411, ds414, ds422, and ds432 to ds461 described in any of Tables 2-1 to 2-4.

Further, the oligonucleotide according to one embodiment of the present invention may have 80% or more sequence identity, 85% or more sequence identity, 90% or more sequence identity, or 95% or more sequence identity to any one pair of oligonucleotides selected from the group consisting of H-0001 to H-0258 shown in any of Tables 4-1 to 4-6, and ds274 to ds461 described in any of Tables 2-1 to 2-4.

Further, the oligonucleotide according to one embodiment of the present invention may have 80% or more sequence identity, 85% or more sequence identity, 90% or more sequence identity, or 95% or more sequence identity to any one pair of base sequences selected from the group consisting of (H1) to (H5) shown in Tables 4-1 to 4-6 described above. Further, the oligonucleotide according to one embodiment of the present invention may have 80% or more sequence identity, 85% or more sequence identity, 90% or more sequence identity, or 95% or more sequence identity to siRNA obtained by subjecting the base sequences of (H1) to (H5) listed above to 2'-modified nucleotides.

Further, the oligonucleotide according to one embodiment of the present invention may have 80% or more sequence identity, 85% or more sequence identity, 90% or more sequence identity, or 95% or more sequence identity to any one pair of oligonucleotides selected from the group consisting of ds274 to ds461 described in any of Tables 2-1 to 2-4.

Further, the oligonucleotide according to one embodiment of the present invention may have 80% or more sequence identity, 85% or more sequence identity, 90% or more sequence identity, or 95% or more sequence identity to any one pair of oligonucleotides selected from the group consisting of ds278, ds280, ds299, ds308, ds347, ds405, ds406, ds411, ds414, ds422, and ds432 to ds461 described in any of Tables 2-1 to 2-4.

In the present specification, the base sequences shown in the tables are written from left to right in the direction from the 5' end to the 3' end.

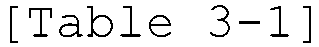

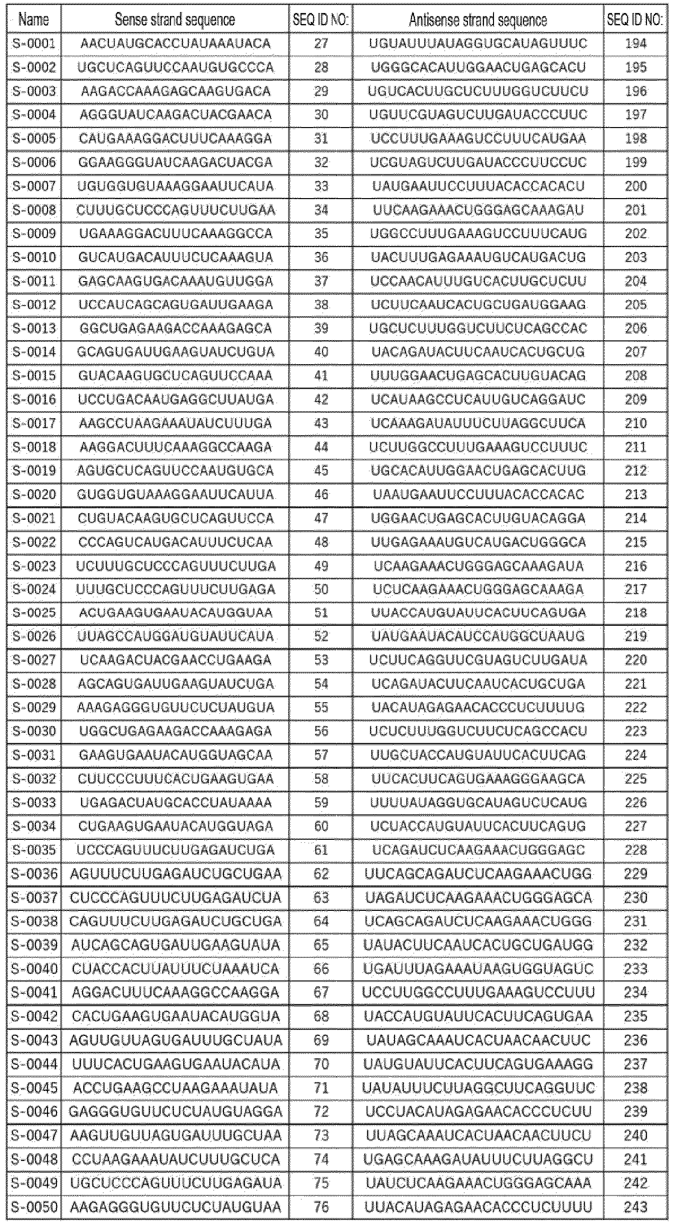

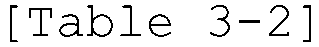

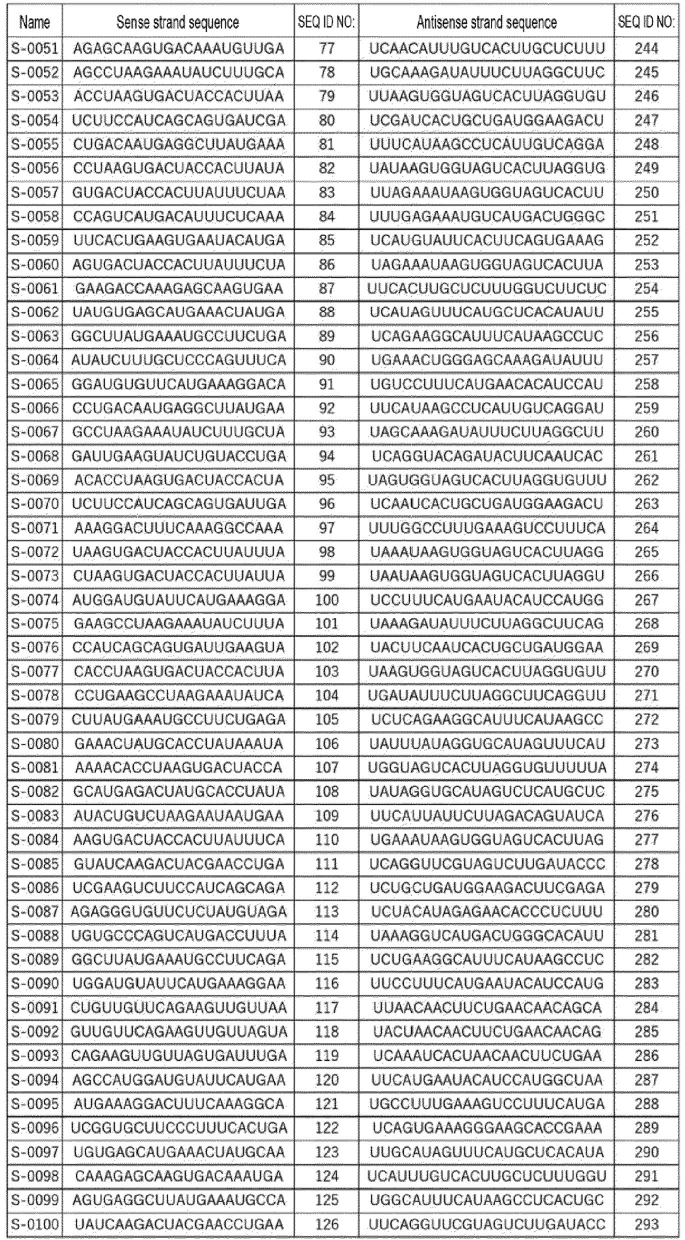

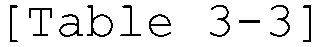

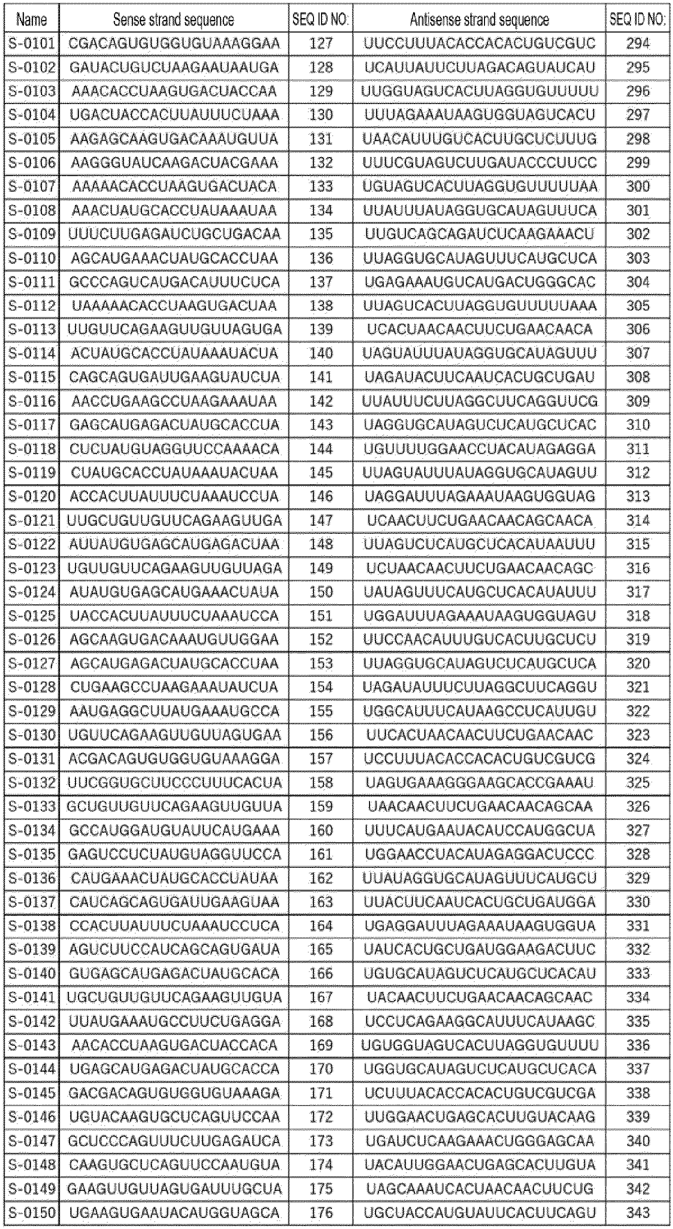

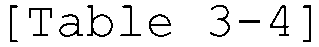

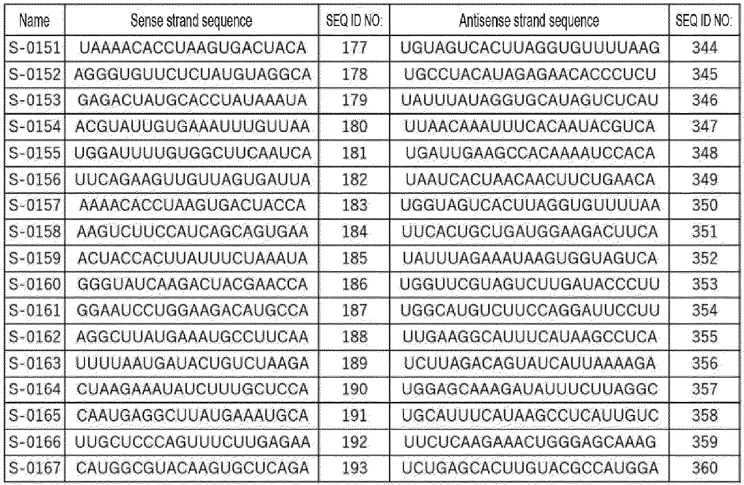

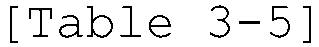

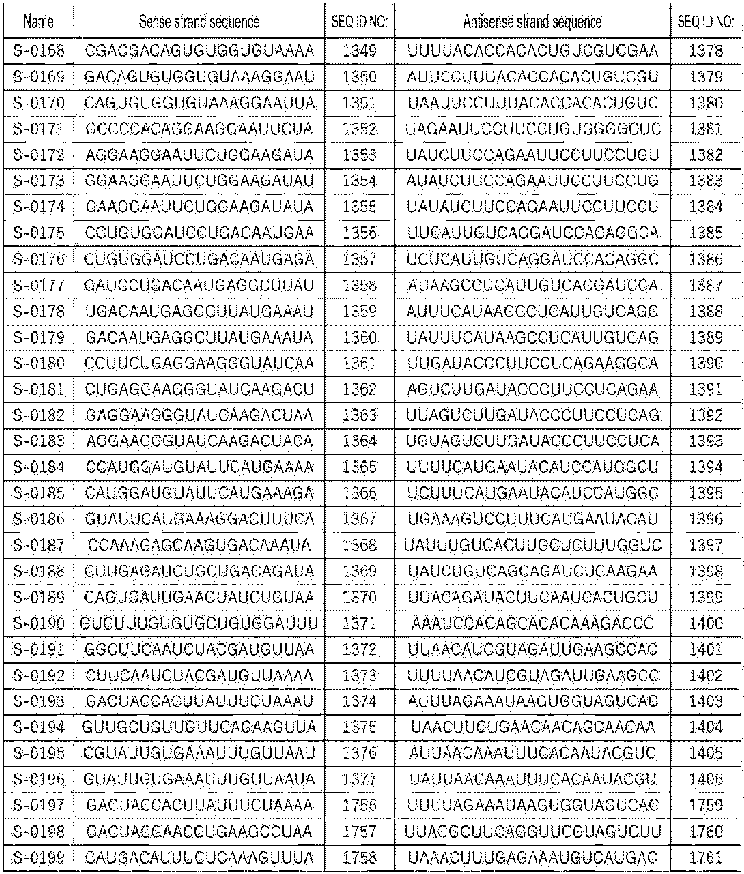

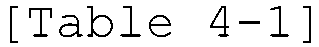

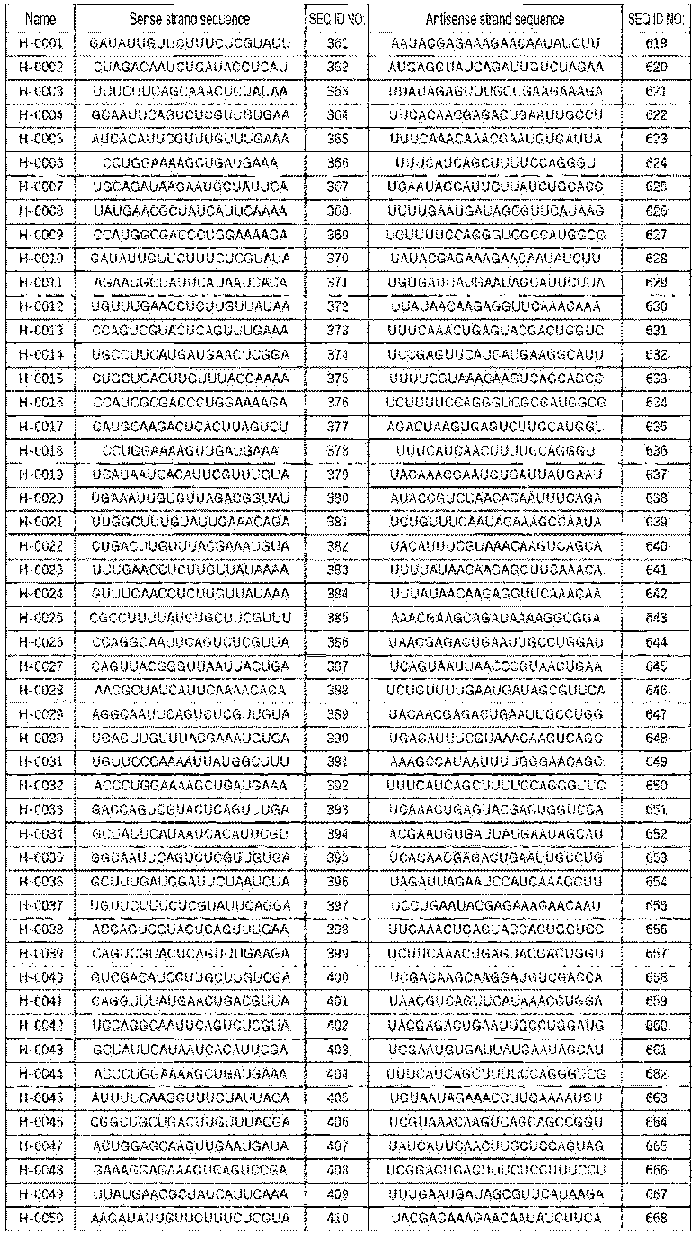

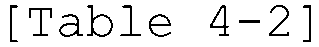

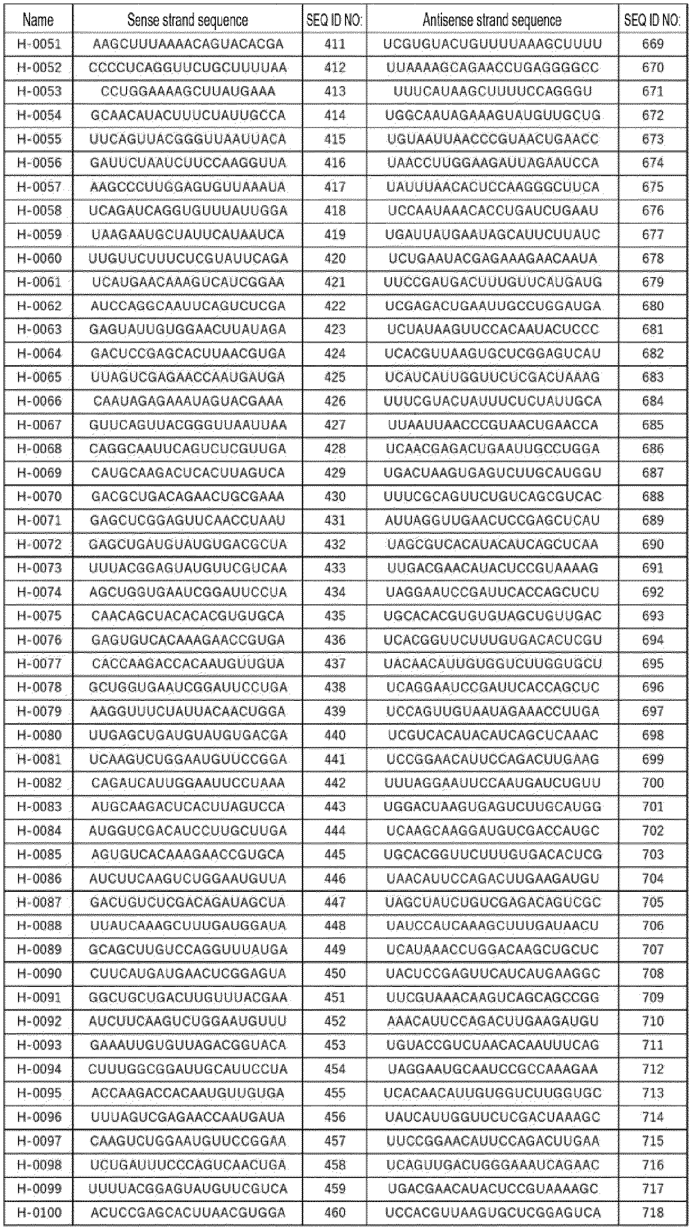

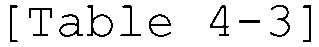

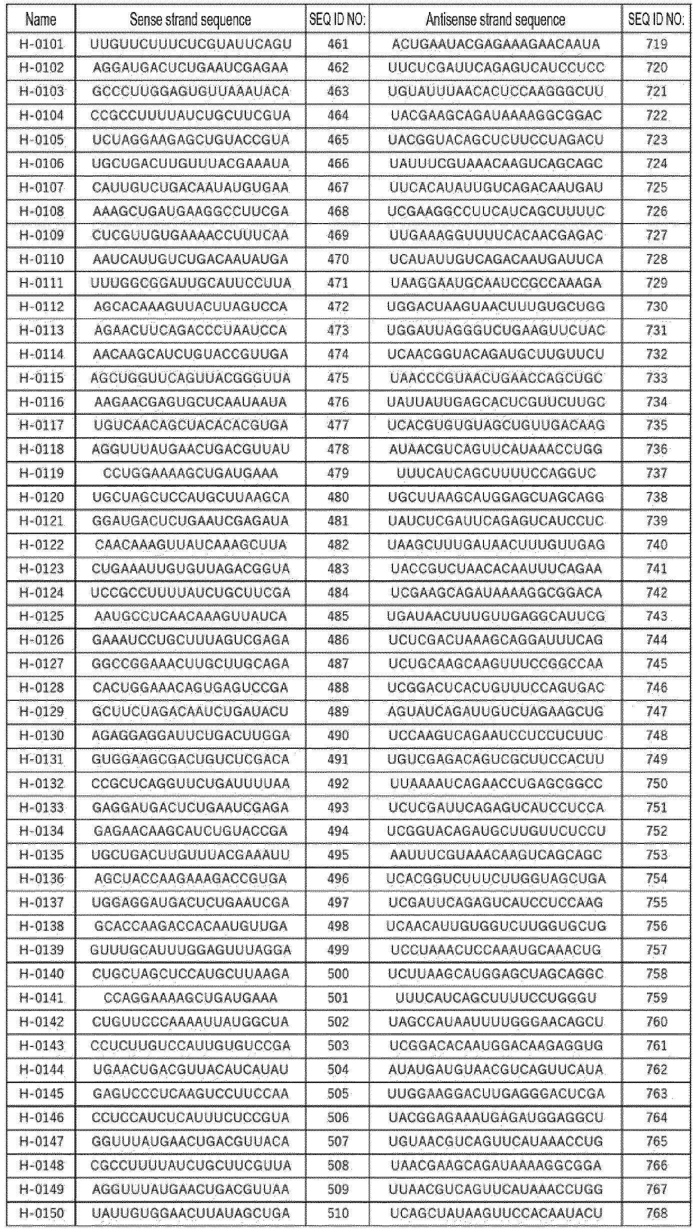

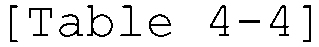

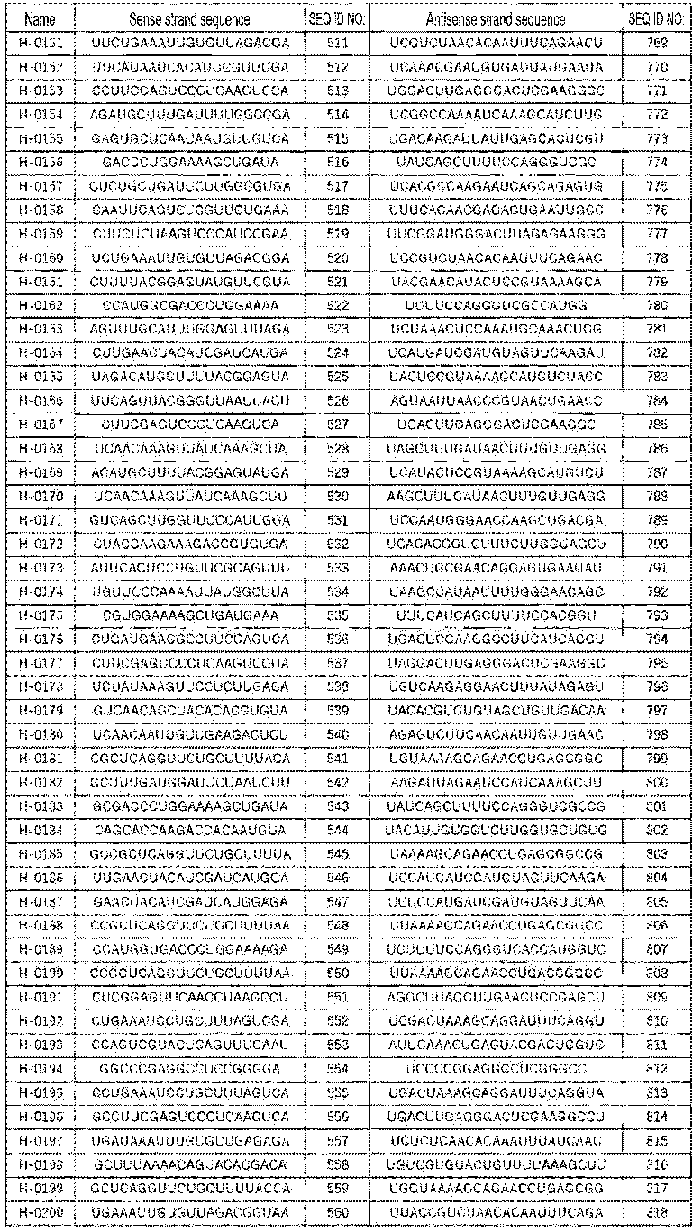

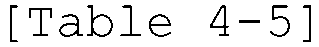

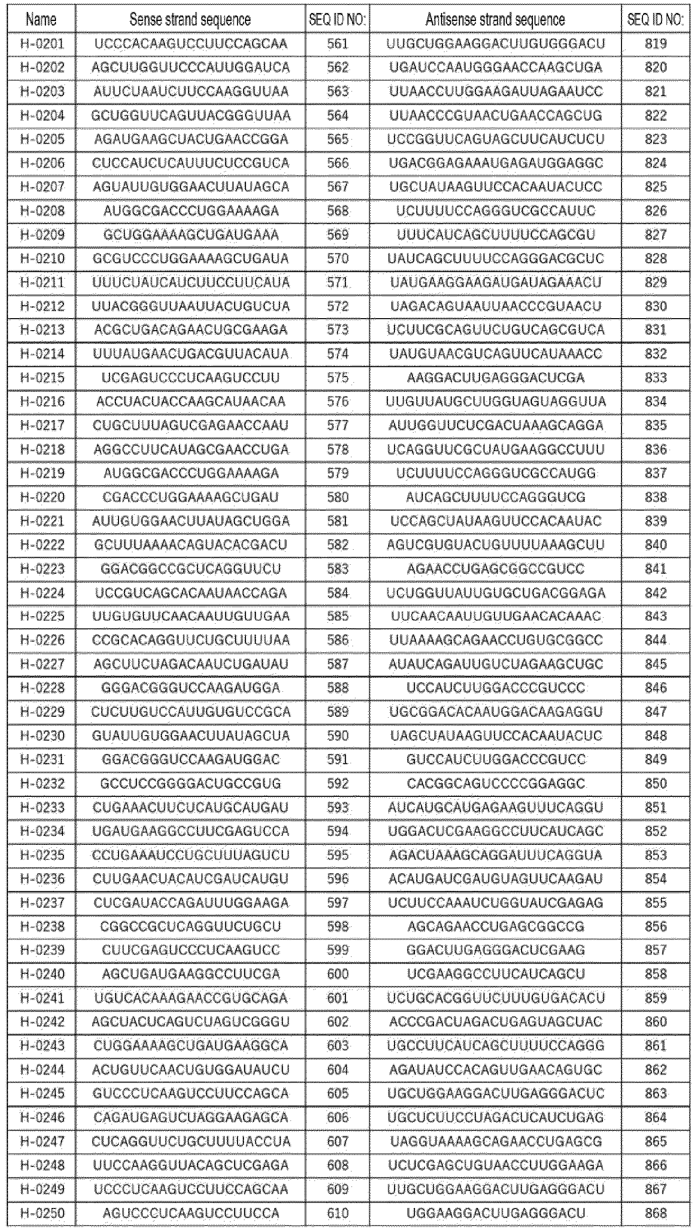

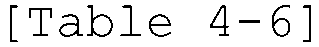

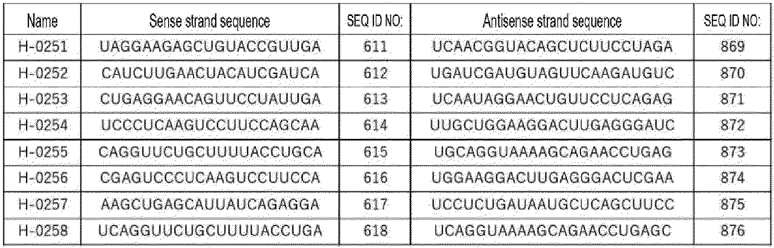

The above-described sugar-modified nucleotides, phosphodiester bond-modified nucleotides, base-modified nucleotides, phosphate group-modified nucleotides, and lipophilic moieties can be appropriately combined with these siRNAs. By combining these nucleotide derivatives and lipophilic moieties, the knockdown activity of siRNA and the transferred amount of siRNA to each region of the brain can be improved.

### (Antisense Oligonucleotide)

When the oligonucleotide according to one embodiment of the present invention is an antisense oligonucleotide, the gene targeted thereby is not particularly limited, and examples thereof include SOD1, FUS, C9ORF72, ATXN2, APP, tau, LRRK2, SNCA, HTT, ATXN3, a UBE3A antisense transcript, or SMN2, which are genes related to diseases of the central nervous system. Among these, at least one selected from the group consisting of the SNCA gene, the SOD1 gene, and the HTT gene is preferable.

In the oligonucleotide according to one embodiment of the present invention, the pattern in which sugar-modified nucleotides are disposed in the oligonucleotide (modification pattern) is not particularly limited, but those disclosed in WO 2004/015107, WO 2012/058210, WO 2013/074974, WO 2022/072447, WO 2021/087036, Mol. Ther. 2018, 26(3): 708-717, Nucleic Acids Research, 2022, Vol. 50, No. 9, 4840-4859, and the like can be preferably used.

### [Method for Producing Oligonucleotide]

The method for producing the oligonucleotide according to one embodiment of the present invention is not particularly limited as long as the oligonucleotide according to the present invention can be produced, and examples thereof include methods using known chemical synthesis, enzymatic transcription methods, and the like. Examples of the method using known chemical synthesis include the phosphoramidite method, the H-phosphonate method, the phosphite method, the phosphotriester method, and the phosphodiester method, and the oligonucleotide of the present invention can be synthesized, for example, using an ABI 3900 High-Throughput Nucleic Acid Synthesizer (manufactured by Applied Biosystems) or an nS-8 automated nucleic acid synthesizer (manufactured by GeneDesign Inc.). After synthesis is completed, cleavage from the solid phase, deprotection of protecting groups, purification of the target product, and the like are performed. It is desirable to obtain a nucleic acid having a purity of 90% or more, preferably 95% or more, by purification.

Further, an oligonucleotide including a nucleotide derivative can be prepared by subjecting a desired nucleotide derivative prepared in advance, such as a sugar-modified nucleotide, a phosphodiester bond-modified nucleotide, a base-modified nucleotide, or a phosphate group-modified nucleotide, to an extension reaction on a solid phase by the phosphoramidite method or the like. As a specific method for producing an oligonucleotide including a nucleotide derivative, the method described in Examples can be employed.

Here, the sugar-modified nucleotide, phosphodiester bond-modified nucleotide, base-modified nucleotide, and phosphate group-modified nucleotide can be synthesized according to known methods, or commercially available products may be used. For example, as the sugar-modified nucleotide, although not particularly limited, phosphoramidites of 2'-O-methyl-RNA and 2'-fluoro-DNA sold by Glen Research can be used.

Further, although the sugar-modified nucleotide, phosphodiester bond-modified nucleotide, base-modified nucleotide, and phosphate group-modified nucleotide are not particularly limited, they can be synthesized, for example, according to the methods described in Bioconjugate Chemistry (2020, 31, 5, 1213-1233) and Chemical Society Reviews (2021, 50, 5126-5164).

Further, a method for producing an oligonucleotide having a lipophilic moiety or ligand is not particularly limited, and an oligonucleotide having a lipophilic moiety or a ligand can be produced by preparing in advance a phosphoramidite unit or a solid-phase support having a linker or a lipophilic group (lipophilic moiety) and subjecting the phosphoramidite unit or the solid support to an elongation reaction on a solid phase together with another nucleotide or nucleotide derivative, or by post-modifying an oligonucleotide in a liquid phase after isolation of the oligonucleotide. The phosphoramidite unit having a lipophilic group (lipophilic moiety) is not particularly limited, but can be produced, for example, according to the methods described in WO 2021/092371 and Nature Biotechnology (40, 1500-1508, 2022). Further, post-modification is not particularly limited, but can be performed by coupling a desired lipophilic compound to the oligonucleotide using a coupling reagent. As specific methods for producing a phosphoramidite unit having a lipophilic group (lipophilic moiety) and an oligonucleotide having a lipophilic moiety, the methods described in Examples can be employed.

When the oligonucleotide according to one embodiment of the present invention is double-stranded, the synthesized and purified sense strand and antisense strand are mixed at an appropriate ratio, for example, 0.1 to 10 equivalents of the sense strand, preferably 0.5 to 2 equivalents, more preferably 0.9 to 1.1 equivalents, and still more preferably an equimolar amount (equal amount), relative to 1 equivalent of the antisense strand, and the resulting mixture is usually annealed. The mixture may be used directly without performing the annealing step.

Annealing may be performed under any conditions as long as the sense strand and the antisense strand can form a double strand, but is usually performed by mixing the antisense strand and the sense strand in substantially equimolar amounts, heating at about 85°C for about 5 minutes, and then allowing the mixture to cool to room temperature.

### <Pharmaceutical Composition and Therapeutic Agent for Central Nervous System Disease>

One aspect of the present invention may be a pharmaceutical composition containing the above oligonucleotide. In other words, the pharmaceutical composition according to one aspect of the present invention may contain the oligonucleotide according to one embodiment of the present invention as an active ingredient. Similarly to the oligonucleotide, the pharmaceutical composition is administered intranasally into the brain of a mammal using an administration device including a needle portion having a puncture portion described below, and is administered through an opening portion of the puncture portion in a state where the puncture portion is disposed within a cribriform foramen of the cribriform plate.

The pharmaceutical composition according to one embodiment of the present invention can be widely used for various diseases, and can be particularly suitably used for treatment, prevention, and/or amelioration of central nervous system diseases. That is, one aspect of the present invention may be a therapeutic agent for a central nervous system disease containing the above oligonucleotide. Here, the therapeutic agent means an agent used for treatment, prevention, and/or amelioration. Similarly to the oligonucleotide and the pharmaceutical composition described above, the therapeutic agent for a central nervous system disease is also administered intranasally into the brain of a mammal using an administration device including a needle portion having a puncture portion described below, and is administered through an opening portion of the puncture portion in a state where the puncture portion is disposed within a cribriform foramen of the cribriform plate.

The pharmaceutical composition and the therapeutic agent for a central nervous system disease according to one embodiment of the present invention can contain various components in addition to the oligonucleotide. For example, they can further contain a carrier effective for transferring the oligonucleotide into cells. Examples of the carrier effective for transferring the oligonucleotide into cells include a cationic carrier. Examples of cationic carriers include a cationic liposome and a cationic polymer. Further, as the carrier effective for transferring the oligonucleotide into cells, a carrier using a viral envelope may be used.

A pharmaceutical composition and therapeutic agent for a central nervous system disease containing the oligonucleotide and the above carrier can be prepared by methods known to those skilled in the art. For example, the pharmaceutical composition or the therapeutic agent for a central nervous system disease of the present invention can be prepared by mixing a carrier dispersion having an appropriate concentration with an oligonucleotide solution.

Further, as the pharmaceutical composition and therapeutic agent for a central nervous system disease according to one embodiment of the present invention, for example, composite particles containing an oligonucleotide and lead particles as constituent components, and a composition composed of the composite particles and, if desired, a lipid membrane coating the composite particles, are also suitably used. Examples of the lead particle include a lipid assembly, a liposome, an emulsion particle, a polymer, a metal colloid, and a microparticle formulation, preferably, a liposome is used, and more preferably, a cationic liposome is used.

The pharmaceutical composition and the therapeutic agent for a central nervous system disease according to one embodiment of the present invention may contain a pharmaceutically acceptable carrier, diluent, or the like in addition to the above carrier. The pharmaceutically acceptable carrier, diluent, or the like is an essentially chemically inert and harmless compound (including a composition), and does not affect the biological activity of the pharmaceutical composition or the therapeutic agent of the present invention. Examples of pharmaceutically acceptable carriers or diluents include, but are not limited to, water, a saline solution, a sugar solution, a glycerol solution, and ethanol.

The pharmaceutical composition and the therapeutic agent for a central nervous system disease according to one embodiment of the present invention preferably contain an amount of the oligonucleotide effective for treatment, prevention, or amelioration of a disease, and are provided in an aspect that can be appropriately administered intranasally to a patient, and a liquid formulation is suitable as such an aspect.

Further, the pharmaceutical composition and the therapeutic agent for a central nervous system disease of the present invention may contain an appropriate amount of any pharmaceutically acceptable additive, for example, an emulsification aid, a stabilizer, an isotonic agent, and/or a pH adjuster. Any pharmaceutically acceptable additive can be added at an appropriate step before or after preparation of the pharmaceutical composition or the therapeutic agent for a central nervous system disease of the present invention.

### [Administration Device and Administration System]

The oligonucleotide according to one embodiment of the present invention is administered intranasally into the brain of a mammal using an administration device including a needle portion having a puncture portion, and is administered through an opening portion of the puncture portion in a state where the puncture portion is disposed within a cribriform foramen of the cribriform plate. The administration device includes a needle portion having a puncture portion, and enables the oligonucleotide to be administered intranasally into the brain of a mammal by administering the oligonucleotide through the opening portion of the puncture portion in a state where the puncture portion is disposed within a cribriform foramen of the cribriform plate.

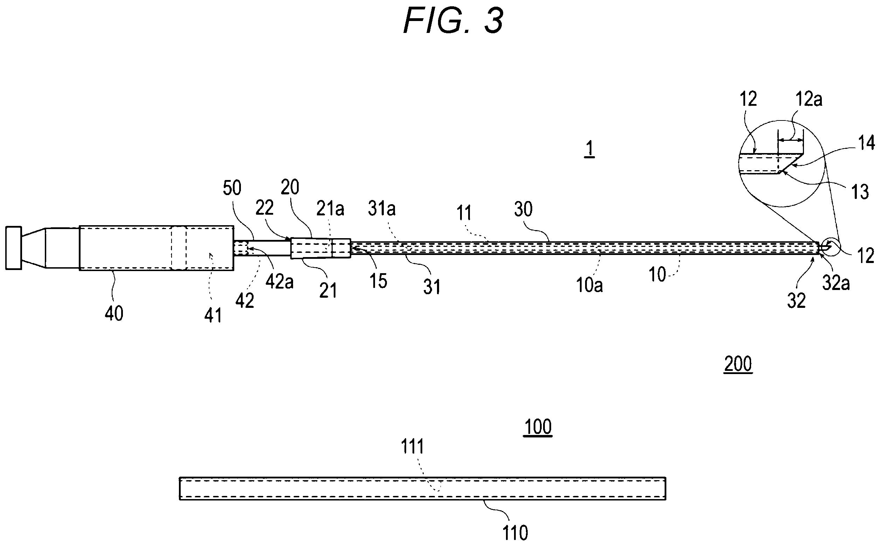

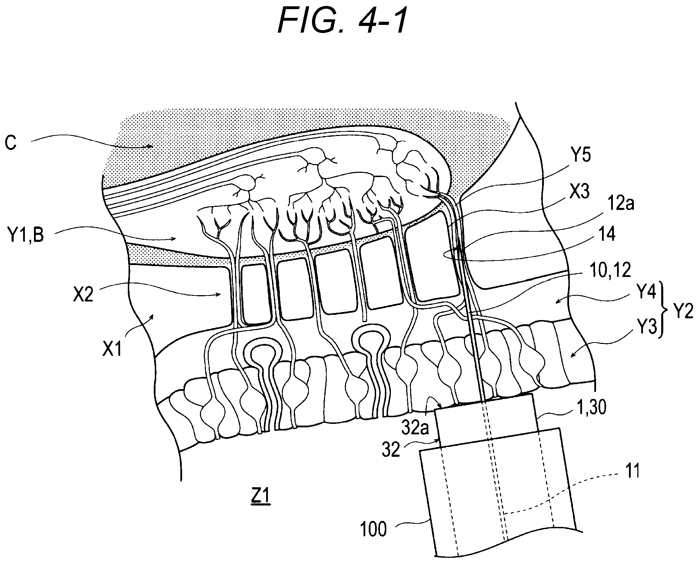

Hereinafter, an administration device 1 and an administration system 200 including the administration device will be described with reference to Figs. 3 and 4-1, but the administration device 1 and the administration system 200 are not limited to the embodiments of Figs. 3 and 4-1.

In addition, in the present specification, the "cribriform foramen X3" punctured by a puncture portion 12 of a needle portion 10 of the administration device 1 is a hole formed in a cribriform plate X2 of an ethmoid bone X1 having a nasal-cavity-side opening portion and an olfactory-bulb-side opening portion as illustrated in Fig. 4-1, and an olfactory nerve (nerve axon) Y5 extending from an olfactory bulb Y1, which is a part of a brain tissue B, to olfactory cells distributed in an olfactory mucosa Y2 (composed of an olfactory epithelium Y3 and a lamina propria Y4) in a nasal cavity Z1 passes therethrough. A large number of cribriform foramina X3 are present not only on the flat surface of the cribriform plate X2 as illustrated in Fig. 4-1, but also in the midline, the lateral wall, the posterior wall of the olfactory fossa, and the like. The cribriform foramen X3, which is a puncture target of the needle portion 10 of the present device, is any hole, among all of these holes, that can be punctured by the puncture portion 12 having penetrated through the olfactory mucosa Y2.

### (Administration System)

As illustrated in Fig. 3, the administration system 200 is configured to include the administration device 1 and a guide catheter 100.

The administration system 200, as illustrated in Fig. 4-1, administers an oligonucleotide A into the brain in a state where the distal end side of the guide catheter 100 is disposed within the nasal cavity Z1 in a posture of being oriented toward the cribriform plate X2, and the administration device 1 is inserted into the guide catheter 100 and the opening portion (distal end opening portion 14) of the puncture portion 12 of the needle portion 10 is disposed within the cribriform foramen X3 formed in the cribriform plate X2. The administration system 200 can deliver the oligonucleotide A intranasally into the brain of a mammal such as a human via a delivery medium such as a cerebrospinal fluid C or the olfactory nerve Y5. That is, the administration system 200 includes an accommodation portion 40 accommodating the oligonucleotide of the present invention and the administration device 1, and is configured such that the oligonucleotide accommodated in the accommodation portion 40 is dischargeable through the opening portion (distal end opening portion 14) of the puncture portion 12.

### (Administration Device)

As illustrated in Fig. 3 or 4-1, the administration device 1 is configured to include the needle portion 10, a hub portion 20, a cannula portion 30, and the accommodation portion 40.

The administration device 1 is an administration device 1 including the needle portion 10 having the puncture portion 12 for delivering the oligonucleotide A intranasally into the brain of a mammal, and administers the oligonucleotide A through the distal end opening portion 14 by disposing the opening portion (distal end opening portion 14) of the puncture portion 12 within the cribriform foramen X3.

The administration device 1 can be used in combination with the guide catheter 100 as the administration system 200, or can be used alone.

### <Needle Portion>

The needle portion 10 has a needle shaft portion 11, a puncture portion 12 formed on the distal end side of the needle shaft portion 11, a distal end opening portion 14 formed in a needle tip portion 12a of the puncture portion 12 (corresponding to the "opening portion" in the claims), and a proximal end opening portion 15 formed at the proximal end of the needle shaft portion 11. The needle portion 10 has a cylindrical shape in which a lumen 10a penetrating in the longitudinal direction is formed from the distal end opening portion 14 formed on the distal end side to the proximal end opening portion 15 formed on the proximal end side. As illustrated in Fig. 3, in the needle portion 10, the needle shaft portion 11 is disposed within a lumen 31a of the cannula portion 30, and at the time of puncture, the puncture portion 12 is disposed to be exposed from the distal end of the cannula portion 30.

The needle shaft portion 11 corresponds to a main body portion of the needle portion 10 and has a lumen 21a communicating with the hub portion 20 or the lumen 31a of the cannula portion 30. The proximal end opening portion 15 is formed at the proximal end of the needle shaft portion 11. The needle shaft portion 11 is communicably connected to the hub portion 20 through the proximal end opening portion 15.

The needle shaft portion 11 is formed to have a length substantially equal to the total length of the cannula portion 30, is inserted through the lumen 31a of the cannula portion 30, and is connected to the hub portion 20 such that the proximal end opening portion 15 communicates with the lumen 21a of the hub portion 20. However, the needle shaft portion 11 may be shorter than the total length of the cannula portion 30, and the proximal end may be disposed in the lumen 31a of the cannula portion 30. When configured in this manner, the oligonucleotide A flows through the lumen 31a of the cannula portion 30 to the proximal end opening portion 15.

The puncture portion 12 is formed on the distal end side of the needle shaft portion 11. The needle tip portion 12a is formed on the distal end side of the puncture portion 12. The needle tip portion 12a has an open end provided with a blade surface 13 formed by cutting the needle shaft portion 11 obliquely with respect to the longitudinal direction on the distal end side. An inner edge of the blade surface 13 defines the distal end opening portion 14 that communicates the lumen of the needle shaft portion 11 with the outside. At the time of administration of the oligonucleotide A, the distal end opening portion 14 is disposed within the cribriform foramen X3 in a state where at least a part of the puncture portion 12 is punctured into the cribriform foramen X3. By being disposed within the cribriform foramen X3, the distal end opening portion 14 can deliver the oligonucleotide A toward the brain against the cerebrospinal fluid C flowing out from the cribriform foramen X3. In addition, the needle tip portion 12a is not limited to a pointed shape having the blade surface 13 at the distal end, and may have a straight tubular shape or a tubular shape having a substantially hemispherical rounded distal end.

From the viewpoints of ease of puncture into the cribriform foramen X3 and liquid feedability of the oligonucleotide A, the puncture portion 12 preferably has an outer diameter of 0.05 mm or more and 2.1 mm or less, and more preferably 0.075 mm or more and 1.2 mm or less. The total length of the puncture portion 12, which is the axial length of the blade surface 13 illustrated in Fig. 3 (corresponding to the exposed length of the needle portion 10 from the distal end of the cannula portion 30), has a length that allows the distal end opening portion 14 to be disposed at least within the cribriform foramen X3, and is preferably 0.25 mm or more and 5.4 mm or less. The total length of the puncture portion 12 is the length in the major-axis direction of the portion exposed from the cannula portion 30 of the needle portion 10, and is the length from the most distal end of the needle tip portion 12a along the major-axis direction of the needle portion 10. The length of the puncture portion 12 can be appropriately set according to the thickness of the olfactory mucosa Y2 of the mammal, which is a puncture target, the total length of the cribriform foramen X3, and the like.

The needle portion 10 can be formed of a metal such as stainless steel (for example, SUS304 or SUS316L) or titanium, a resin material, or the like. However, the constituent material of the needle portion 10 is not particularly limited to the above-described materials, as long as the material can be used in the medical field and is suitable for the needle portion 10.

In addition, in order to improve ease of puncture into the cribriform foramen X3, the needle portion 10 may be formed of a plastically deformable material or a shape-memory material, and may be used in an aspect in which the needle shaft portion 11 is deformed by changing the orientation of the puncture portion 12 when the device is used. At this time, the shape of the needle portion 10 may be deformed into any shape immediately before use, may be deformed in advance, or may be further finely adjusted at the time of use in a state of being deformed in advance.

In addition, the needle portion 10 may use different materials for the puncture portion 12 exposed from the cannula portion 30 and for the needle shaft portion 11. For example, the needle portion 10 may be configured such that the puncture portion 12 is formed of a material having rigidity that does not deform or is difficult to deform in order to puncture the olfactory mucosa or the like, and the needle shaft portion 11 is formed of a material deformable in accordance with the shape or the like of the guide catheter 100.

### <Hub Portion>

The hub portion 20 holds the proximal end of the needle shaft portion 11 of the needle portion 10 and/or the proximal end of the cannula portion 30 such that the oligonucleotide A can flow therethrough. The hub portion 20 has a main body portion 21 and a connection portion 22. The hub portion 20 is connected to the accommodation portion 40 in a state where the distal end opening portion 42a of the accommodation portion 40 and the proximal end opening portion 15 of the needle portion 10 are in communication with each other.

The main body portion 21 has the lumen 21a through which the oligonucleotide A can flow, and causes the distal end opening portion 42a of the connected accommodation portion 40 to communicate with the proximal end opening portion 15 of the needle shaft portion 11 of the needle portion 10 or the lumen 31a of the cannula portion 30.

The connection portion 22 is provided on the proximal end side of the hub portion 20, is connected to the accommodation portion 40, and maintains a communication state between the hub portion 20 and the accommodation portion 40. In the present embodiment, the connection portion 22 is in an aspect of being connected to the accommodation portion 40 via a connection member 50 such as a tube. However, the connection portion 22 may be in an aspect in which both the connection portion 22 and the accommodation portion 40 are directly and detachably fitted to each other in accordance with the shape of the distal end side of the accommodation portion 40 (for example, a luer taper type or a luer lock type). The connection portion 22 is not particularly limited as long as the connection portion 22 is in an aspect in which at least the accommodation portion 40 and the hub portion 20 are connected such that the oligonucleotide A can flow therebetween.

### <Cannula Portion>

The cannula portion 30 is a tubular member formed of a flexible material, and has a tubular main body portion 31 in which the lumen 31a communicating from the distal end to the proximal end is formed. As one example, the cannula portion 30 holds at least a part of the needle shaft portion 11 of the needle portion 10.

A stopper portion 32 having an abutment portion 32a that comes into contact with the olfactory epithelium Y3 of the olfactory mucosa Y2 is provided at the distal end of the main body portion 31 of the cannula portion 30. In the aspect illustrated in Fig. 3, the stopper portion 32 is a distal end part of the main body portion 21, and the distal end surface of the main body portion 31 functions as the abutment portion 32a. In the present embodiment, the stopper portion 32 is the distal end part of the main body portion 31, and the abutment portion 32a is the distal end surface of the main body portion 31. However, the stopper portion 32 and the abutment portion 32a are not limited to these configurations. The stopper portion 32 may be constituted by a detachable or fixed separate member that can be disposed on the distal end side of the cannula portion 30, and the abutment portion 32a may be constituted by a portion of the separate member that comes into contact with the olfactory epithelium Y3.

When the puncture portion 12 of the needle portion 10 punctures the cribriform foramen X3, the stopper portion 32 functions as a stopper that prevents the needle tip portion 12a of the puncture portion 12 from puncturing excessively deeply by bringing the abutment portion 32a into contact with the olfactory epithelium Y3 as illustrated in Fig. 4-1.

Further, during puncture of the needle portion 10, the stopper portion 32 can stabilize the puncture posture of the administration device 1 by bringing the abutment portion 32a into contact with the olfactory epithelium Y3. Accordingly, the needle portion 10 can puncture the cribriform foramen X3, which is a puncture target, without positional displacement. In addition, the stopper portion 32 is preferably in a state of being pressed to slightly sink into the olfactory epithelium Y3 such that the puncture posture of the administration device 1 is fixed.

In the stopper portion 32, when the abutment portion 32a is the distal end surface of the cannula portion 30, in a case where the cross-sectional shape thereof is substantially circular (such as elliptical) close to the structure of the upper nasal cavity, the length of the longest portion in the radial direction (maximum width) of the distal end surface of the cannula portion 30 has a minor axis of 0.2 mm or more and 3.0 mm or less and a major axis of 0.2 mm or more and 15 mm or less. In the abutment portion 32a, when the cross-sectional shape of the cannula portion 30 is circular, the diameter is preferably 0.2 mm or more and 3.0 mm or less, and more preferably 0.2 mm or more and 2.1 mm or less. The maximum width of the abutment portion 32a can be appropriately set to be at least larger than the diameter of the needle portion 10 such that the stopper function of preventing over-puncture of the needle portion 10 into the cribriform foramen X3 is exhibited.

The cannula portion 30 may be formed in a straight tubular shape, but from the viewpoint of ease of puncture of the needle portion 10 into the cribriform foramen X3, the cannula portion 30 may have a shape in which a part thereof is curved in advance. In addition, from the viewpoints of ease of insertion into the nasal cavity Z1 and ease of puncture of the needle portion 10 into the cribriform foramen X3, the cannula portion 30 may be formed of a material that allows a part or all of the main body portion 31 to be plastically deformed into any shape.
Thus, when an insertion aid such as the guide catheter 100 for guiding the administration device 1 to the olfactory mucosa Y2 is used, the cannula portion 30 can be inserted while being deformed along the lumen shape of the guide catheter 100.

The length of the cannula portion 30 is not particularly limited as long as the cannula portion 30 has a length that allows the proximal end to be exposed from the external naris and the distal end has a length that allows insertion to the olfactory mucosa Y2 in the vicinity of the cribriform plate X2 and operation of the needle tip portion 12a, but for example, the total length may be 25 mm or more and 2000 mm or less, 30 mm or more and 1500 mm or less, 40 mm or more and 1000 mm or less, or 55 mm or more and 410 mm or less.

The cannula portion 30 may be disposed to cover the total length of the needle shaft portion 11 of the needle portion 10, or may be disposed in a state where the proximal end side of the needle shaft portion 11 is exposed.

The cannula portion 30 may be configured to be disposed integrally with the hub portion 20, or may be configured to be attachable to and detachable from the hub portion 20.

### <Accommodation Portion>

The accommodation portion 40 accommodates the oligonucleotide A to be administered to the cribriform foramen X3. The accommodation portion 40 has an accommodation space 41 that accommodates the oligonucleotide A and a liquid feeding portion 42 that sends the oligonucleotide A in the accommodation space 41 to the hub portion 20. In Fig. 3, the accommodation portion 40 is connected to the hub portion 20 via the connection member 50 such as a tube such that the oligonucleotide A can flow therethrough.

The accommodation portion 40 is communicably connected to the needle portion 10 via the hub portion 20. A distal end opening portion 42a of the liquid feeding portion 42 formed at the distal end of the liquid feeding portion 42 communicates with the proximal end opening portion 15 of the needle portion 10 through the lumen 21a of the hub portion 20. As a result, the oligonucleotide A accommodated in the accommodation portion 40 can flow through the needle shaft portion 11. The accommodation portion 40 is preferably configured such that the administration amount of the oligonucleotide A accommodated in the accommodation space 41 can be adjusted, and for example, a syringe having a plunger can be used. In addition, the accommodation portion 40 is not limited to a syringe, and may be any instrument that at least accommodates the oligonucleotide A and allows the oligonucleotide A to flow to the needle portion 10 through the hub portion 20. Further, the accommodation portion 40 may be connected such that the liquid feeding portion 42 and the hub portion 20 can communicate with each other in a state where the accommodation portion 40 is attached to a medical device having an operation portion capable of controlling the discharge amount, discharge timing, and the like of the oligonucleotide A.

### <Guide Catheter>

The guide catheter 100 is used as an insertion aid when the administration device 1 is inserted into the nasal cavity Z1.

As illustrated in Fig. 3, the guide catheter 100 has a catheter main body 110 constituted by a tubular member having a lumen 111 that penetrates in the longitudinal direction from the distal end to the proximal end.

The guide catheter 100 can be formed of a material such as a metal or resin usable in the medical field, and stainless steel such as SUS304 or polyurethane can be used as one example.

The distal end portion of the catheter main body 110 may be provided with a curved portion that is disposed adjacent thereto and curved to move away from the axis of the catheter main body 110 toward the distal end side. In addition, the guide catheter 100 may also be subjected to processing such as a spiral cut in the circumferential direction and various surface treatments for improving insertability, on the outer surface thereof.

As illustrated in Fig. 3, the catheter main body 110 may be used in a straight state, may be configured to be pre-shaped to be easily inserted into the nasal cavity Z1, or may be configured to be plastically deformable by fine adjustment before or during use.

Here, examples of the shape of the guide catheter 100 will be described. The guide catheter 100 can have a total length of 90 mm, an outer diameter of φ0.82 mm, an inner diameter of φ0.68 mm, and a bending angle of the curved portion 113 of 45°. Further, the guide catheter 100 can be a double lumen having a total length of 45 mm, an outer diameter of φ1.35 mm, and inner diameters of φ0.45 mm and φ0.70 mm.

The guide catheter 100 can be supplied to the market as the administration system 200 in combination with the administration device 1. In addition, the administration device 1 or the guide catheter 100 can be made to function as the administration system 200 by using either of the devices of the administration device 1 or the guide catheter 100, each of which has already been supplied as a single device.

### [Method of Using Device]

Next, a method of using the above-described administration device 1 will be described. The method of use described below includes a procedure corresponding to a preparation stage up to placing the administration device 1 at a predetermined position and starting administration of the oligonucleotide, and a procedure for administering the oligonucleotide after the preparation stage. A method of using the administration device 1 includes: a step of inserting the administration device 1 from at least the external naris, puncturing the cribriform foramen X3 of the cribriform plate X2 while puncturing the olfactory mucosa Y2 with the needle portion 10, thereby disposing the puncture portion 12 within the cribriform plate X2; a step of disposing the distal end opening portion 14 within the cribriform foramen X3 or within the cerebral ventricle beyond the cribriform plate X2 in a state where the puncture portion 12 is disposed within the cribriform plate X2; and a step of administering the oligonucleotide into the brain through the distal end opening portion 14.

As illustrated in Fig. 4-2A, a user such as a physician first inserts the administration device 1 from the external naris of a patient toward the olfactory mucosa Y2. When inserting the administration device 1 into the nasal cavity Z1, the device is inserted while confirming the insertion position using a rigid endoscope or the like in a state where the patient's nose has been anesthetized. The administration device 1 may be inserted using a straight instrument such as a rigid endoscope, may be directly inserted alone into the nasal cavity Z1, or may be inserted using an insertion aid such as the guide catheter 100.

Next, as illustrated in Fig. 4-2B, the user causes the needle portion 10 to puncture the olfactory mucosa Y2 (in the order of the olfactory epithelium Y3 and the lamina propria Y4).

Next, as illustrated in Fig. 4-2C, the user further advances puncture of the needle portion 10 into the olfactory mucosa Y2 and causes the needle tip portion 12a of the puncture portion 12 to puncture the cribriform foramen X3. At this time, the abutment portion 32a of the stopper portion 32 of the cannula portion 30 comes into contact with the olfactory epithelium Y3, thereby restricting puncturing movement of the needle portion 10 and preventing over-puncture of the needle portion 10 into the cribriform foramen X3. In addition, when the stopper portion 32 is brought into contact with the olfactory epithelium Y3 to be pressed thereagainst, the puncture posture of the administration device 1 is stabilized, and the puncture portion 12 can puncture toward the cribriform foramen X3, which is a puncture target, without positional displacement. That is, the administration device 1 has the cannula portion 30 that is formed of a tubular member and is disposed to cover the needle portion 10 such that the puncture portion 12 is exposed, the opening portion (distal end opening portion 14) is provided on the distal end side of the puncture portion 12, the stopper portion 32 having the abutment portion 32a that abuts against the olfactory epithelium Y3 of the olfactory mucosa Y2 is formed at the distal end portion of the cannula portion 30, and the opening portion (distal end opening portion 14) is disposed within the cribriform plate X2 in a state where the abutment portion 32a of the stopper portion 32 is brought into contact with the olfactory epithelium Y3. When puncturing of the puncture portion 12 into the cribriform foramen X3 is completed and the distal end opening portion 14 is in a state of being disposed within the cribriform foramen X3 or within a cerebral ventricle beyond the cribriform plate X2, the administration device 1 is capable of initiating administration of the oligonucleotide. Fig. 4-2D illustrates a state where the distal end opening portion 14 is disposed within the cribriform foramen X3.

Then, as illustrated in Fig. 4-2D, the user administers the oligonucleotide accommodated in the accommodation portion 40. When the accommodation portion 40 is a syringe, the plunger is operated manually or using a syringe pump or the like, and the oligonucleotide is appropriately administered in an amount necessary for administration. Accordingly, the oligonucleotide is administered from the distal end opening portion 14 of the puncture portion 12 into the cribriform foramen X3. After administering the oligonucleotide, the user removes the administration device 1 from the nasal cavity Z1 and completes the series of processing.

As illustrated in Fig. 4-2E, the oligonucleotide administered into the cribriform foramen X3 flows toward the olfactory-bulb-side opening portion of the cribriform foramen X3 and is delivered to the brain tissue B via a delivery medium such as the cerebrospinal fluid C or the olfactory nerve Y5. In addition, in Figs. 4-2D and 4-2E, illustration of the cerebrospinal fluid C is omitted in order to make the flow after administration of the oligonucleotide easier to understand.

As described above, the administration device 1 according to the present embodiment is an administration device 1 including the needle portion 10 having the puncture portion 12 for delivering the oligonucleotide intranasally into the brain of a mammal, in which the oligonucleotide is administered into the brain through an opening portion (distal end opening portion 14) of the puncture portion 12 in a state where the puncture portion 12 is disposed within the cribriform plate X2.

With such a configuration, by puncturing the puncture portion 12 into the cribriform foramen X3 of the cribriform plate X2 and administering the oligonucleotide such as a drug through the distal end opening portion 14 in a state where the distal end opening portion 14 provided in the puncture portion 12 is disposed within the cribriform foramen X3 or within a cerebral ventricle beyond the cribriform plate X2, the oligonucleotide can be delivered to the brain tissue B in a minimally invasive manner via a delivery medium such as the cerebrospinal fluid C or the olfactory nerve Y5. In particular, when a therapeutic macromolecular drug such as a protein or antibody, which is currently difficult to administer to the brain tissue B, is administered as the oligonucleotide, the drug can be efficiently delivered to the brain tissue B while bypassing the blood-brain barrier or the like. In addition, since the oligonucleotide is administered within the cribriform foramen X3 formed in the cribriform plate X2 after passing through the olfactory mucosa Y2, where a large number of blood vessels and lymphatic vessels are present, the amount of the oligonucleotide flowing into blood vessels and lymphatic vessels can be minimized while suppressing leakage into the nasal cavity Z1. Furthermore, compared with the intrathecal administration method, the intranasal spray method, and the intraventricular administration method, which have conventionally been known as methods for administering the oligonucleotide to the brain tissue B, the present invention can transfer the oligonucleotide to the brain in a minimally invasive manner and in an extremely efficient manner.

The oligonucleotide of the present invention can be intranasally administered into the brain of a mammal using the administration device 1 including the cannula portion 30 that is formed of a tubular member and is disposed to cover the needle portion 10 such that the puncture portion 12 is exposed, in which the opening portion (distal end opening 14) is provided on the distal end side of the puncture portion 12, the stopper portion 32 having the abutment portion 32a that abuts against the olfactory epithelium Y3 of the olfactory mucosa Y2 is formed at the distal end portion of the cannula portion 30, and the opening portion (distal end opening portion 14) is disposed within the cribriform plate X2 in a state where the abutment portion 32a of the stopper portion 32 is brought into contact with the olfactory epithelium Y3.

Further, the oligonucleotide of the present invention can be administered intranasally into the brain of a mammal using the administration device 1 having the hub portion 20 to which the accommodation portion 40 accommodating the oligonucleotide can be attached, in which the hub portion 20 holds the proximal end of the cannula portion 30 and/or the proximal end of the needle shaft portion 11 of the needle portion 10 inserted through the lumen of the cannula portion 30.

The oligonucleotide of the present invention can be administered intranasally into the brain of a mammal using the administration device 1 in which the cross-sectional shape of the abutment portion 32a of the stopper portion 32 of the cannula portion 30 is circular or elliptical.

### [Other Aspects]

Other embodiments of the present invention include a treatment method using the above-described oligonucleotide. Here, the treatment method includes not only a method for treating a disease, but also a method for prevention and a method for amelioration. In addition, the disease is not particularly limited, but the treatment method according to one embodiment of the present invention is suitably used for a central nervous system disease. That is, the present invention may also include the following embodiments.
1. A method for treating a brain disease, including an administration step of administering an oligonucleotide intranasally into the brain of a mammal by injecting the oligonucleotide from within a cribriform foramen of the cribriform plate.
2. The method for treating a brain disease according to the above 1., in which, in the administration step, the oligonucleotide is injected intranasally into the brain of a mammal through an opening portion of a puncture portion in a state where the puncture portion of the administration device including the needle portion having the puncture portion is disposed within a cribriform foramen of the cribriform plate.
3. The method for treating a brain disease according to the above 1. or 2., in which the oligonucleotide is any one from the group consisting of an aptamer, an antisense oligonucleotide, a decoy nucleic acid, a ribozyme, siRNA, miRNA, and mRNA.
4. The method for treating a brain disease according to any one of the above 1. to 3., in which the oligonucleotide is siRNA or antisense oligonucleotide.
5. The method for treating a brain disease according to any one of the above 1. to 4., in which the oligonucleotide is siRNA.
6. The method for treating a brain disease according to the above 5., in which the proportion of 2'-modified nucleotides among nucleotides constituting the siRNA is 80% or more.
7. The method for treating a brain disease according to the above 5. or 6., in which all nucleotides constituting the siRNA are 2'-modified nucleotides.
8. The method for treating a brain disease according to any one of the above 5. to 7., in which the siRNA has one or more lipophilic moieties.
9. The method for treating a brain disease according to the above 8., in which the lipophilic moiety is at least one selected from the group consisting of a substituted or unsubstituted alkyl chain having 14 to 24 carbon atoms, eicosapentaenoic acid (EPA), arachidonic acid (ARA), and cholic acid.
10. The method for treating a brain disease according to any one of the above 5. to 9., in which the target gene of the siRNA is SNCA or HTT.
11. The method for treating a brain disease according to the above 5. to 10., in which all phosphodiester bonds linking the first and second nucleotides counted from both ends of the sense strand and antisense strand of the siRNA are replaced with phosphorothioate bonds.
12. The method for treating a brain disease according to the above 5. to 11., in which the siRNA includes any one pair of nucleic acid sequences selected from the group consisting of the following (S1) to (S11):
   (S1) SEQ ID NO: 46 (sense strand) and SEQ ID NO: 213 (antisense strand);
   (S2) SEQ ID NO: 83 (sense strand) and SEQ ID NO: 250 (antisense strand);
   (S3) SEQ ID NO: 93 (sense strand) and SEQ ID NO: 260 (antisense strand);
   (S4) SEQ ID NO: 169 (sense strand) and SEQ ID NO: 336 (antisense strand);
   (S5) SEQ ID NO: 180 (sense strand) and SEQ ID NO: 347 (antisense strand);
   (S6) SEQ ID NO: 1360 (sense strand) and SEQ ID NO: 1389 (antisense strand);
   (S7) SEQ ID NO: 1366 (sense strand) and SEQ ID NO: 1395 (antisense strand);
   (S8) SEQ ID NO: 1368 (sense strand) and SEQ ID NO: 1397 (antisense strand);
   (S9) SEQ ID NO: 1372 (sense strand) and SEQ ID NO: 1401 (antisense strand);
   (S10) SEQ ID NO: 1374 (sense strand) and SEQ ID NO: 1403 (antisense strand); and
   (S11) SEQ ID NO: 1756 (sense strand) and SEQ ID NO: 1759 (antisense strand).
13. The method for treating a brain disease according to the above 5., in which the siRNA includes the sequence of any one pair of oligonucleotides selected from the group consisting of ds2, ds9, ds17 to ds249, and ds254 to ds273 described in any of Tables 1-1 to 1-6.
14. The method for treating a brain disease according to the above 13., in which the siRNA includes a sequence of any one pair of oligonucleotides selected from the group consisting of the ds9, the ds28, the ds35, the ds36, the ds40, the ds42, the ds79, the ds105, the ds113, the ds156, the ds164, the ds172, the ds175, the ds176, the ds177, the ds178, the ds187, the ds198, the ds201, the ds211, and the ds213 to the ds246 described in any of the Tables 1-1 to 1-6.
15. The method for treating a brain disease according to the above 13., in which the siRNA is any one pair of oligonucleotides selected from the group consisting of the ds2, the ds254, the ds257, the ds258, the ds259, the ds261, the ds263, the ds267, the ds268, and the ds273 described in any of Tables 1-1 to 1-6.
16. The method for treating a brain disease according to the above 5., in which the siRNA includes the sequence of any one pair of oligonucleotides selected from the group consisting of ds274 to ds461 described in any of Tables 2-1 to 2-4.
17. The method for treating a brain disease according to the above 16., in which the siRNA includes a sequence of any one pair of oligonucleotides selected from the group consisting of the ds278, the ds280, the ds299, the ds308, the ds347, the ds405, the ds406, the ds411, the ds414, the ds422, and the ds432 to ds461 described in any of the Tables 2-1 to 2-4.
18. The method for treating a brain disease according to the above 2., in which
   the administration device has a cannula portion that is formed of a tubular member and is disposed to cover the needle portion such that the puncture portion is exposed,
   the opening portion is provided on a distal end side of the puncture portion,
   a stopper portion having an abutment portion that abuts against an olfactory epithelium of an olfactory mucosa is formed at a distal end portion of the cannula portion, and
   the opening portion is disposed within the cribriform plate in a state where the abutment portion of the stopper portion is brought into contact with the olfactory epithelium.
19. The method for treating a brain disease according to the above 18., in which
   the administration device has a hub portion to which an accommodation portion accommodating the oligonucleotide is attachable, and
   the hub portion holds a proximal end of the cannula portion and/or a proximal end of a needle shaft portion of the needle portion inserted through a lumen of the cannula portion.
20. The method for treating a brain disease according to the above 18. or 19., in which a cross-sectional shape of the abutment portion of the stopper portion is circular or elliptical.
21. The method for treating a brain disease according to any one of the above 1. to 20., in which the brain disease is a central nervous system disease.
22. The method for treating a brain disease according to any one of the above 1. to 21., in which the brain disease is Alzheimer's disease, Parkinson's disease, Huntington's disease, amyotrophic lateral sclerosis (ALS), frontotemporal dementia, or dementia with Lewy bodies.

Other embodiments of the present invention include a method for administering the above-described oligonucleotide to a subject. Here, the subject is not particularly limited as long as the subject is a mammal, but is preferably a human.

That is, the present invention may also include the following embodiments.
1. A method for administering an oligonucleotide, including an administration step of administering an oligonucleotide intranasally into the brain of a subject (mammal) by injecting the oligonucleotide from within a cribriform foramen of a cribriform plate.
2. The method for administering an oligonucleotide according to the above 1., in which, in the administration step, the oligonucleotide is administered intranasally into the brain of a mammal through an opening portion of a puncture portion in a state where the puncture portion of the administration device including the needle portion having the puncture portion is disposed within a cribriform foramen of the cribriform plate.
3. The method for administering an oligonucleotide according to the above 1. or 2., in which the oligonucleotide is any one selected from the group consisting of an aptamer, an antisense oligonucleotide, a decoy nucleic acid, a ribozyme, siRNA, miRNA, and mRNA.
4. The method for administering an oligonucleotide according to any one of 1. to 3., in which the oligonucleotide is siRNA or an antisense oligonucleotide.
5. The method for administering an oligonucleotide according to any one of the above 1. to 4., in which the oligonucleotide is siRNA.
6. The method for administering an oligonucleotide according to the above 5., in which the proportion of 2'-modified nucleotides among nucleotides constituting the siRNA is 80% or more.
7. The method for administering an oligonucleotide according to the above 5. or 6., in which all nucleotides constituting the siRNA are 2'-modified nucleotides.
8. The method for administering an oligonucleotide according to any one of the above 5. to 7., in which the siRNA has one or more lipophilic moieties.
9. The method for administering an oligonucleotide according to the above 8., in which the lipophilic moiety is at least one selected from the group consisting of a substituted or unsubstituted alkyl chain having 14 to 24 carbon atoms, eicosapentaenoic acid (EPA), arachidonic acid (ARA), and cholic acid.
10. The method for administering an oligonucleotide according to the above 5. to 9., in which the target gene of the siRNA is SNCA or HTT.
11. The method for administering an oligonucleotide according to the above 5. to 10., in which all phosphodiester bonds linking the first and second nucleotides counted from both ends of the sense strand and antisense strand of the siRNA are replaced with phosphorothioate bonds.
12. The method for administering an oligonucleotide according to the above 5. to 11., in which the siRNA includes any one pair of nucleic acid sequences selected from the group consisting of the following (S1) to (S11):
   (S1) SEQ ID NO: 46 (sense strand) and SEQ ID NO: 213 (antisense strand);
   (S2) SEQ ID NO: 83 (sense strand) and SEQ ID NO: 250 (antisense strand);
   (S3) SEQ ID NO: 93 (sense strand) and SEQ ID NO: 260 (antisense strand);
   (S4) SEQ ID NO: 169 (sense strand) and SEQ ID NO: 336 (antisense strand);
   (S5) SEQ ID NO: 180 (sense strand) and SEQ ID NO: 347 (antisense strand);
   (S6) SEQ ID NO: 1360 (sense strand) and SEQ ID NO: 1389 (antisense strand);
   (S7) SEQ ID NO: 1366 (sense strand) and SEQ ID NO: 1395 (antisense strand);
   (S8) SEQ ID NO: 1368 (sense strand) and SEQ ID NO: 1397 (antisense strand);
   (S9) SEQ ID NO: 1372 (sense strand) and SEQ ID NO: 1401 (antisense strand);
   (S10) SEQ ID NO: 1374 (sense strand) and SEQ ID NO: 1403 (antisense strand); and
   (S11) SEQ ID NO: 1756 (sense strand) and SEQ ID NO: 1759 (antisense strand).
13. The method for administering an oligonucleotide according to the above 5., in which the siRNA includes the sequence of any one pair of oligonucleotides selected from the group consisting of ds2, ds9, ds17 to ds249, and ds254 to ds273 described in any of Tables 1-1 to 1-6.
14. The method for administering an oligonucleotide according to the above 13., in which the siRNA includes a sequence of any one pair of oligonucleotides selected from the group consisting of the ds9, the ds28, the ds35, the ds36, the ds40, the ds42, the ds79, the ds105, the ds113, the ds156, the ds164, the ds172, the ds175, the ds176, the ds177, the ds178, the ds187, the ds198, the ds201, the ds211, and the ds213 to the ds246 described in any of the Tables 1-1 to 1-6.
15. The method for administering an oligonucleotide according to any one of the above 13., in which the siRNA is any one pair of oligonucleotides selected from the group consisting of the ds2, the ds254, the ds257, the ds258, the ds259, the ds261, the ds263, the ds267, the ds268, and the ds273 described in any of Tables 1-1 to 1-6.
16. The method for administering an oligonucleotide according to the above 5., in which the siRNA includes the sequence of any one pair of oligonucleotides selected from the group consisting of ds274 to ds461 described in any of Tables 2-1 to 2-4.
17. The method for administering an oligonucleotide according to the above 16., in which the siRNA includes a sequence of any one pair of oligonucleotides selected from the group consisting of the ds278, the ds280, the ds299, the ds308, the ds347, the ds405, the ds406, the ds411, the ds414, the ds422, and the ds432 to ds461 described in any of the Tables 2-1 to 2-4.
18. The method for administering an oligonucleotide according to the above 2., in which
   the administration device has a cannula portion that is formed of a tubular member and is disposed to cover the needle portion such that the puncture portion is exposed,
   the opening portion is provided on a distal end side of the puncture portion,
   a stopper portion having an abutment portion that abuts against an olfactory epithelium of an olfactory mucosa is formed at a distal end portion of the cannula portion, and
   the opening portion is disposed within the cribriform plate in a state where the abutment portion of the stopper portion is brought into contact with the olfactory epithelium.
19. The method for administering an oligonucleotide according to the above 18., in which
   the administration device has a hub portion to which an accommodation portion accommodating the oligonucleotide is attachable, and
   the hub portion holds a proximal end of the cannula portion and/or a proximal end of a needle shaft portion of the needle portion inserted through a lumen of the cannula portion.
20. The method for administering an oligonucleotide according to the above 18. or 19., in which a cross-sectional shape of the abutment portion of the stopper portion is circular or elliptical.

Further, the oligonucleotide according to the present invention is administered intranasally into the brain of a mammal by being injected from within a cribriform foramen of a cribriform plate, and accordingly, the oligonucleotide can be efficiently delivered into the brain while being highly minimally invasive, and can further exhibit high efficacy as a nucleic acid drug. In this manner, the oligonucleotide described in the present specification is particularly suitable for intranasal administration, but on the other hand, certain efficacy as a nucleic acid drug (for example, reduction (knockdown) of expression of a predetermined protein) can also be expected by non-nasal administration. That is, the oligonucleotide described in the present specification is not limited to a specific administration method, and the oligonucleotide itself has certain efficacy as a nucleic acid drug. For example, the oligonucleotide described in the present specification can also be used as siRNA targeting a predetermined gene, without being limited to a specific administration method, as shown below.
1. An siRNA targeting SNCA, having the sequence of any one pair of oligonucleotides selected from the group consisting of the following (S6) to (S11):
   (S6) SEQ ID NO: 1360 (sense strand) and SEQ ID NO: 1389 (antisense strand) (S-0179);
   (S7) SEQ ID NO: 1366 (sense strand) and SEQ ID NO: 1395 (antisense strand) (S-0186);
   (S8) SEQ ID NO: 1368 (sense strand) and SEQ ID NO: 1397 (antisense strand) (S-0187);
   (S9) SEQ ID NO: 1372 (sense strand) and SEQ ID NO: 1401 (antisense strand) (S-0191);
   (S10) SEQ ID NO: 1374 (sense strand) and SEQ ID NO: 1403 (antisense strand) (S-0193); and
   (S11) SEQ ID NO: 1756 (sense strand) and SEQ ID NO: 1759 (antisense strand) (S-0197).
2. The siRNA according to the above 1., in which the proportion of 2'-modified nucleotides among nucleotides constituting the siRNA is 80% or more.
3. The siRNA according to the above 1. or 2., in which all nucleotides constituting the siRNA are 2'-modified nucleotides.
4. The siRNA according to any one of the above 1. to 3., in which the siRNA has one or more lipophilic moieties.
5. The siRNA according to the above 4., in which the lipophilic moiety is at least one selected from the group consisting of a substituted or unsubstituted alkyl chain having 14 to 24 carbon atoms, eicosapentaenoic acid (EPA), arachidonic acid (ARA), and cholic acid.
6. The siRNA according to any one of the above 1. to 5., in which all phosphodiester bonds linking the first and second nucleotides counted from both ends of the sense strand and antisense strand of the siRNA are replaced with phosphorothioate bonds.
7. An siRNA targeting SNCA including the sequence of any one pair of oligonucleotides selected from the group consisting of ds9, ds28, ds35, ds36, ds40, ds42, ds79, ds105, ds113, ds156, ds164, ds172, ds175, ds176, ds177, ds178, ds187, ds198, ds201, ds211, and ds213 to ds246 described in any of Tables 1-1 to 1-6.
8. An siRNA targeting SNCA, in which the siRNA is one pair of oligonucleotides selected from the group consisting of ds2, ds254, ds257, ds258, ds259, ds261, ds263, ds267, ds268, and ds273 described in any one of Tables 1-1 to 1-6.

According to one aspect of the present invention, 9. there is provided an siRNA targeting HTT, having the sequence of any one pair of oligonucleotides selected from the group consisting of the following (H1) to (H5):
(H1) SEQ ID NO: 365 (sense strand) and SEQ ID NO: 623 (antisense strand) (H-0005);
(H2) SEQ ID NO: 370 (sense strand) and SEQ ID NO: 628 (antisense strand) (H-0010);
(H3) SEQ ID NO: 396 (sense strand) and SEQ ID NO: 654 (antisense strand) (H-0036);
(H4) SEQ ID NO: 407 (sense strand) and SEQ ID NO: 665 (antisense strand) (H-0047); and
(H5) SEQ ID NO: 469 (sense strand) and SEQ ID NO: 727 (antisense strand) (H-0109).

10. In the siRNA according to the above 9., the proportion of 2'-modified nucleotides among nucleotides constituting the siRNA is 80% or more.

11. In the siRNA according to the above 9. or 10., all nucleotides constituting the siRNA are 2'-modified nucleotides.

12. In the siRNA according to any one of the above 9. to 11., all phosphodiester bonds linking the first and second nucleotides counted from both ends of the sense strand and antisense strand of the siRNA are replaced with phosphorothioate bonds.

13. An siRNA targeting HTT including the sequence of any one pair of oligonucleotides selected from the group consisting of ds274 to ds461 described in any of Tables 2-1 to 2-4.

14. An siRNA targeting HTT including the sequence of any one pair of oligonucleotides selected from the group consisting of ds278, ds280, ds299, ds308, ds347, ds405, ds406, ds411, ds414, ds422, and ds432 to ds461 described in any of Tables 2-1 to 2-4.

Above, although the embodiments of the present invention have been described in detail, this is illustrative and exemplary and not restrictive, and it is clear that the scope of the present invention should be interpreted by the appended claims.

Hereinafter, the present invention will be described by Examples. However, the present invention is not limited to these Examples.

### Examples

The effects of the present invention will be described using the following Examples and Comparative Examples. The materials, amounts used, proportions, treatment details, treatment procedures, and the like illustrated in the following Production Examples and Examples can be changed as appropriate without departing from the spirit of the present invention. Therefore, the technical scope of the present invention is not limited only to the following Examples. It should be noted that the quantitative ratios of raw materials used as solutions or dispersions (units: "parts by mass" and "% by mass") are all values converted into solid content. In addition, unless otherwise specified, each operation was performed at room temperature (hereinafter room temperature is 25°C). It should be noted that, in the following steps, "N" in "mN", "fN", "dN", "rN", "eN", and "lN" represents each of A, T, C, or G, in the case of DNA, and represents each of A, U, C, or G, in the case of RNA.

### <Preparation of siRNA (ds1 to ds6) Used in Examples and Reference Examples>

### (Step 1) Synthesis of oligonucleotides (ss6 and ss9)

The oligonucleotides were synthesized using ns-8 (manufactured by GeneDesign Inc.). As the solid-phase support, 2'-OMe-RNA CPG (manufactured by Glen Research) was used, and as phosphoramidites, 2'-OMe and 2'-F ribonucleoside phosphoramidites (manufactured by Glen Research) and Solid Chemical Phosphorylation Reagent II (manufactured by Glen Research) were used, which were prepared to 0.12 mol/L with ultra-dehydrated MeCN (manufactured by FUJIFILM Wako Pure Chemical Corporation). As the activator, 0.25 mol/L BTT in MeCN (manufactured by Glen Research) was used; as the oxidizing agent, 0.02 mol/L iodine in THF/pyridine/water (7:2:1, manufactured by Glen Research) was used; as the sulfurizing agent, 0.1 mol/L DDTT in pyridine (manufactured by ChemGenes) was used; as the deblocking reagent, 3% TCA in dichloromethane (manufactured by FUJIFILM Wako Pure Chemical Corporation) was used; and as the capping agent, CAP A (Ac20/lutidine/THF = 1:1:8, manufactured by Glen Research) and CAP B (10% 1-methylimidazole in THF, manufactured by Glen Research) were used. Solid-phase synthesis of the oligonucleic acids was performed using RNA1.0 or RNA10, which are standard methods accompanying ns-8.

Cleavage from the solid-phase support and deprotection were performed according to the protocol provided by the reagent company, using an AMA solution prepared by mixing 28% aqueous ammonia and a 40% methylamine aqueous solution at 1:1. The obtained crude product was concentrated under reduced pressure and then purified by anion-exchange chromatography (system: Prominence (manufactured by Shimadzu Corporation); column: Mono Q (registered trademark) 5/50 GL or Mono Q (registered trademark) 10/100 GL (manufactured by Cytiva); Solution A: 30% MeCN, 10 mmol/L Tris-HCl (pH 8); Solution B: 1 mol/L NaBr, 30% MeCN, 10 mM Tris-HCl (pH 8)), and the obtained fractions were analyzed by LC-MS (system: Infinity 1260 (manufactured by Agilent); column: ACQUITY Premier Oligonucleotide C18 Column, 130 Å, 1.7 µm, 2.1 × 50 mm (manufactured by Waters); Solution A: 8.6 mmol/L TEA/100 mmol/L HFIP; Solution B: MeOH; gradient: 10% B to 90% B in 9 min; flow rate: 0.6 mL/min; column temperature: 60°C; detection: PDA (260 nm), ESI-MS). Fractions containing the target product were desalted with distilled water (manufactured by Otsuka Pharmaceutical Factory) using Amicon (registered trademark) Ultra-15, Ultracel, 3 kDa (manufactured by Merck). The obtained solution was microfiltered using Ultrafree (registered trademark)-MC or -CL, PVDF, 0.22 µm pore size, sterile (manufactured by Merck), to synthesize oligonucleotides ss6 and ss9 shown in Table 5. The molecular weight of the obtained oligonucleotide was calculated by deconvolution using software attached to the LC-MS. The sequences of all oligonucleotides used in Examples, and the calculated values and measured values of molecular weights of the obtained oligonucleotides, are shown in Table 5.

### (Step 2) Synthesis of oligonucleotides (ss1, ss2, ss4, and ss5) using special amidites

The oligonucleotides were synthesized using AKTA oligopilot (manufactured by Cytiva). NittoPhase (registered trademark) HL 2'OMeU350 (manufactured by Kinovate) and NittoPhase (registered trademark) HL 2'OMeA350 (manufactured by Kinovate) were used as the solid-phase supports. As phosphoramidites, phosphoramidites corresponding to (vnT), (vmU), and [C16U] shown in Figs. 1 and 2, synthesized according to the methods described in the Journal of Medicinal Chemistry (2018, 61(3), 734-744) and WO 2021/092371, together with 2'-OMe and 2'-F ribonucleoside phosphoramidites (Thermo Fisher Scientific (hereinafter also referred to as "TFS")), were used, and were prepared to 0.15 mol/L with ultra-dehydrated MeCN. As the activator, 0.25 mol/L BTT in MeCN (manufactured by Glen Research) was used; as the oxidizing agent, 0.05 mol/L Iodine in pyridine/water (9:1, manufactured by Sigma-Aldrich) was used; as the sulfurizing agent, 0.1 mol/L DDTT in pyridine (manufactured by ChemGenes) was used; as the deblocking reagent, 3% DCA in toluene (manufactured by FUJIFILM Wako Pure Chemical Corporation) was used; and as the capping agent, CAP A (MeCN:N-methylimidazole = 8:2, manufactured by Sigma-Aldrich), CAP B1 (Ac2O:MeCN = 4:6, manufactured by FUJIFILM Wako Pure Chemical Corporation), and CAP B2 (2,6-lutidine:MeCN = 6:4, manufactured by FUJIFILM Wako Pure Chemical Corporation) were used. Solid-phase synthesis of the oligonucleic acids was performed by changing some conditions of Column 6 and 12 ml AKTAop plus 100 Ed CA, which is a standard method accompanying AKTA oligopilot (Coupling: 4 eq. phosphoramidite, 10 min; Oxidation: 4 eq., 1 min; Sulfurzation: 5 CV, 10 min).

Cleavage from the solid-phase support and deprotection were performed using 28% aqueous ammonia or 28% aqueous ammonia to which DEA was added to 3% (v/v) (3% DEA/ammonia aqueous solution), with reference to the protocol described in the above literature. The obtained crude product was concentrated under reduced pressure and then purified by anion-exchange chromatography (system: AKTA pure (registered trademark) 150 (manufactured by Cytiva); column: TSKgel (registered trademark) SuperQ-5PW, 13 µm, 21.5 × 150 mm (manufactured by Tosoh); Solution A: 20% MeCN, 10 mmol/L Tris-HCl (pH 8); Solution B: 1 mol/L NaBr, 20% MeCN, 10 mM Tris-HCl (pH 8)), and the obtained fractions were analyzed by LC-MS under the same conditions as in Step 1. Fractions containing the target product were desalted with distilled water (manufactured by Otsuka Pharmaceutical Factory) using an LV Centramate TFF system (manufactured by PALL) equipped with a Centramate (registered trademark) cassette, Omega PES membrane, 1 kDa cut-off (manufactured by PALL). The obtained solution was microfiltered using a Millex-GP Syringe Filter Unit, 0.22 µm, polyethersulfone, 33 mm, gamma sterilized (manufactured by Merck), to synthesize oligonucleotides ss2 and ss5 having a vinylphosphonate group at the 5' end, and oligonucleotides ss1 and ss4 having a hexadecyl group in a nucleotide within the strand (refer to Table 5).

### (Step 3) Preparation of EPA-PFP ester

Eicosapentaenoic acid (300 mg, 990 µmol, 1.2 equivalents) was dissolved in DMF (N,N-dimethylformamide) (8.3 mL), then triethylamine (350 µL, 2.5 Mmol, 3 equivalents) and PFTU (pentafluorophenol-tetramethyluronium hexafluorophosphate) (350 mg, 830 µmol, 1 equivalent) were added, and the mixture was stirred at room temperature for 1 hour to obtain a PFP ester (pentafluorophenyl ester) solution of eicosapentaenoic acid (about 100 mM). The obtained compound was used in the next conjugation reaction without isolation.

### (Step 4) Synthesis of 5'-EPA-bound oligonucleotide (ss3)

Using PDA-C6-Amino-Modifier Amidite (manufactured by Glen Research), an oligonucleotide having an amino group at the 5' end, which was synthesized according to the method of Step 1, and the PFP ester obtained in Step 3 were reacted in a 75% DMF aqueous solution containing Pierce (registered trademark) 2X Borate Buffer (manufactured by TFS) at pH 8.5. Thereafter, the reaction solution containing the reaction product was purified by ion-pair reversed-phase HPLC to obtain oligonucleotide ss3.

### (Step 5) Synthesis of 3'-EPA-bound oligonucleotide (ss7 and ss8)

Using 3'-PT-Amino-Modifier C6 CPG (manufactured by Glen Research), oligonucleotides having an amino group at the 3' end synthesized according to the method of Step 1 and the PFP ester obtained in Step 3 were reacted in a 75% DMF aqueous solution containing Pierce (registered trademark) 2X Borate Buffer at pH 8.5, and then purified by ion-pair reversed-phase HPLC to obtain oligonucleotides ss7 and ss8.

### (Step 6) Preparation of double-stranded siRNA

In the combinations shown in Table 5, the antisense strand and the sense strand were mixed in equal amounts, heated at 85°C for 5 minutes, and then allowed to cool to room temperature. After cooling, about 200 pmol of the sample was diluted in 30% MeCN in 1×PBS, and formation of a double strand was confirmed by SEC-HPLC analysis (system: Prominence (manufactured by Shimadzu Corporation); column: X-Bridge Protein BEH SEC Column, 200 Å, 3.5 µm, 7.8 mm × 300 mm (manufactured by Waters); buffer: 30% MeCN in 1×PBS (isocratic conditions)). The obtained double strands were lyophilized, compounds ds1 to ds2 were prepared as an aCSF solution, ds3 was prepared as an aCSF solution or a 1xPBS solution, and ds4 to 6 were prepared as 1xPBS solutions, and these solutions were used for evaluation. Fig. 2 illustrates the structures of nucleotides and elements contained in siRNA. In Table 5, "mN" represents 2'-0-methyl RNA, "fN" represents 2'-fluoro-DNA, "dN" represents DNA, "(vnT)" represents 5'-vinylphosphate-2'-O-(N-methylacetamide)-thymidine, "(vmU)" represents 5'-vinylphosphate-2'-O-methyl-uridine, "(C16U)" represents 2'-O-hexadecyluridine, "p" represents 5'-phosphate, and "^" represents a phosphorothioate bond. In addition, the structures of "[L1]", "(C16U)", "(vnT)", and "(vmU)" in Table 5 are illustrated in Figs. 1 and 2.

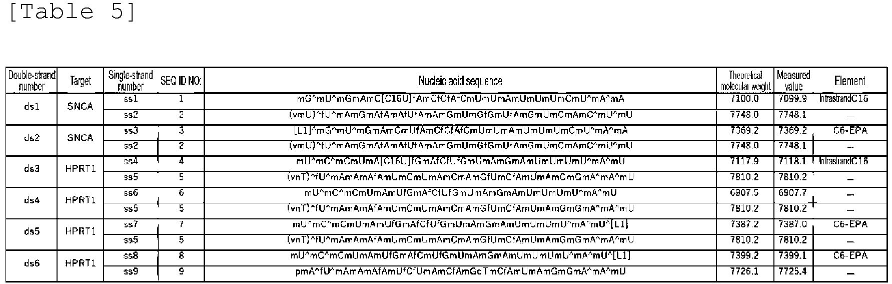

### <Preparation of siRNA (ds7 to ds16) Used in Reference Examples>

### (Step 7) Synthesis of single-stranded oligonucleotides (ss12, ss14)

Oligonucleotides ss12 and ss14 were synthesized by the same methods as in Step 1 and Step 2. The sequences of the oligonucleotides used in Reference Examples, and the calculated values and measured values of molecular weights of the obtained oligonucleotides, are shown in Tables 5 and 6.

### (Step 8) Synthesis of cyclic single-stranded oligonucleotide (ss10)

Oligonucleotide ss10 was synthesized by the method described in WO 2018/199340.

### (Step 9) Synthesis of 3'-EPA-bound oligonucleotide (ss13)

Oligonucleotide ss13 was synthesized by the same method as in Step 5.

### (Step 10) Preparation of C16-PFP ester

Palmitic acid (11 mg, 43 µmol, 1 equivalent) was dissolved in DMF (850 µL), then triethylamine (18 µL, 130 µmol, 3 equivalents) and pentafluorophenyl trifluoroacetate (13 mg, 47 µmol, 1.1 equivalents) were added, and the mixture was stirred at room temperature for 5 hours to obtain a PFP ester solution of palmitic acid (about 50 mM). The obtained compound was used in the next conjugation reaction without isolation.

### (Step 11) Synthesis of 3'-C7-C22-bound oligonucleotide (ss16)

C22-C7-CPG was synthesized by the method described in Tetrahedron Letters (Vol. 55, pp. 94-97, 2014). Subsequently, using the C22-C7-CPG, oligonucleotide ss16 was obtained by the same method as in Step 1.

### (Step 12) Preparation of ARA-PFP ester

A PFP ester solution of arachidonic acid (about 50 mM) was obtained by the same method as in Step 10 using arachidonic acid. The obtained compound was used in the next conjugation reaction without isolation.

### (Step 13) Preparation of cholic acid-PFP ester

Tritrifluoroacetyl cholic acid (400 mg, 570 µmol, 1 equivalent), which was synthesized by the method described in Journal of the Chemical Society, Perkin Transactions 1 (Vol. 8, pp. 2245-2250, 1990), was dissolved in dichloromethane (10 mL), N,N-diisopropylethylamine (500 µL, 2.9 Mmol, 5 equivalents) was added under ice cooling, and pentafluorophenyl trifluoroacetate (0.12 mL, 700 µmol, 1.2 equivalents) was then added. The obtained reaction mixture was warmed to room temperature and stirred for 2 hours. After completion of the reaction, the reaction mixture was diluted with dichloromethane and washed with a saturated sodium hydrogen carbonate aqueous solution and brine. The obtained solution was dried over anhydrous sodium sulfate and then filtered, and the solvent was distilled off under reduced pressure to obtain a crude product. The crude product was purified by reversed-phase column chromatography by elution with 100% acetonitrile (without buffer) to obtain cholic acid-PFP ester (180 mg, 210 µmol, 35%) as a white solid.

In addition, the compound isolated under the following conditions was measured by a nuclear magnetic resonance apparatus (apparatus name: Ascend (registered trademark) 400, manufactured by Bruker).

¹H NMR (400 MHz, DMSO-d6): δ (ppm): 0.79 (s, 3H), 0.86 (d, J = 5.6 Hz, 3H), 0.96 (s, 3H), 1.15-1.48 (m, 8H), 1.50-2.02 (m, 13H), 2.14-2.19 (m, 1H), 2.68-2.70 (m, 1H), 2.78-2.79 (m, 1H), 4.81-4.83 (m, 1H), 5.14 (br s, 1H), 5.39 (br s, 1H).

### (Step 14) Synthesis of 3'-C7-C16-bound oligonucleotide (ss15)

Using 3'-Amino-Modifier C7 CPG (manufactured by Glen Research), the oligonucleotide having an amino group at the 3' end, which was synthesized according to Step 1, and the C16-PFP ester obtained in Step 10 were reacted in a 70% DMF aqueous solution containing Pierce (registered trademark) 2X Borate Buffer (manufactured by Thermo Fisher Scientific) at pH 8.5, thereby obtaining a reaction solution. Thereafter, oligonucleotide ss15 was obtained by purification using ion-pair reversed-phase HPLC.

### (Step 15) Synthesis of 3'-C7-ARA-bound oligonucleotide (ss18)

Oligonucleotide ss18 was obtained by the same method as in Step 14, except that the ARA-PFP ester obtained in Step 12 was used.

### (Step 16) Synthesis of 3'-C7-cholic acid-bound oligonucleotide (ss19)

A reaction solution was obtained by the same method as in Step 14, except that the cholic acid-PFP ester obtained in Step 13 was reacted in a 70% DMF aqueous solution containing Pierce (registered trademark) 2X Borate Buffer (manufactured by TFS) at pH 8.5. Thereafter, after completion of the reaction was confirmed by LC-MS analysis, 600 µL of a 40% methylamine aqueous solution was added, and completion of the deprotection reaction was confirmed by LC-MS analysis. The obtained crude product was purified by ion-pair reversed-phase HPLC to obtain oligonucleotide ss19.

### (Step 17) Synthesis of 5'-EPA-bound oligonucleotide (ss11)

Oligonucleotide ss11 was synthesized by the same method as in Step 4.

### (Step 18) Synthesis of 3'-C7-EPA-bound oligonucleotide (ss17)

Oligonucleotide ss17 was obtained using the EPA-PFP ester obtained in Step 3 by the same method as in Step 14.

### (Step 19) Preparation of double-stranded siRNA

Double strands were prepared by mixing the antisense strand and the sense strand in equal amounts in the combinations shown in Table 6 by the same method as in Step 6. The obtained double strands were lyophilized as needed, prepared as a 1xPBS solution, and used for evaluation. In Table 6, "mN" represents 2'-O-methyl RNA, "fN" represents 2'-fluoro-DNA, "dN" represents DNA, "(vnT)" represents 5'-vinylphosphate-2'-O-(N-methylacetamide)-thymidine, "(vmU)" represents 5'-vinylphosphate-2'-O-methyl-uridine, "(C16U)" represents 2'-O-hexadecyl-uridine, "p" represents 5'-phosphate, and "^" represents a phosphorothioate bond. In addition, the structures of "[L1]", "[L2]", "[L3]", "[L4]", "[L5], "[L6]", "[CL]", "(C16U)", "(vnT)", and "(vmU)" in Table 6 are illustrated in Figs. 1 and 2.

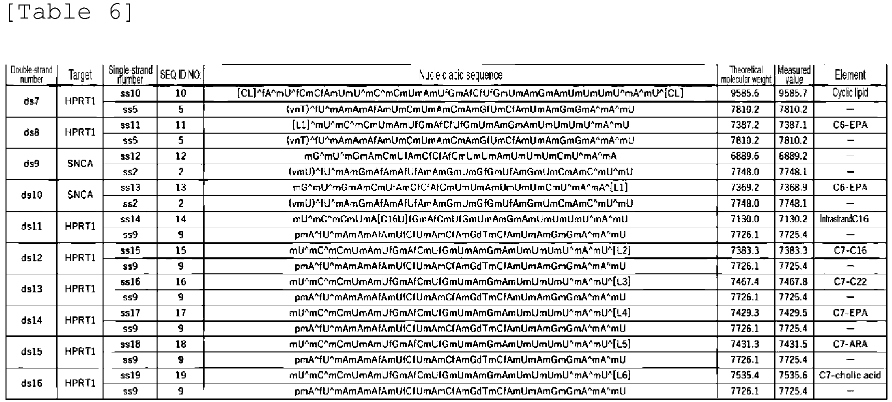

### <Preparation of siRNA (ds17 to ds249) Targeting SNCA>

For ds17 to 249, after single-stranded oligonucleic acids were synthesized by a method according to Step 1 of the preparation of siRNA (ds1 to ds6), double strands were prepared by mixing the antisense strand and the sense strand in equal amounts in 1×PBS in the combinations shown in Tables 7-1 to 7-5 by the same method as in Step 6. In addition, the synthesis of ds17 to 171 was outsourced to GeneDesign Inc. In Tables 7-1 to 7-5, "mN" represents 2'-O-methyl-RNA, "fN" represents 2'-fluoro-DNA, "dN" represents DNA, "(vmU)" represents 5'-vinylphosphate-2'-O-methyl-uridine, "p" represents 5'-phosphate, and "^" represents a phosphorothioate bond. The structure of "(vmU)" is illustrated in Fig. 1.

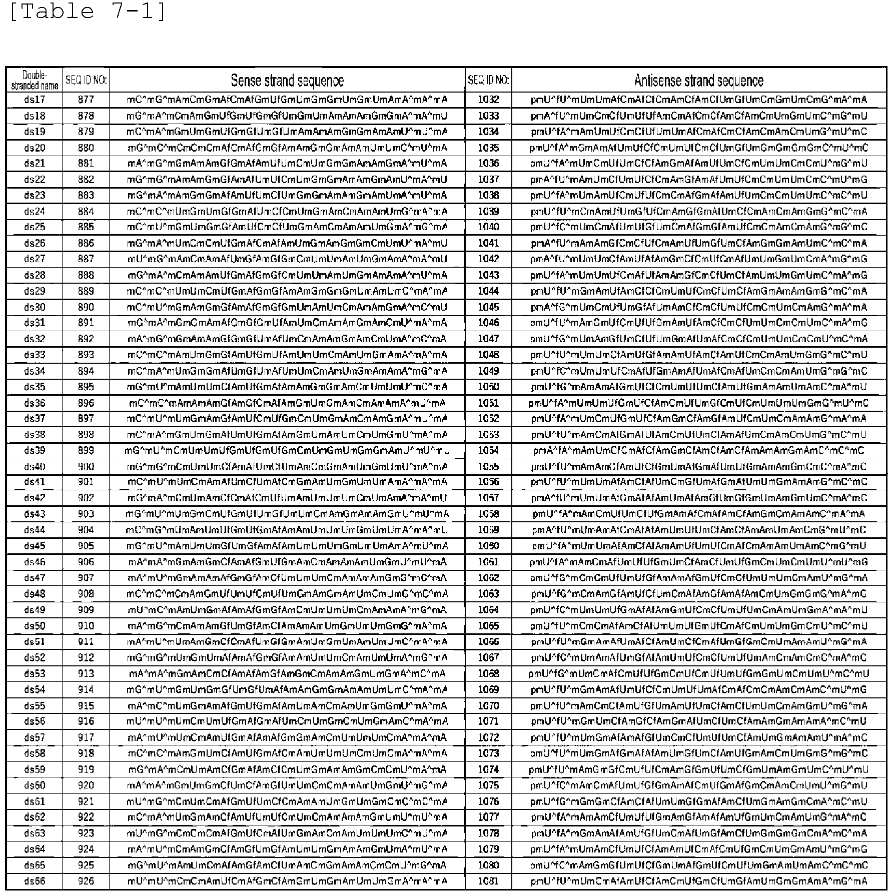

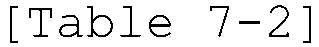

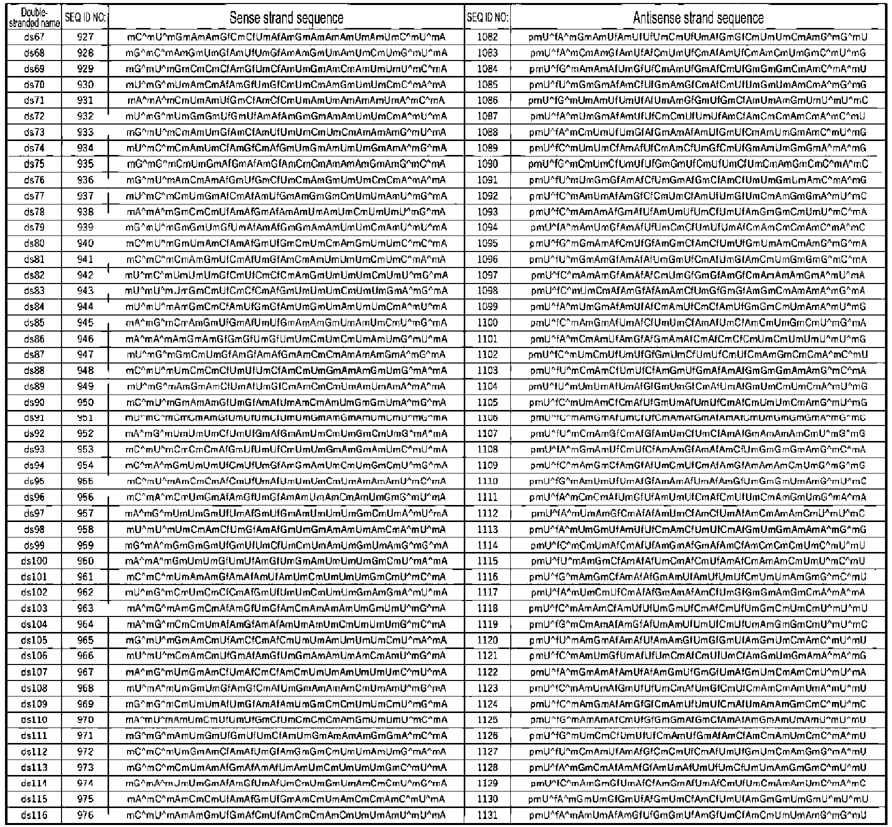

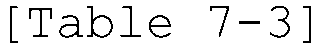

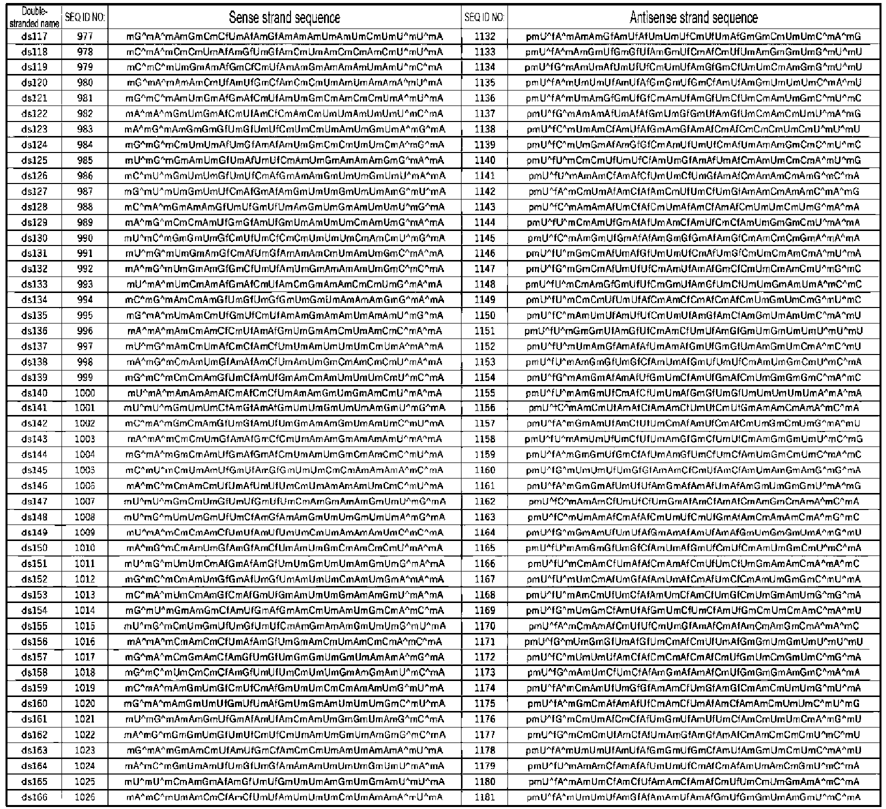

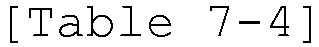

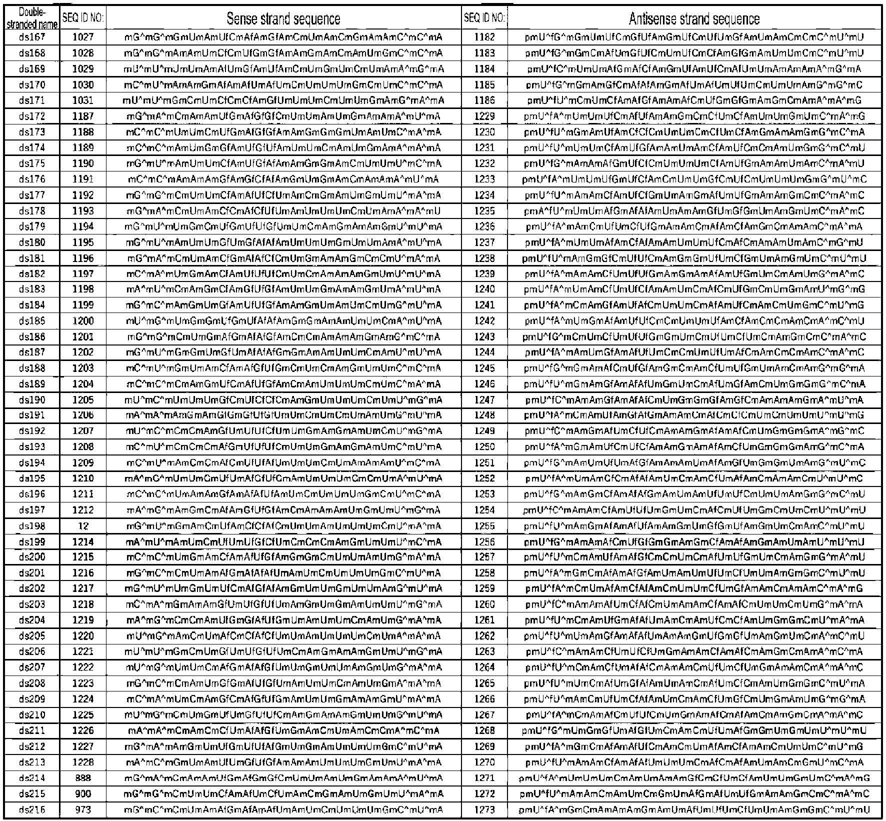

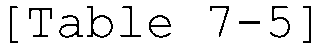

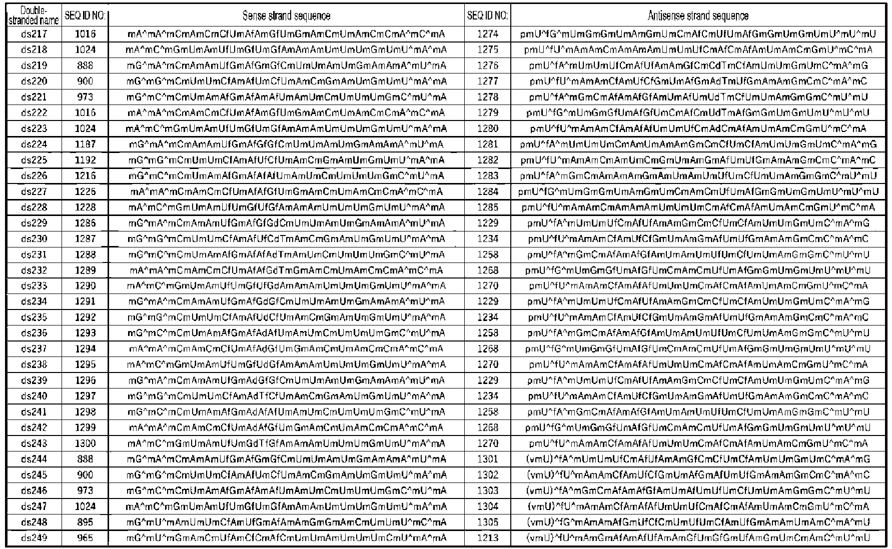

### <Preparation of siRNA (ds250 to ds253) Targeting SNCA>

For ds250 to 253, single-stranded oligonucleic acids were synthesized by a method according to Step 2 and Step 4 of the preparation of siRNA (ds1 to ds6) using 2'-TBDMS ribonucleoside phosphoramidite (manufactured by TFS), and then double strands were prepared by mixing the antisense strand and the sense strand in equal amounts in the combinations illustrated in Table 8 by the same method as in Step 6. The obtained double strands were lyophilized, prepared as an aCSF solution, and used for evaluation. In Table 8, "mN" represents 2'-O-methyl-RNA, "fN" represents 2'-fluoro-DNA, "rN" represents RNA, "(vmU)" represents 5'-vinylphosphate-2'-O-methyl-uridine, and "^" represents a phosphorothioate bond. The structures of "[L1]" and "(vmU)" are illustrated in Figs. 1 and 2.

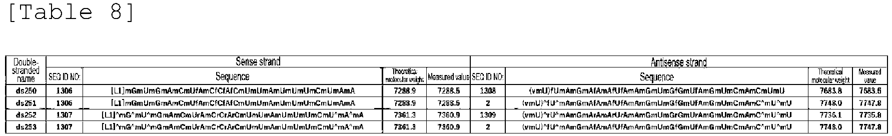

### <Preparation of siRNA (ds254 to ds257) Targeting SNCA>

For ds254 to 257, single-stranded oligonucleic acids were synthesized by a method according to Step 2 and Step 4 of the preparation of siRNA (ds1 to ds6), and then double strands were prepared by mixing the antisense strand and the sense strand in equal amounts in the combinations illustrated in Table 9 by the same method as in Step 6. The obtained double strands were lyophilized, prepared as an aCSF solution, and used for evaluation. In Table 9, "mN" represents 2'-O-methyl-RNA, "fN" represents 2'-fluoro-DNA, "(vmU)" represents 5'-vinylphosphate-2'-O-methyl-uridine, and "^" represents a phosphorothioate bond. The structures of "[L1]" and "(vmU)" are illustrated in Figs. 1 and 2.

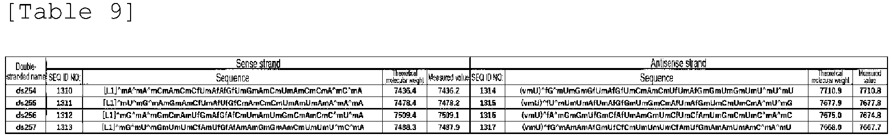

### <Preparation of siRNA (ds258 to ds273) Targeting SNCA>

For ds258 to 273, single-stranded oligonucleic acids were synthesized by a method according to Step 2 and Step 4 of the preparation of siRNA (ds1 to ds6), and then double strands were prepared by mixing the antisense strand and the sense strand in equal amounts in the combinations illustrated in Table 10 by the same method as in Step 6. The obtained double strands were lyophilized, prepared as an aCSF solution, and used for evaluation. In Table 10, "mN" represents 2'-O-methyl-RNA, "fN" represents 2'-fluoro-DNA, "(vmU)" represents 5'-vinylphosphate-2'-O-methyl-uridine, and "^" represents a phosphorothioate bond. The structures of "[L1]" and "(vmU)" are illustrated in Figs. 1 and 2.

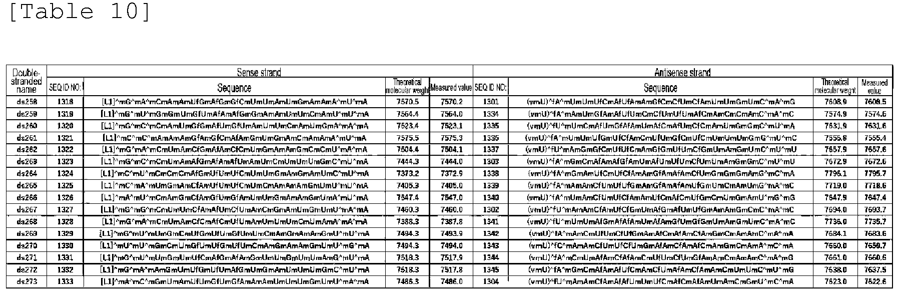

### <Preparation of siRNA (ds274 to ds461) Targeting HTT>

After single-stranded oligonucleic acids were synthesized by a method according to Step 1 of the preparation of siRNA (ds1 to ds6), double strands were prepared by mixing the antisense strand and the sense strand in equal amounts in 1×PBS in the combinations shown in Tables 11-1 to 11-4 by the same method as in Step 6. In Tables 11-1 to 11-4, "mN" represents 2'-O-methyl-RNA, "fN" represents 2'-fluoro-DNA, "dN" represents DNA, "p" represents 5'-phosphate, and "^" represents a phosphorothioate bond.

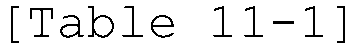

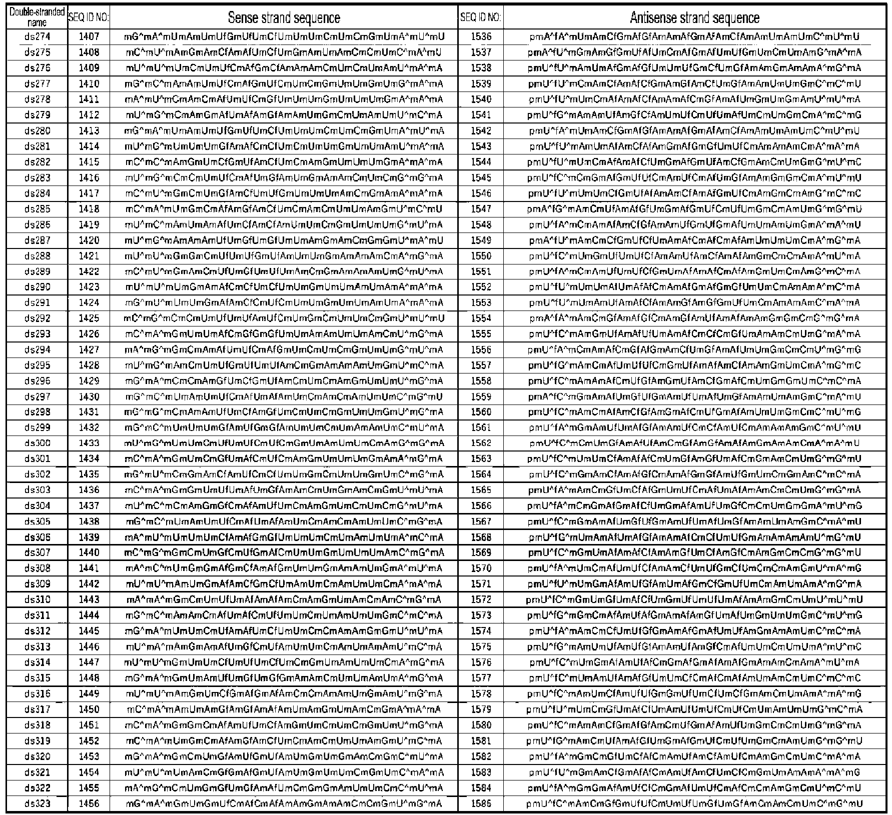

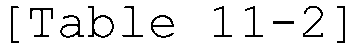

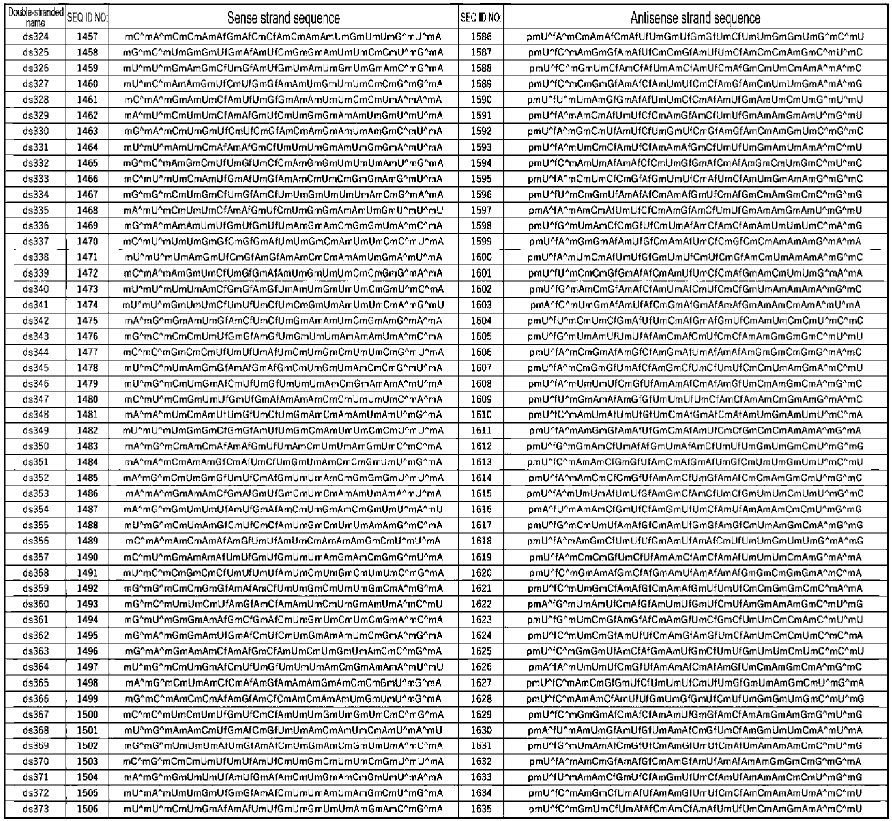

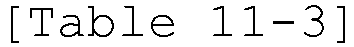

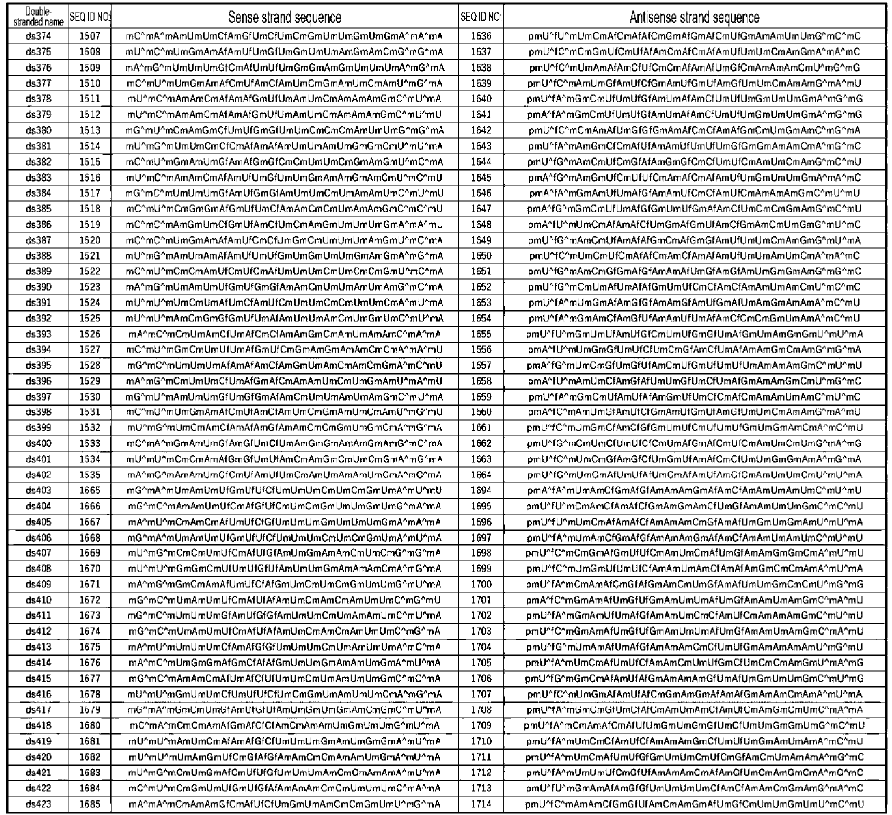

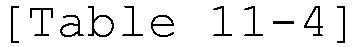

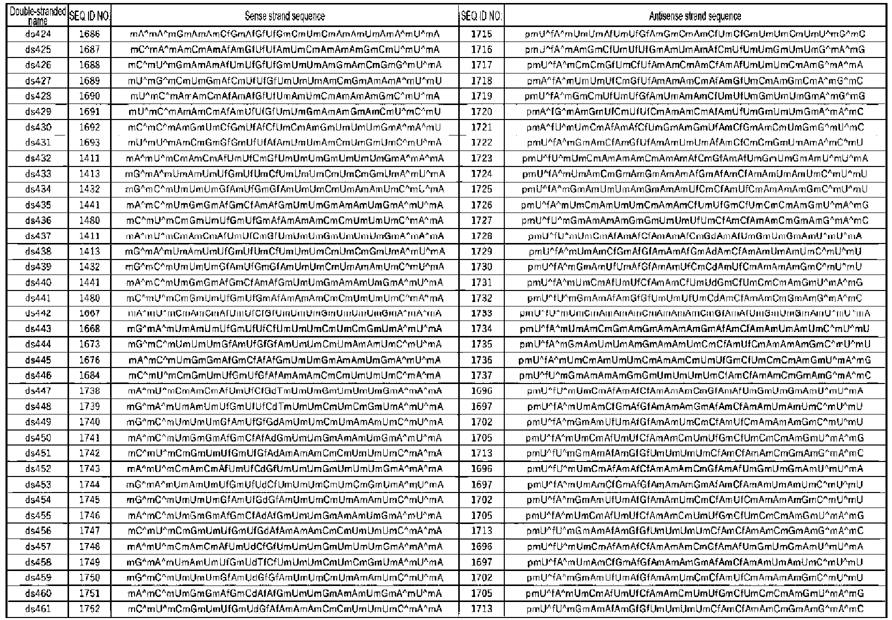

### <Preparation of Antisense Oligonucleotides (SEQ ID NOs: 1346 to 1348)>

SEQ ID NO: 1346 was synthesized using LNA phosphoramidite (manufactured by FUJIFILM Wako Pure Chemical Corporation), with reference to the method described in JCI Insight (2021) 6(5), e135633. SEQ ID NO: 1347 was synthesized according to the method described in The Journal of Clinical Investigation (2006) 116(8), 2290-2296, and SEQ ID NO: 1348 was synthesized according to the method described in Nucleic Acids Research. The obtained oligonucleic acids were lyophilized, prepared as an aCSF solution, and used for evaluation. In Table 12, "eN" represents 2'-O-methoxyethyl-RNA, "lN" represents LNA, "dN" represents DNA, "H" represents 5-methylcytosine, and "^" indicates a phosphorothioate bond. Further, "eH" represents 2'-O-methoxyethyl-5-methylcytosine, and "lH" represents LNA having 5-methylcytosine as a base.

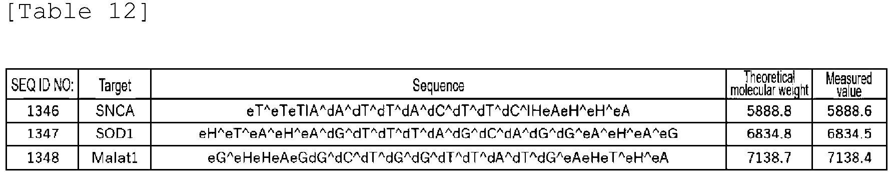

### [Reference Example 1-1: Knockdown Test for Cervical Administration in Rat Using Hypoxanthine-Guanine Phosphoribosyltransferase 1 (Hereinafter Referred to as HPRT1)-Targeted siRNA]

ds4, ds3, and ds7 diluted to 20 mg/mL with D-PBS were administered cervically (intracisternal administration) to 9-week-old SD rats (male) at 30 µL (600 µg/head), and 7 days after administration, each region of the brain was collected and analyzed (N = 5). With respect to ds7, abnormalities occurred in 2 of 5 rats, and therefore the 3 rats in which no abnormalities occurred (N = 3) were analyzed.

To each collected organ, 500 µL of TRIzol (registered trademark) Reagent (manufactured by TFS, 15596-018) and zirconia balls φ5 mm (manufactured by Nikkato Corporation, YTZ-5) were added, and the organ was disrupted using TissueLyser II (manufactured by QIAGEN). After centrifugation, the supernatant was collected, and 40% by volume of chloroform was added and mixed. After centrifugation, the supernatant was collected, and RNA was purified using a KingFisher MagMax-96 Total RNA Isolation Kit (manufactured by TFS, AM1830) with KingFisher (registered trademark) Flex (manufactured by TFS). Using EvoScript Universal cDNA Master (manufactured by Roche Diagnostics, 07912455001), a reverse transcription reaction was performed according to the instructions attached to the kit to produce cDNA.

Using this cDNA as a template for PCR reaction, the HPRT1 gene and, as a control, the beta actin (hereinafter referred to as ACTB) gene were subjected to PCR reaction by the TaqMan (registered trademark) probe method using a QuantStudio 12K Flex real-time PCR system (manufactured by Applied Biosystems), the amounts of mRNA amplification were measured, respectively, and a semiquantitative value of HPRT1 mRNA was calculated using the amount of ACTB mRNA amplification as an internal control. Further, the amounts of mRNA amplification of HPRT1 and ACTB in the negative control group were measured in the same manner, respectively, and a semiquantitative value of HPRT1 mRNA was calculated.

TaqMan probe Rn01527840_m1 (manufactured by Applied Biosystems) was used for measurement of the HPRT1 gene, Rn00667869_m1 (manufactured by Applied Biosystems) was used for measurement of the ACTB gene, and TaqMan (registered trademark) Gene Expression Master Mix (manufactured by Applied Biosystems, 4369542) was used as the reaction reagent, according to the attached protocol. The amount of target mRNA in the nucleic acid-administered individuals was calculated as a relative ratio when the amount of HPRT1 mRNA in the negative control group (non-administered group) was taken as 1. The results expressed as mean ± standard error of the relative ratio of the amount of mRNA are illustrated in Fig. 5. The data for each region in Fig. 5 represent, from left to right, the results for negative control (non-administration), ds4, ds3, and ds7.

The test samples (ds3 and ds7) showed improved knockdown activity as compared with ds4.

### [Reference Example 1-2: Knockdown Test for Cervical Administration in Rat Using HPRT1-Targeted siRNA]

ds4, ds3, ds5, and ds8 diluted to 10 mg/mL with D-PBS were administered cervically to 9-week-old SD rats (male) at 30 µL (300 µg/head), and 7 days after administration, each region of the brain was collected and analyzed (N = 5). Here, with respect to the cerebral cortex of one individual from each of the non-administered group and ds3, the data was limited to four individuals due to sample loss. Thereafter, the expression level of mRNA was measured by the same method as in Reference Example 1-1. The amount of target mRNA in the nucleic acid-administered individuals was calculated as a relative ratio when the amount of HPRT1 mRNA in the negative control group (non-administered group) was taken as 1. The results expressed as mean ± standard error of the relative ratio of the amount of mRNA are illustrated in Fig. 6. The data for each region in Fig. 6 represent, from left to right, the results for negative control (non-administration), ds4, ds3, ds5, and ds8.

The test samples (ds3, ds5, and ds8) showed improved knockdown activity as compared with ds4.

### [Reference Example 1-3: Knockdown Test for Cervical Administration in Rat Using Alpha-Synuclein (Hereinafter Referred to as SNCA)-Targeted siRNA]

ds9, ds1, ds10, and ds2 diluted to 10 mg/mL with D-PBS were administered cervically to 10-week-old SD rats (male) at 30 µL (300 µg/head), and 7 days after administration, each region of the brain was collected and analyzed (N = 5). Thereafter, cDNA was prepared by the same method as in Reference Example 1-1.

Using this cDNA as a template for PCR reaction, the SNCA gene and, as a control, the ACTB gene were subjected to PCR reaction by the TaqMan (registered trademark) probe method using a QuantStudio 12K Flex real-time PCR system, the amounts of mRNA amplification thereof were measured, respectively, and a semiquantitative value of SNCA mRNA was calculated using the amount of ACTB mRNA amplification as an internal control. Further, the amounts of mRNA amplification of SNCA and ACTB in the negative control group were measured in the same manner, respectively, and a semiquantitative value of SNCA mRNA was calculated.

TaqMan probe Rn01425143_m1 (manufactured by Applied Biosystems) was used for measurement of the SNCA gene, Rn00667869_m1 (manufactured by Applied Biosystems) was used for measurement of the ACTB gene, and TaqMan (registered trademark) Gene Expression Master Mix was used as the reaction reagent, according to the attached protocol. The amount of target mRNA in the nucleic acid-administered individuals was calculated as a relative ratio when the amount of SNCA mRNA in the negative control group (non-administered group) was taken as 1. The results expressed as mean ± standard error of the relative ratio of the amount of mRNA are illustrated in Fig. 7. The data for each region in Fig. 7 represent, from left to right, the results for negative control (non-administration), ds9, ds1, ds10, and ds2.

The test samples (ds1, ds10, and ds2) showed improved knockdown activity as compared with ds9.

### [Reference Example 2: Knockdown Test for Intracerebroventricular Administration in Mouse Using HPRT1-Targeted siRNA]

ds11, ds12, ds13, ds14, ds15, and ds16 diluted to 25 mg/mL with D-PBS were administered intracerebroventricularly to 6-week-old ICR mice (male) at 5 µL (125 µg/head), and 7 days after administration, each region of the brain was collected and analyzed (N = 5). Here, ds14 was excluded from subsequent analysis because administration failure occurred in one individual, and ds15 was excluded because administration failure occurred in three individuals. For RNA extraction, RNA was purified using an RNeasy mini kit (manufactured by QIAGEN, 74104). Using Transcriptor First Strand cDNA synthesis Kit (manufactured by Roche Diagnostics, 04897030001), a reverse transcription reaction was performed according to the instructions attached to the kit to produce cDNA.

Using this cDNA as a template for PCR reaction, the HPRT1 gene and, as a control, the beta actin (hereinafter referred to as ACTB) gene were subjected to PCR reaction by the TaqMan (registered trademark) probe method using a CFX Connect (registered trademark) Real-Time System (manufactured by BIO-RAD), the amounts of mRNA amplification were measured, respectively, and a semiquantitative value of HPRT1 mRNA was calculated using the amount of ACTB mRNA amplification as an internal control. Further, the amounts of mRNA amplification of HPRT1 and ACTB in the negative control group were measured in the same manner, respectively, and a semiquantitative value of HPRT1 mRNA was calculated.

Using TaqMan probe Mm01545399_m1 (manufactured by Applied Biosystems) for measurement of the HPRT1 gene, and using Mm00607939_s1 (manufactured by Applied Biosystems) for measurement of the ACTB gene, TaqMan (registered trademark) Gene Expression Master Mix was used as the reaction reagent, according to the attached protocol. The amount of target mRNA in the nucleic acid-administered individuals was calculated as a relative ratio when the amount of HPRT1 mRNA in the negative control group (D-PBS-administered group) was taken as 1. The results expressed as mean ± standard error of the relative ratio of the amount of mRNA are illustrated in Fig. 8. The data for each region in Fig. 8 represent, from left to right, the results for negative control (D-PBS administration), ds11, ds12, ds13, ds14, ds15, and ds16.

All test samples (ds11, ds12, ds13, ds14, ds15, and ds16) showed knockdown activity as compared with the negative control (D-PBS).

### [Example 1: Knockdown Test for Intranasal Administration in Rat Using HPRT1-Targeted siRNA]

In Example 1, using living rats as subjects, intranasal administration into a cribriform foramen of a cribriform plate was performed, and after survival for 7 days following administration, the transferred amount of the oligonucleotide and the intensity of knockdown in each region of the brain of each specimen were evaluated.

An administration device 1A produced in Example 1 had the following specifications. Fig. 9 illustrates a configuration diagram of the administration device 1A produced in Example 1.

An inner tube catheter having an outer diameter of 0.47 mm, an inner diameter of 0.37 mm, and a total length of 125 mm (produced using PEEK as the material) was inserted into an outer tube catheter having an outer diameter of 0.6 mm, an inner diameter of 0.52 mm, and a total length of 115 mm (produced using PEEK as the material), and the distal end of a gap was bonded with a UV adhesive in a state where the inner tube catheter was exposed by 1 mm from the outer tube catheter toward the distal end side, thereby producing a double-tube catheter. Next, an 18G needle with a hub (product name: Terumo Non-Bevel Needle 18G 1 1/2, manufactured by Terumo Corporation) was cut at a position 10 mm from the proximal end of the exposed needle tube. Subsequently, the inner tube catheter of the produced double-tube catheter was inserted into the needle tube from the distal end of the needle tube of the hub after cutting, with the inner tube catheter being inserted from the distal end side at which 1 mm was exposed, and the gap was bonded with a UV adhesive. Next, at the end portion of the double-tube catheter opposite to the end portion bonded to the needle tube of the hub, the distal end of the inner tube catheter was cut such that 7 mm of the inner tube catheter was exposed from the outer tube catheter. Subsequently, a double-tube catheter structure was produced by filling and smoothing the step between the catheters near the cut part with an epoxy adhesive (product name: Bond Quick 30, manufactured by Konishi Co., Ltd.). The hub portion 20 of the produced administration device 1A is the hub part of the above-described 18G needle with a hub, and the cannula portion 30 is constituted by the double-tube catheter.

A needle tip (having an outer diameter of 0.1 mm, an inner diameter of 0.06 mm, a total length of 15 mm, and a blade surface length of 0.1 mm, and produced from a material SUS304) as the needle portion 10 was inserted into the distal end of the inner tube catheter and bonded with a UV adhesive such that the exposed length of the needle tip from the distal end of the inner tube catheter in the cannula portion 30 having the above configuration (the length of the puncture portion of the needle portion 10 in the major-axis direction) was set to 0.3 mm, thereby producing the administration device. A microsyringe (product name: Gastight Syringe 1705TLL, manufactured by Hamilton Company) was attached to the proximal end side of the hub portion 20 as the accommodation portion 40.

The produced administration device 1A was inserted into the nasal cavity using a guide catheter 100A as an insertion aid. Guide catheter 100A was formed of polyurethane and had a double-lumen shape with a total length of 45 mm, an outer diameter of φ1.35 mm, and inner diameters of φ0.45 mm and φ0.70 mm.

Example 1 was performed according to the test procedure shown below.

First, male rats (slc:SD rats (16 weeks old), manufactured by Japan SLC), which are subjects, were subjected to induction anesthesia (2 to 4%) using isoflurane inhalation anesthetic liquid (manufactured by Pfizer), and then the experiment was performed under continuous anesthesia (1.5 to 3%).

Next, the guide catheter was inserted into one nasal cavity of the subject at an angle parallel to the ridge line of the rat nose. During insertion, the distal end opening of the guide catheter was directed toward the cribriform plate side.

Next, the device was inserted into the guide catheter, and the olfactory mucosa was punctured by the exposed length of the needle tip (the length of the puncture portion). At that time, a point which is the position at which the length of insertion does not change when pushed in with a force of about 0.8 N, and a point at which the length of the part of the device inserted into the rat body was 25 to 33 mm were confirmed.

By confirming the above two points, it can be confirmed that the puncture portion of the device is in a state of being disposed within a cribriform foramen of the cribriform plate. More specifically, first, when the length of the part of the device inserted into the rat body is 25 to 33 mm, it can be confirmed that the puncture portion has reached at least the cribriform plate. In addition, it is confirmed that, when the position at which the length of the puncture portion does not change when pushed in with a force of about 0.8 N is reached, the puncture portion is in a state of being disposed within a cribriform foramen. This is because, since the rim of the nasal-cavity-side opening of the cribriform foramen has a recessed shape (that is, the nasal-cavity-side opening portion of the cribriform foramen has a tapered shape that gradually narrows from the opening end toward the brain side), the distal end of the needle portion 10 is inserted into the cribriform foramen while sliding along the rim by pushing during puncture, but, when pushing is continued, the stopper portion at the distal end of the cannula portion 30 comes into contact with the olfactory epithelium, resulting in a state where further pushing is no longer possible.

Next, 30 µL of each of the nucleic acid solutions was respectively administered by operating the microsyringe using a microsyringe pump (product name: IC3200, manufactured by KD Scientific) at 20 µL/min, and then allowed to stand for 5 minutes. For each administration, nucleic acid solutions prepared by individually dissolving three types of oligonucleotides, ds4, ds5, and ds6, in 1×PBS such that each oligonucleotide had a concentration of 60 mg/mL were used.

After survival for 7 days from the day of administration, euthanasia was performed by carbon dioxide inhalation. Thereafter, the skull was cut, and while the excised brain was cooled on a cool plate, each region was sampled, snap-frozen in liquid nitrogen, and then stored frozen.

Thereafter, the expression level of mRNA was measured by the same method as in Reference Example 1-1. The amount of target mRNA in the nucleic acid-administered individuals was calculated as a relative ratio when the amount of HPRT1 mRNA in the negative control group (non-administered group) was taken as 1. The results expressed as mean ± standard error of the relative ratio of the amount of mRNA are illustrated in Fig. 10. The data for each region in Fig. 10 represent, from left to right, the results for negative control (non-administration), ds4, ds5, and ds6.

The test samples (ds4, ds5, and ds6) showed knockdown activity as compared with the negative control (non-administered group).

### [Example 2: Knockdown Test for Intranasal Administration in Rat Using HPRT1-Targeted siRNA (Changing the Depth of Puncture)]

In Example 2, using living rats as subjects, intranasal administration into a cribriform foramen of a cribriform plate was performed, and after survival for 7 days following administration, the transferred amount of the oligonucleotide and the intensity of knockdown in each region of the brain of each specimen were evaluated.

In Example 2, intranasal administration into the cribriform foramina of the cribriform plate was performed at two different puncture depths. For shallow puncture, intranasal administration to living rats was performed by the same method as in Example 1. On the other hand, for deep puncture, the experiment was performed using an administration device 1B by the method described below.

In the administration device 1B produced in Example 2, the exposed length of the administration device 1A produced in Example 1 (0.3 mm) was adjusted to 0.85 mm in order to deeply puncture the cribriform foramen of the cribriform plate, and the other configurations had the same specifications. Fig. 9 illustrates a configuration diagram of the administration device 1B produced in Example 2.

The administration device 1B was inserted into the nasal cavity using the guide catheter 100A as an insertion aid. The guide catheter 100A was formed of polyurethane and had a double-lumen shape having a total length of 45 mm, an outer diameter of φ1.35, and inner diameters of φ0.45 and φ0.70.

In Example 2, in order to deeply puncture a cribriform foramen of the cribriform plate, the test was performed according to the test procedure shown below.

First, male rats (slc:SD rats (16 weeks old), manufactured by Japan SLC), which are subjects, were subjected to induction anesthesia (2 to 4%) using isoflurane inhalation anesthetic liquid (manufactured by Pfizer), and then the experiment was performed under continuous anesthesia (1.5 to 3%).

Next, the guide catheter was inserted into one nasal cavity of the subject while being angled at about 45° with respect to the ridge line of the rat nose. During insertion, the distal end opening of the guide catheter was directed toward the cribriform plate side and adjusted to be positioned at a cribriform foramen (having a total length of approximately 2 mm) near the skull where olfactory nerves converge on the deep side of the rat olfactory epithelium.

Next, the device was inserted into the guide catheter on the inner diameter φ0.45 side, the olfactory mucosa was punctured by the exposed length of the needle tip (the length of the puncture portion), and it was confirmed using a 16Ch X-ray CT apparatus (product name: Bright Speed Elite, manufactured by GE Healthcare) that the distal end of the puncture portion had reached the region deep in the olfactory epithelium where olfactory nerves converge.

Next, 30 µL of the nucleic acid solution was administered by operating the microsyringe using a microsyringe pump (product name: IC3200, manufactured by KD Scientific) at 20 µL/min, and then allowed to stand for 5 minutes. For administration, a nucleic acid solution prepared by dissolving an intrastrand C16-siHPRT1 (ds3) oligonucleotide in 1xPBS to a concentration of 60 mg/mL was used.

After survival for 7 days from the day of administration, euthanasia was performed by exsanguination under isoflurane anesthesia. Thereafter, the skull was cut, and while the excised brain was cooled on a cool plate, each region was sampled, snap-frozen in liquid nitrogen, and then stored frozen.

Thereafter, the expression level of mRNA was measured by the same method as in Reference Example 1-1. The amount of target mRNA in the nucleic acid-administered individuals was calculated as a relative ratio when the amount of HPRT1 mRNA in the negative control group (non-administered group) was taken as 1. The results expressed as mean ± standard error of the relative ratio of the amount of mRNA are illustrated in Fig. 11. In addition, the data for each region in Fig. 11 represent, from left to right, negative control (non-administered), ds3 (shallow), and ds3 (deep).

The test sample ds3 (deep) showed improved knockdown activity as compared with ds3 (shallow).

To the collected organ, 9 volumes of Cell Lysis buffer 2 (manufactured by TFS, FNN0021) and zirconia beads were added, the organ was disrupted using TissueLyser II (manufactured by QIAGEN), and the supernatant was collected after centrifugation. The supernatant and standard siRNA were converted to cDNA using TaqMan (registered trademark) MicroRNA RT Kit (manufactured by TFS, 4369016) and Primer 1. Using this cDNA as a template for PCR reaction, quantitative PCR reaction was performed using a QuantStudio 12K Flex real-time PCR system (manufactured by TFS). Primer 2, Primer 3, and TaqMan (registered trademark) MGB probe 1 (manufactured by TFS, 4316033) were used in the reaction. The base sequences of each primer and probe are shown in Table 13.

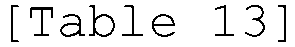

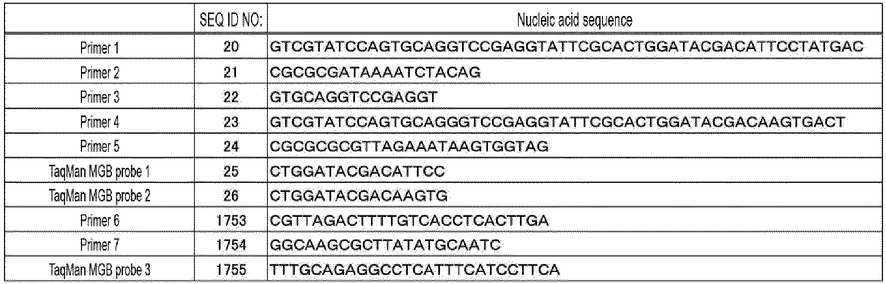

The results expressed as mean ± standard error of the amount of nucleic acid in each brain region are illustrated in Fig. 12. In addition, the data for each region in Fig. 12 represent, from left to right, ds3 (shallow), and ds3 (deep).

The amount of nucleic acid is considered to be the amount of administered nucleic acid transferred to each brain region (olfactory bulb, striatum, and cerebral cortex (cervical side)). The test sample ds3 (deep) showed an improved nucleic acid transfer amount as compared with ds3 (shallow).

### [Example 3: Knockdown Test for Intranasal Administration in Cynomolgus Monkey Using HPRT1-Targeted siRNA]

In Example 3, using living cynomolgus monkeys as subjects, intranasal administration of the nucleic acid solution (ds3) into a cribriform foramen of a cribriform plate was performed, and after survival for 7 days following the final administration, the transferred amount of the oligonucleotide and the intensity of knockdown in each region of the brain of each specimen were evaluated.

The administration device used in Example 3 had the following specifications. Fig. 13 shows configuration diagrams of the administration device 1C produced in Example 3 ((a) in Fig. 13) and a guide catheter 100B ((b) in Fig. 13).

As the administration device 1C, devices having different exposed lengths of the needle tip (exposed lengths: 1.3 mm to 4.2 mm) were prepared.

First, an inner tube catheter having an outer diameter of 0.47 mm, an inner diameter of 0.37 mm, and a total length of 165 mm (produced using PEEK as the material) was inserted into an outer tube catheter having an outer diameter of 0.6 mm, an inner diameter of 0.52 mm, and a total length of 155 mm (produced using PEEK as the material), and the distal end of a gap was bonded with a UV adhesive in a state where the inner tube catheter was exposed by 1 mm from the outer tube catheter toward the distal end side, thereby producing a double-tube catheter. Next, an 18G needle with a hub (product name: Terumo Non-Bevel Needle 18G 1 1/2, manufactured by Terumo Corporation) was cut at a position 10 mm from the proximal end of the exposed needle tube. Subsequently, the inner tube catheter of the produced double-tube catheter was inserted into the needle tube from the distal end of the needle tube of the hub after cutting, with the inner tube catheter being inserted from the distal end side at which 1 mm was exposed, and the gap was bonded with a UV adhesive. Next, at the end portion of the double-tube catheter opposite to the end portion bonded to the needle tube of the hub, the distal end of the inner tube catheter was cut such that 7 mm of the inner tube catheter was exposed from the outer tube catheter. Subsequently, a double-tube catheter structure was produced by filling and smoothing the step between the catheters near the cut part with an epoxy adhesive (product name: Bond Quick 30, manufactured by Konishi Co., Ltd.). The hub portion 20 of the produced administration device 1C is the hub part of the above-described 18G needle with a hub, and the cannula portion 30 is constituted by the double-tube catheter.

A needle tip (having an outer diameter of 0.2 mm, an inner diameter of 0.12 mm, a total length of 15 mm, and a blade surface length of 0.2 mm, and produced from a material SUS304) as the needle portion 10 was inserted into the distal end of the inner tube catheter and bonded with a UV adhesive such that the exposed length of the needle tip from the distal end of the inner tube catheter in the cannula portion 30 having the above configuration (the length of the puncture portion 12 of the needle portion 10 in the major-axis direction) was set to each of the lengths ranging from 1.3 mm to 4.2 mm shown in Table 14, thereby producing each administration device. A microsyringe (product name: Gastight Syringe 1002TLL, manufactured by Hamilton Company) was attached to the proximal end side of the hub portion 20 of each device as the accommodation portion 40.

**[Table 14]**

| Nucleic acid solution | Monkey No. | Length of puncture portion in major-axis direction (mm) | | | |
|---|---|---|---|---|---|
| | | First time | Second time | Third time | Fourth time |
| aCSF | 1 | 1.3 | 2.0 | 2.0 | 2.0 |
| | 2 | 4.2 | 4.2 | 3.5 | 3.5 |
| | 3 | 3.3 | 3.1 | 3.3 | 3.5 |
| ds3 | 4 | 2.8 | 3.5 | 3.1 | 3.3 |
| | 5 | 3.9 | 3.9 | 3.9 | 4.2 |
| | 6 | 2.8 | 2.8 | 2.4 | 2.5 |

The produced administration device 1C was inserted into the nasal cavity using the guide catheter 100B as an insertion aid. The guide catheter 100B was formed of SUS304 and had a total length of 90 mm, an outer diameter of φ0.82mm, an inner diameter of φ0.68mm, and a bending angle of a curved part on the distal end side of 45°. In addition, the guide catheter used was one in which a straight portion was provided with a spiral cut to impart flexibility and enable bending.

Example 3 was performed according to the test procedure shown below.

First, cynomolgus monkeys, which are subjects (4 years and 0 months to 4 years and 9 months old, male), were anesthetized. The anesthetic agents and administration methods were as follows. In addition, anesthesia and administration were each performed four times for each subject, and administration was performed using devices having the lengths of the puncture portion shown in Table 14 for each time.
- Induction anesthesia: Ketalar for intramuscular injection 500 mg, Administration route: intramuscular administration, Administration amount: 10 mg/kg (0.2 mL/kg).
- Maintenance anesthesia: Propofol injection 1% for animal "Mylan", Administration route: intravenous administration, Administration amount: 0.5 to 30 mg/kg/hr (0.05 to 3 mL/kg/hour).

Next, contrast imaging was performed using a 16-channel X-ray CT apparatus (product name: Bright Speed Elite, manufactured by GE Healthcare), and the guide catheter was inserted into one nasal cavity of the subject while confirming contrast images. At the time of insertion, the distal end opening of the guide catheter was directed toward the cribriform plate side, adjusted such that the distal end was positioned immediately below the cribriform plate, and fixed. Next, the thickness of the cribriform plate positioned on a straight line extending from the opening portion of the guide catheter was measured. Based on the measured thickness of the cribriform plate, the exposed length of the needle tip (length of the puncture portion) capable of puncturing 60% or more was calculated, and a puncture portion having a length corresponding to 60% to 100% of the thickness of the cribriform plate was selected.

Next, the selected device was inserted into the guide catheter, the olfactory mucosa was punctured by the exposed length of the needle tip (the length of the puncture portion), and it was confirmed with the above X-ray CT apparatus that the needle tip was punctured into a cribriform foramen. 2 mL of nucleic acid solution was administered by operating the microsyringe using a microsyringe pump (product name: IC3200, manufactured by KD Scientific) at 100 uL/min. For administration, a nucleic acid solution prepared by dissolving the ds3 oligonucleotide in aCSF to a concentration of 60 mg/mL was used.

Using the same method once every week, 2 mL per administration was administered to the same nostril as that used for the first administration at a rate of 100 uL/min. A total of four administrations, that is, a total of 8 mL, were performed. After survival for 7 days from the fourth and final administration day, thoracotomy was performed under isoflurane anesthesia, the descending aorta was blocked, a catheter was inserted into the left ventricle, and perfusion with heparin-containing physiological saline was performed, thereby carrying out euthanasia by exsanguination. Thereafter, the skull was cut, the brain was cut in the coronal plane direction at a width of 6 mm using a brain matrix, each region of the brain was sampled, snap-frozen in liquid nitrogen, and then stored frozen. In addition, the spinal cord (cervical spinal cord, thoracic spinal cord, and lumbar spinal cord) was also sampled and similarly stored frozen. aCSF was administered to the comparative control group, and the same operation was performed.

Thereafter, cDNA preparation and quantitative PCR were performed for each brain region and the spinal cord (cervical spinal cord, thoracic spinal cord, and lumbar spinal cord) by the same method as in Reference Example 1-1. TaqMan probe Hs99999909_m1 (manufactured by Applied Biosystems) was used for measurement of the HPRT1 gene, and Hs01060665_g1 (manufactured by Applied Biosystems) was used for measurement of the ACTB gene. The results expressed as mean ± standard error of the relative ratio of the amount of mRNA are illustrated in Figs. 14-1 and 14-2. In addition, the data for each region in Figs. 14-1 and 14-2 represent, from left to right, negative control (aCSF) and ds3.

The test sample (ds3) showed significant knockdown activity of the HPRT1 gene in each region of the brain as compared with the comparative control (aCSF).

In addition, the transferred amount of nucleic acid in each brain region was measured by the same method as in Example 2. The results expressed as mean ± standard error of the amount of nucleic acid in each brain region and spinal cord (cervical spinal cord, thoracic spinal cord, and lumbar spinal cord) are illustrated in Figs. 15-1 and 15-2. The test samples (ds3) showed significant nucleic acid transfer in each region of the brain. In addition, the data for each region in Fig. 15-1 represent, from left to right, the result of the left brain and the result of the right brain.

### [Example 4]

In Example 4, using living rats as subjects, intranasal administration into a cribriform foramen of a cribriform plate was performed, and after survival for 7 days following administration, the transferred amount of the administered substance and the intensity of knockdown in each region of the brain of each specimen were evaluated.

In Example 4, administration was performed by two types of methods, intranasal administration and nasal drop administration. Regarding intranasal administration, intranasal administration to living rats was performed by the same method as in Example 2 using the administration device 1B produced in Example 2 (length of puncture portion: 0.85 mm) (refer to Fig. 5). On the other hand, regarding nasal drop administration, Pipetman (manufactured by GILSON) was used as an administration device for nasal drop administration, and the experiment was performed by the method described below. It should be noted that, for the intranasal administration and the nasal drop administration, two types of nucleic acid solutions were used, each prepared by dissolving each of the oligonucleotides ds1 and ds2 in aCSF to a concentration of 60 mg/mL. In addition, administration of aCSF alone was also performed by the same method as the administration of the nucleic acid solutions described below.

First, male rats (slc:SD rats (16 weeks old), manufactured by Japan SLC), which are subjects, were subjected to induction anesthesia (2 to 4%) using isoflurane inhalation anesthetic liquid (manufactured by Pfizer), and then the experiment was performed under continuous anesthesia (1.5 to 3%) in a state where a small-animal anesthesia mask was attached.

Next, the nasal drop administration device was operated to fill the tip with 5 µL of the nucleic acid solution. Next, the small-animal anesthesia mask attached to the rat nose was removed, the rat was placed in a supine position, the tip of the pipette was not inserted into the nostril but was brought to the vicinity of the nostril on the administration side, and the solution was slowly pushed out over 2 to 3 minutes. After administration of 5 µL, the rat was maintained in a supine position in the anesthesia box for 2 minutes. The above method was repeated six times for the same nostril used for the first administration, for a total administration of 30 µL. After completion of administration, the rat was maintained in a supine position in the anesthesia box for 5 minutes.

After survival for 7 days from the day of administration, euthanasia was performed by exsanguination under isoflurane anesthesia. Thereafter, the skull was cut, and while the excised brain was cooled on a cool plate, each region was sampled, snap-frozen in liquid nitrogen, and then stored frozen.

Thereafter, cDNA preparation and quantitative PCR were performed by the same method as in Reference Example 1-3. The results expressed as mean ± standard error of the relative ratio of the amount of mRNA are illustrated in Fig. 16. In addition, the data for each region in Fig. 16 represent, from left to right, the results of aCSF, ds1, and ds2 (each by nasal drop administration) and the results of aCSF, ds1, and ds2 (each by intranasal administration).

Intranasal administration showed significant SNCA gene knockdown activity in each region of the brain as compared with nasal drop administration.

In addition, the transferred amount of nucleic acid in each brain region was measured by the same method as in Example 2. Conversion into cDNA was performed using TaqMan (registered trademark) MicroRNA RT Kit (manufactured by TFS, 4369016) and Primer 4. Using this cDNA as a template for PCR reaction, quantitative PCR reaction was performed using a QuantStudio 12K Flex real-time PCR system. Primer 5, primer 3, and TaqMan (registered trademark) MGB probe 2 (manufactured by TFS, 4316033) were used in the reaction. It should be noted that the base sequences of the respective primers and probes are shown in Table 13.

The results expressed as mean ± standard error of the amount of nucleic acid in each brain region are illustrated in Fig. 17. Intranasal administration exhibited an improved transfer amount of nucleic acid in each region of the brain as compared with nasal drop administration. In addition, the data for each region in Fig. 17 represent, from left to right, the results of ds1 and ds2 (each by nasal drop administration) and the results of ds1 and ds2 (each by intranasal administration).

### [Example 5: In Vitro Evaluation of siSNCA]

### <Design of siRNA>

A set of 405 siRNAs targeting the human synuclein alpha gene (SNCA; human NCBI refseq ID NM_000345.4; NCBI GeneID: 6622) was designed using a custom Perl script. For the design of siRNA, the base sequences described in WO 2022/072447 were also referenced. Scoring of the designed siRNAs was performed with reference to the s-Biopredsi method (Nucleic Acids Research (2007) 35(18), e123) and the siDirect method (Nucleic Acids Research (2004) 32 (Web Server Issue), W124-W129), and off-target evaluation was performed using GGGenome (manufactured by Retriever Co., Ltd.) .

### <Cell Culture>

BE(2)-C cells (KAC Co., Ltd.) were cultured at 37°C under 5% CO₂ using a medium prepared by adding 15% heat-inactivated fetal bovine serum (hereinafter also referred to as "FBS") to a solution obtained by mixing an EMEM medium (manufactured by ATCC) and a Ham's F-12 medium (manufactured by TFS) in equal amounts. Subculture of the cells was performed twice per week by washing the cells with PBS (manufactured by TFS) and then detaching the cells by treatment with 0.25% Trypsin-EDTA (manufactured by TFS). Cells passaged 6 or 7 times were used for the assay.

### <Addition of siRNA>

A 500 nmol/L siRNA PBS solution was diluted with Opti-MEM medium (manufactured by TFS) to 50 pmol/L, 500 pmol/L, or 5 nmol/L in a 384 well black bottom clear plate (manufactured by Greiner) to make 10 µL. Subsequently, Lipofectamine RNAiMAX (manufactured by TFS) was diluted 80-fold with Opti-MEM medium, and 10 µL was added to the plate to which siRNA had been added. A suspension of BE(2)-C cells prepared to 1.33 × 10⁵ cells/mL was added thereto in an amount of 30 µL to be 4000 cells/well. After this plate was left standing at room temperature for about 15 minutes, the plate was cultured overnight at 37°C under 5% CO₂.

### <Evaluation of Knockdown Activity>

Cell lysis, the reverse transcription reaction, and the qPCR reaction were performed in accordance with the standard protocol of the TaqMan Fast Advanced Cells-to-CT Kit (manufactured by TFS). Using cDNA prepared from cells lysed by the kit, TaqMan probe Hs00240906_m1 SNCA (manufactured by Applied Biosystems) for measurement of the SNCA gene, and Hs01060665_g1 (manufactured by Applied Biosystems) for measurement of the ACTB gene, the SNCA gene and, as a control, the ACTB gene were subjected to PCR reaction using a QuantStudio 7 Flex (manufactured by Applied Biosystems), and the respective Ct values were measured. A semiquantitative value of SNCA mRNA was calculated using the Ct value of ACTB as an internal control. Further, the Ct values of SNCA and ACTB in the negative control group (siRNA 0 pmol) were measured in the same manner, respectively, and a semiquantitative value of SNCA mRNA was calculated. The results for siRNAs (ds17 to 171) for which the average of two measured values showed knockdown activity of 70% or more at 100 pmol/L or 1 nmol/L are shown in Tables 15-1 to 15-4.

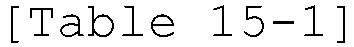

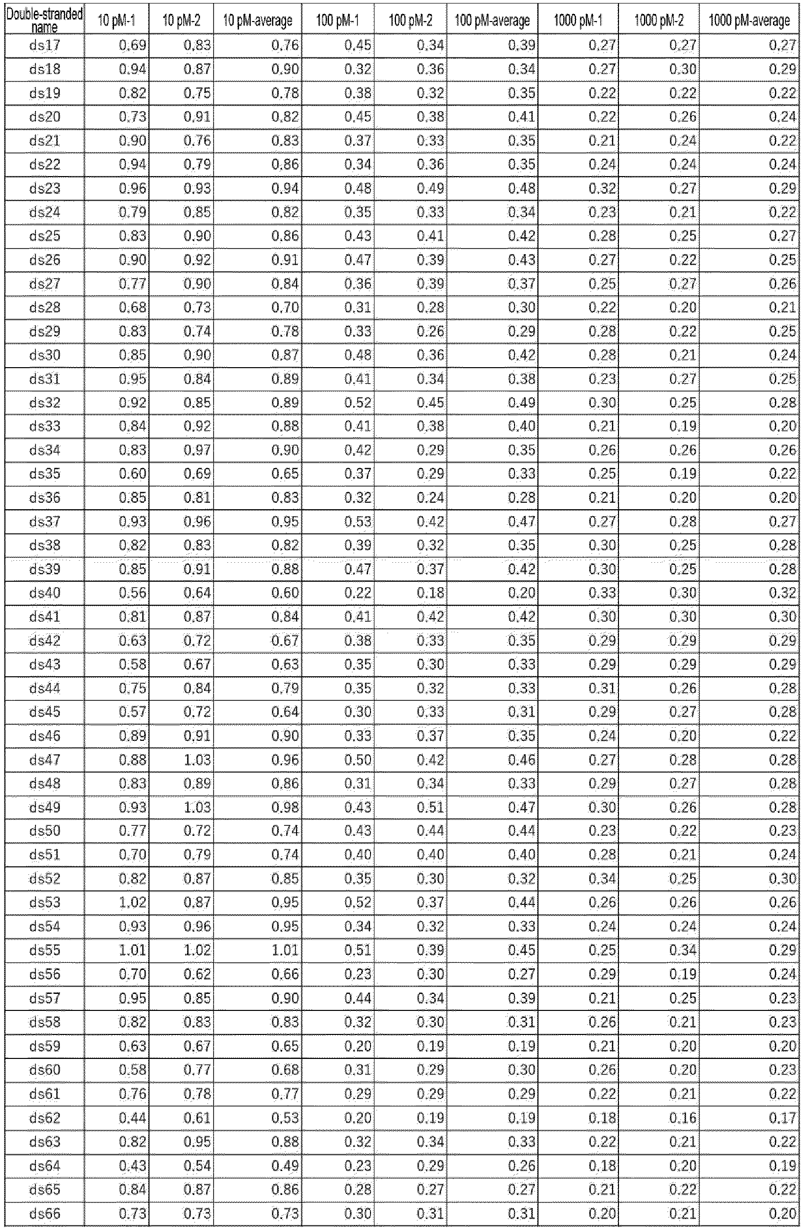

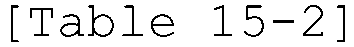

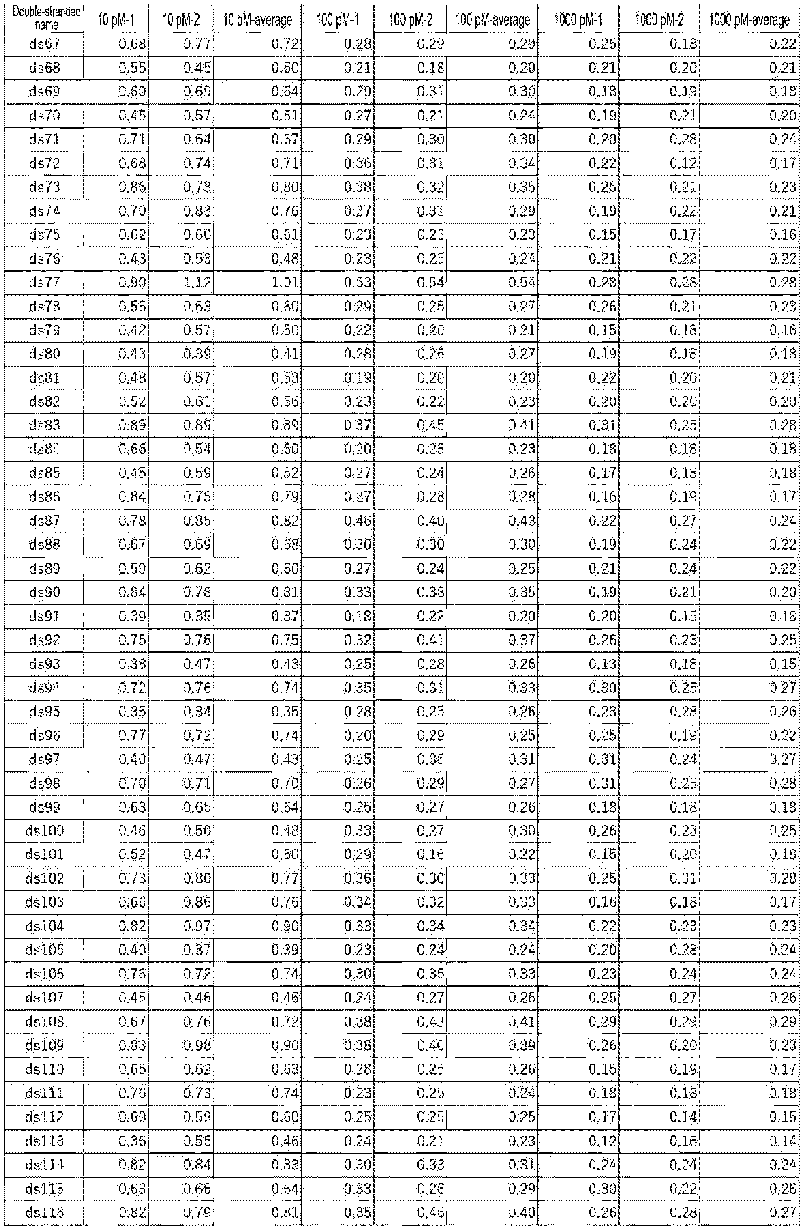

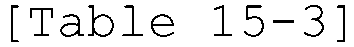

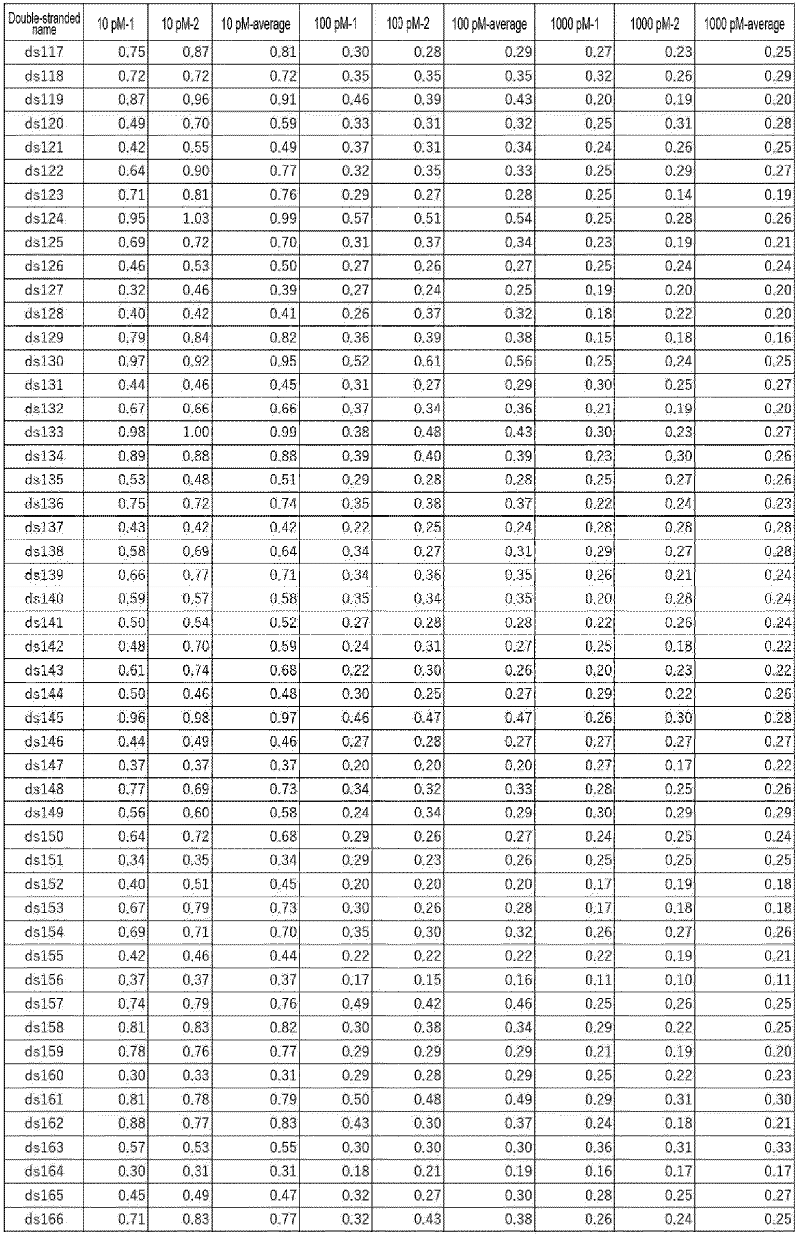

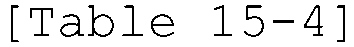

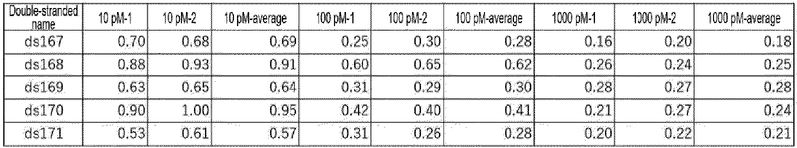

In vitro evaluation of ds9 and ds172 to 249 was performed according to the procedure described above [In Vitro Evaluation of siSNCA]. The results are shown in Tables 16-1 and 16-2.

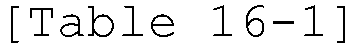

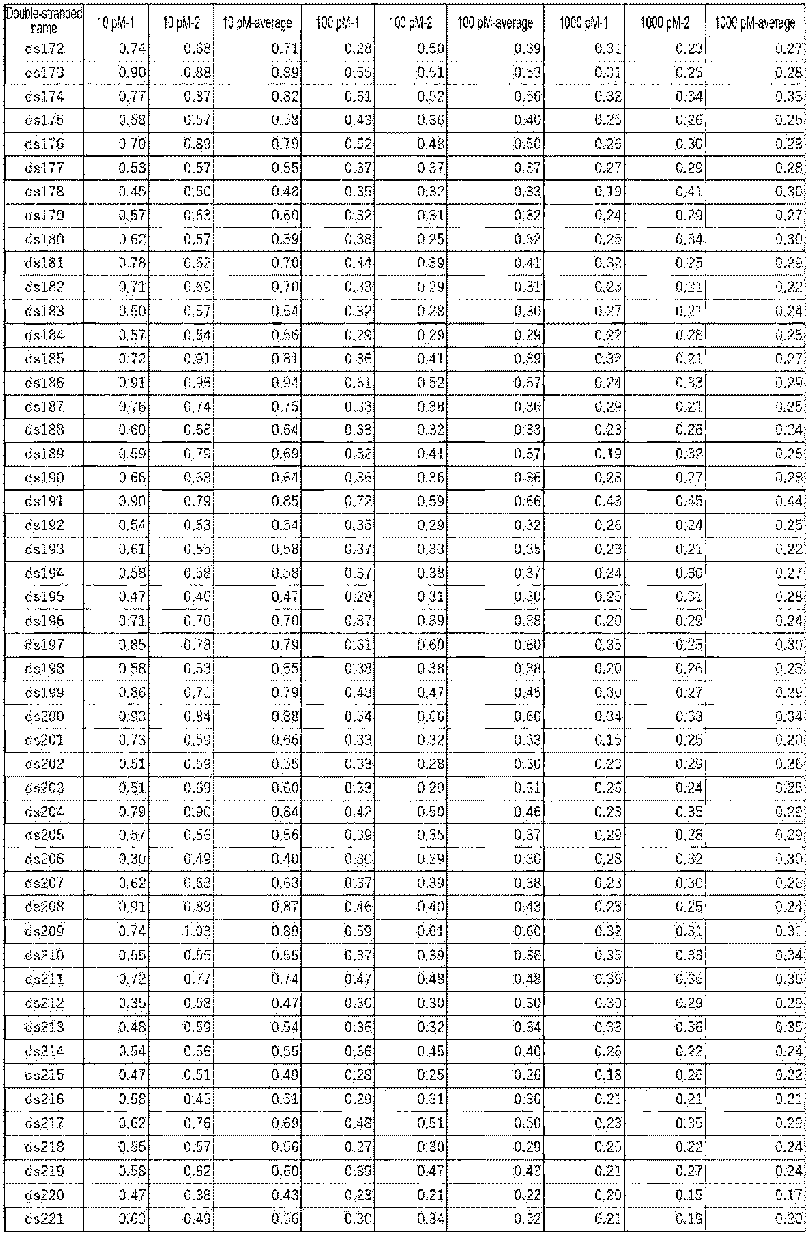

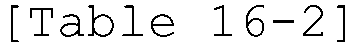

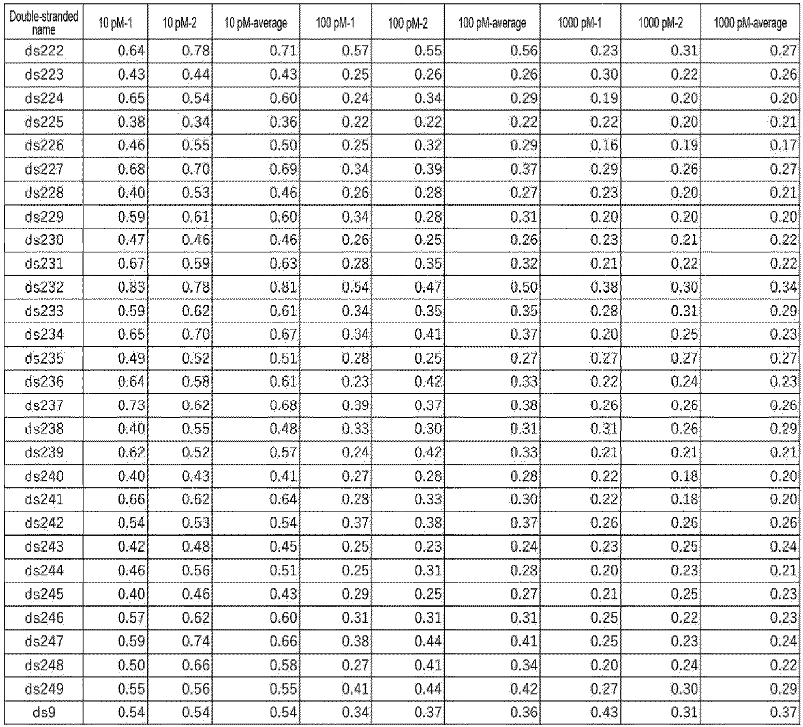

### [Example 6: In Vitro Evaluation of siHTT]

### <Design of siRNA>

A set of 236 siRNAs was designed with reference to the base sequences in WO 2022/072447.

### <Evaluation of Knockdown Activity>

By a method according to Example 5, using TaqMan probe Hs00918174_m1 (manufactured by Applied Biosystems) for measurement of the HTT gene and Hs01060665_g1 (manufactured by Applied Biosystems) for measurement of the ACTB gene, the HTT gene and, as a control, the ACTB gene were subjected to PCR reaction, and the respective Ct values were measured. A semiquantitative value of HTT mRNA was calculated using the Ct value of ACTB as an internal control. Further, the Ct values of HTT and ACTB in the negative control group (siRNA 0 pmol) were measured in the same manner, respectively, and a semiquantitative value of HTT mRNA was calculated. The results for siRNAs (ds274 to 402) for which the average of two measured values showed knockdown activity of 50% or more at 100 pmol/L or 60% or more at 1 nmol/L are shown in Tables 17-1 to 17-3.

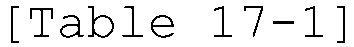

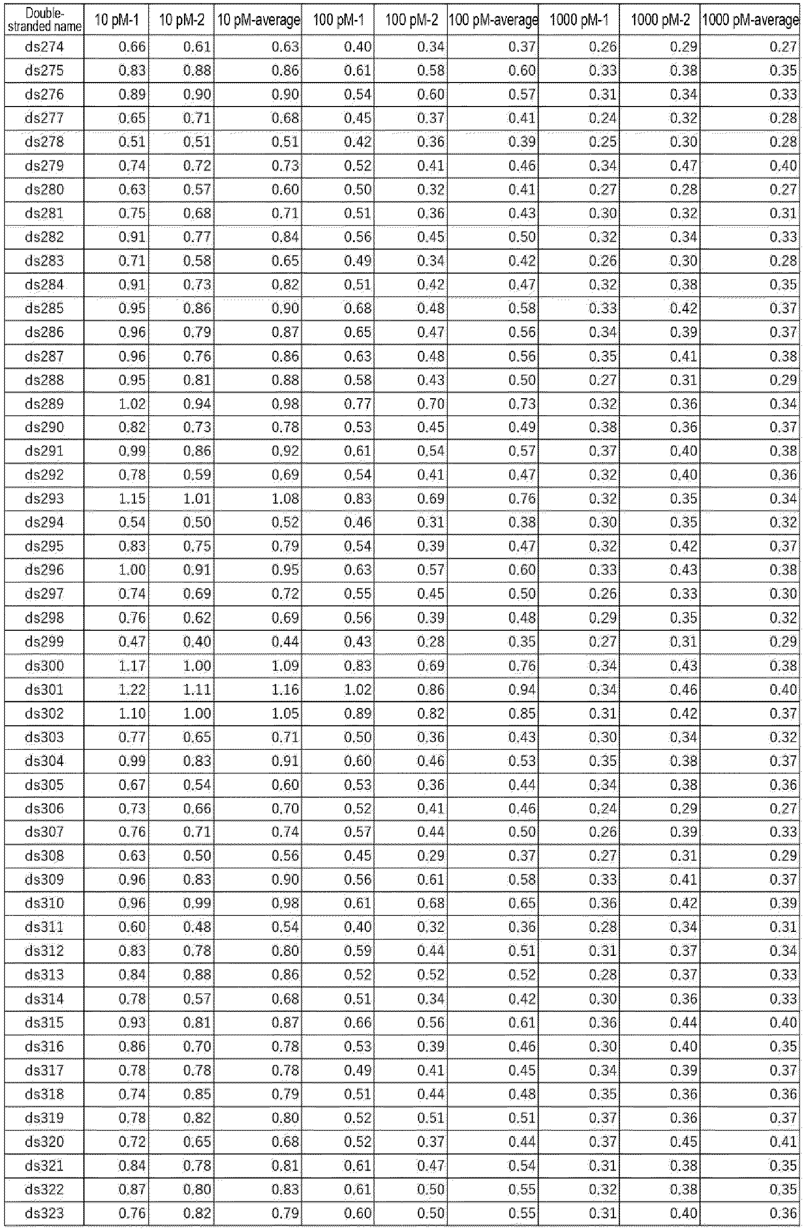

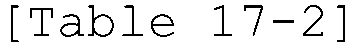

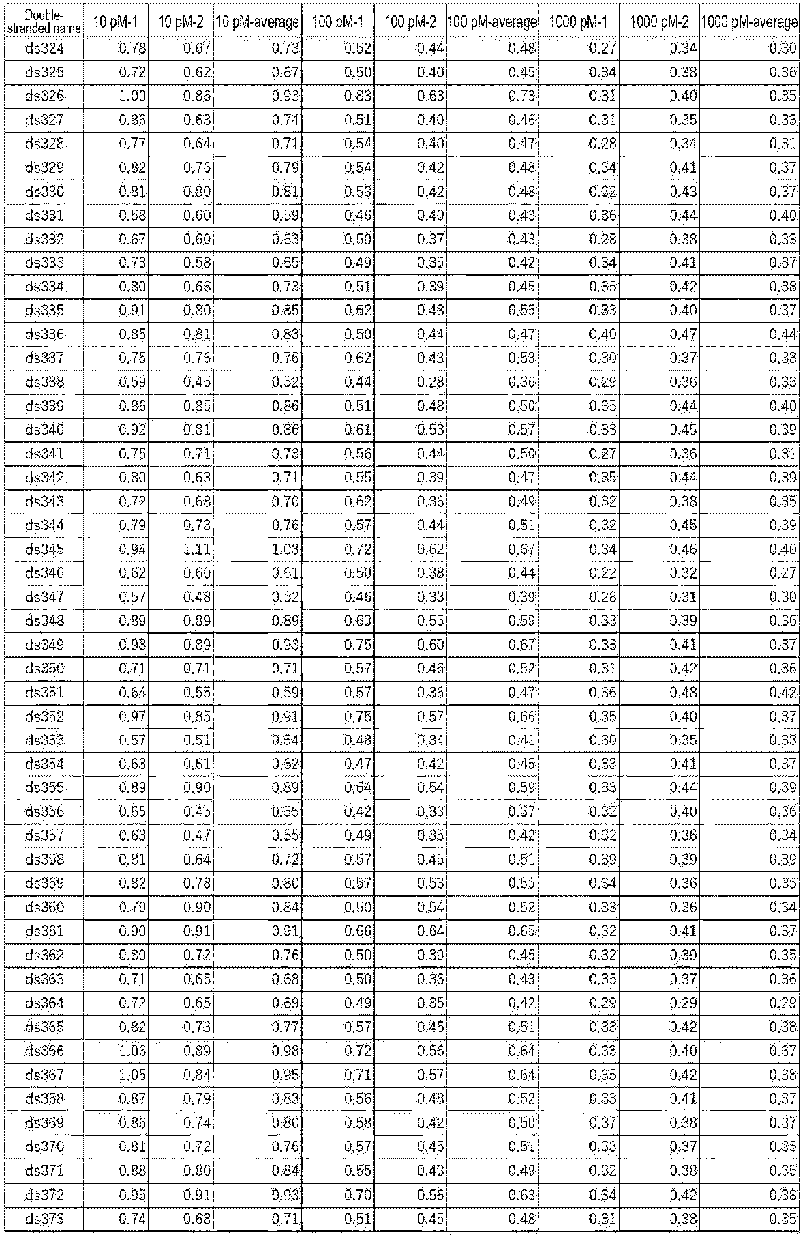

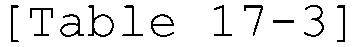

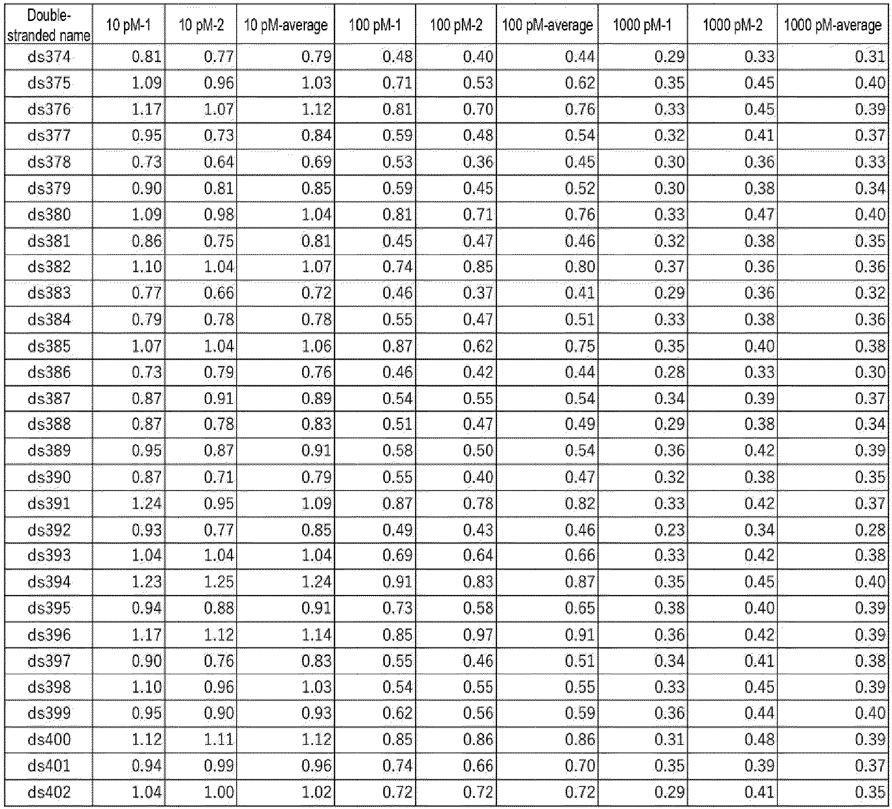

Similarly, in vitro evaluation of ds403 to 461 was performed according to the method described above [In Vitro Evaluation of siHTT]. The results are shown in Tables 18-1 and 18-2.

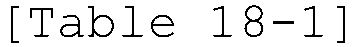

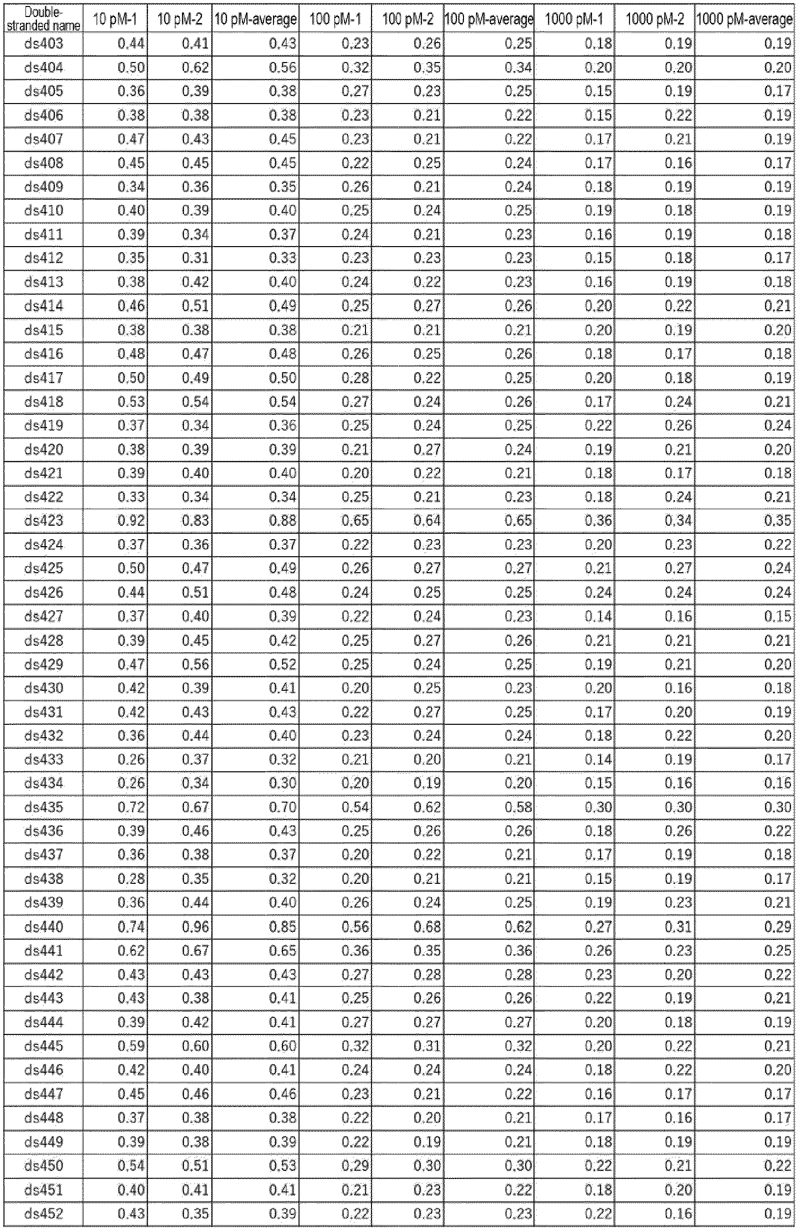

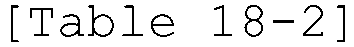

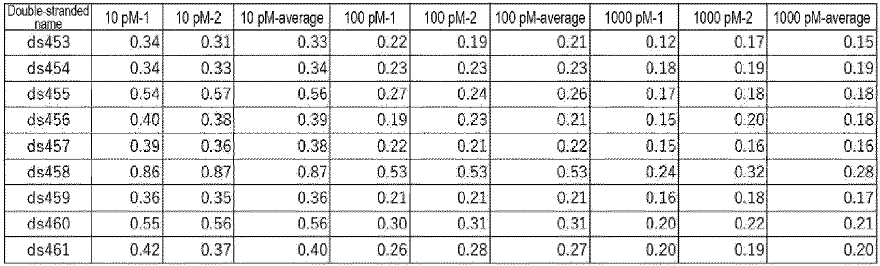

### [Example 7]

In Example 7, using human SNCA transgenic mice (Neuroscience Research (2012), 73(2), 173-177) as subjects, intracerebroventricular administration was performed, and after survival for 7 days following administration, the transferred amount of the administered substance and the intensity of knockdown in each region of the brain of each specimen were evaluated. Administration was performed by the same method as in Reference Example 2.

For intracerebroventricular administration, 5 µL of a nucleic acid solution prepared by dissolving each of the oligonucleotides ds2, ds254, and ds257 to ds273 in aCSF to a concentration of 20 mg/mL was administered. In addition, administration of aCSF alone was also performed by the same method as the administration of the nucleic acid solutions.

7 days after administration, each region of the brain was collected and analyzed (N = 4). Thereafter, RNA was purified by the same method as in Reference Example 2. Using FastGene Scriptase II (manufactured by NIPPON Genetics, NE-LS65), a reverse transcription reaction was performed according to the instructions attached to the kit to prepare cDNA.

Using this cDNA as a template for PCR reaction, the human SNCA gene and, as a control, the ACTB gene were subjected to PCR reaction by the TaqMan (registered trademark) probe method using a QuantStudio 12K Flex real-time PCR system, the amounts of mRNA amplification thereof were measured, respectively, and a semiquantitative value of SNCA mRNA was calculated using the amount of ACTB mRNA amplification as an internal control. Further, the amounts of mRNA amplification of human SNCA and ACTB in the negative control group were measured in the same manner, respectively, and a semiquantitative value of human SNCA mRNA was calculated.

TaqMan probe Hs00240907_m1 (manufactured by Applied Biosystems) was used for measurement of the human SNCA gene, Mm00607939_s1 (manufactured by Applied Biosystems) was used for measurement of the ACTB gene, and TaqMan (registered trademark) Gene Expression Master Mix was used as the reaction reagent, according to the attached protocol. The amount of target mRNA in nucleic acid-administered individuals was calculated as a relative ratio to the amount of human SNCA mRNA in the negative control group (aCSF group). The results expressed as mean ± standard error of the relative ratio of the amount of mRNA are illustrated in Fig. 18. The data for each region in Fig. 18 represent, from left to right, the results for negative control (aCSF administration), ds2, ds254, and ds257 to ds273.

All test samples showed knockdown activity as compared with the aCSF-administered group, and in particular ds2, ds254, ds257, ds259, ds261, ds263, ds267, and ds268 showed strong knockdown activity.

### [Example 8]

In Example 8, using male rats (slc:SD rats, 13 weeks of age, manufactured by Japan SLC, Inc.) as subjects, intranasal administration into a cribriform foramen of a cribriform plate was performed, and after survival for 7 days following administration, the transferred amount of the administered substance and the intensity of knockdown in each region of the brain of each specimen were evaluated.

In Example 8, in order to accommodate the use with a 3D micro X-ray CT apparatus described below, an administration device 1D was used, in which the total length (115 mm) of the outer tube catheter of the administration device 1A produced in Example 1 was changed to 915 mm, and the total length (125 mm) of the inner tube catheter was changed to 930 mm. The configuration other than the total lengths of the outer tube catheter and inner tube catheter was the same as that of administration device 1A produced in Example 1. Fig. 19 illustrates configuration diagrams of the administration device 1D produced in Example 8.

Using the administration device 1D, living rats were contrast-imaged under triple mixed anesthesia using a 3D micro X-ray CT apparatus R_mCT2 (manufactured by Rigaku), and the guide catheter was inserted into one nasal cavity of the subject while confirming contrast images. Subsequently, the administration device 1D (length of puncture portion: 0.85 mm) was inserted into the guide catheter, and after confirming that the needle tip was in a state of passing through the cribriform foramen without penetrating into the brain, intranasal administration was performed. For intranasal administration, each of the oligonucleotides ds2 and ds250 to 253 was dissolved in aCSF to a concentration of 60 mg/mL, respectively, and 30 µL thereof was administered.

7 days after administration, each region of the brain was collected, cDNA was then prepared by the same method as in Example 7, and quantitative PCR was performed by the same method as in Example 4. The results expressed as mean ± standard error of the relative ratio of the amount of mRNA are illustrated in Fig. 20. In addition, the data for each region in Fig. 20 represent, from left to right, the results for non-administration, ds2, and ds250 to 253.

ds2 (5'EPA-siSNCA) showed significant knockdown activity of the SNCA gene in each region of the brain as compared with ds250 and ds251, which have low phosphate moiety modification, or ds252 and ds253, which have low sugar moiety modification rates. However, although ds253, which has a low sugar moiety modification rate in the sense strand, did not reach the level of ds2, it showed higher knockdown activity as compared with ds250, ds251, and ds252.

In addition, the transferred amount of nucleic acid in each brain region was measured by the same method as in Example 4. The results expressed as mean ± standard error of the amount of nucleic acid in each brain region are illustrated in Fig. 21. In addition, the data for each region in Fig. 21 represent, from left to right, the results for ds2 and ds250 to 253. The test samples ds2 (5'EPA-siSNCA) showed significant nucleic acid transfer in each region of the brain.

### [Example 9]

In Example 9, using male rats (slc:SD rats, 13 weeks of age, manufactured by Japan SLC, Inc.) as subjects, intranasal administration into a cribriform foramen of a cribriform plate was performed, and after survival for 7 days following administration, the transferred amount of the administered substance and the intensity of knockdown in each region of the brain of each specimen were evaluated.

In Example 9, intranasal administration was performed in the same manner as in Example 8. For intranasal administration, each of the oligonucleotides ds2 and ds254 to 257 was dissolved in aCSF to a concentration of 60 mg/mL, respectively, and 30 µL thereof was administered.

7 days after administration, each region of the brain was collected, and then cDNA preparation and quantitative PCR were performed by the same method as in Example 8. The results expressed as mean ± standard error of the relative ratio of the amount of mRNA are illustrated in Fig. 22. In addition, the data for each region in Fig. 22 represent, from left to right, the results for non-administration, ds2, and ds254 to 257.

The test samples (ds2 and ds254 to 257) showed significant knockdown activity of the SNCA gene in each region of the brain as compared with the non-administered group.

### [Example 10]

In Example 10, using live cynomolgus monkeys as subjects, intranasal administration of a nucleic acid solution (ds2) to cribriform foramina of the cribriform plate was performed, and after survival for 56 days from the initial administration day, the transferred amount of oligonucleotide in each region of the brain of each specimen and the intensity of knockdown were evaluated (N = 3).

Administration was performed at 2 mL by the same method as in Example 3. For administration, a nucleic acid solution prepared by dissolving the ds2 oligonucleotide in aCSF to a concentration of 60 mg/mL was used.

For the group receiving two administrations, by the same method 1 week after the first administration, 2 mL per administration was administered to the same nostril as that used for the first administration at a rate of 100 µL/min. After survival for 56 days from the initial administration day, each region of the brain was sampled by the same method as in Example 3, snap-frozen in liquid nitrogen, and then stored frozen. In addition, CSF was also sampled and similarly stored frozen. In addition, the spinal cord (cervical spinal cord, thoracic spinal cord, and lumbar spinal cord) was also sampled and similarly stored frozen. aCSF was administered to the comparative control group, and the same operation was performed.

Thereafter, cDNA preparation and quantitative PCR were performed for each brain region by the same method as in Example 7. TaqMan probe Mf02793033_m1 (manufactured by Applied Biosystems) was used for measurement of the SNCA gene, and Mf04354341_g1 (manufactured by Applied Biosystems) was used for measurement of the ACTB gene. The results expressed as mean ± standard error of the relative ratio of the amount of mRNA are illustrated in Fig. 23-1. In addition, the data for each region in Fig. 23-1 represent, from left to right, negative control (aCSF), single administration of ds2, and two administrations of ds2. Further, the graph on the left in Fig. 23-1 shows the results on the administration side, and the graph on the right shows the results on the non-administration side. Here, the administration side means the brain to which the oligonucleotide has been administered, out of the right brain and the left brain, and the non-administration side means the other brain to which the oligonucleotide has not been administered. The same analysis was performed for the spinal cord (cervical spinal cord, thoracic spinal cord, and lumbar spinal cord). The results are illustrated in Fig. 23-2.

The test samples (single administration of ds2 and two administrations of ds2) showed significant knockdown activity of the SNCA gene in each region of the brain and spinal cord as compared with the comparative control (aCSF) .

In addition, the transferred amount of nucleic acid in each brain region was measured by the same method as in Example 4. The results expressed as mean ± standard error of the amount of nucleic acid in each brain region are illustrated in Fig. 24-1. The test samples (single administration of ds2 and two administrations of ds2) showed significant nucleic acid transfer in each region of the brain. In addition, the data for each region in Fig. 24-1 represent, from left to right, single administration of ds2, and two administrations of ds2. Further, the graph on the left in Fig. 24-1 shows the results on the administration side, and the graph on the right shows the results on the non-administration side. The same analysis was performed for the spinal cord (cervical spinal cord, thoracic spinal cord, and lumbar spinal cord). The results are illustrated in Fig. 24-2.

Furthermore, the amount of α-synuclein protein contained in the brain extract was quantified. For quantification, LEGEND MAX Human α-Synuclein (Colorimetric) ELISA Kit (manufactured by Biolegend, 448607) was used, and measurement was performed according to the instructions attached to the kit. The results expressed as mean ± standard error of the amount of α-synuclein protein in each brain region and CSF are illustrated in Fig. 25-1. In addition, the data for each region in Fig. 25-1 represent, from left to right, negative control (aCSF), single administration of ds2, and two administrations of ds2. Further, the graph on the left in Fig. 25-1 shows the results on the administration side, and the graph on the right shows the results on the non-administration side.
The same analysis was performed for the spinal cord extract (cervical spinal cord, thoracic spinal cord, and lumbar spinal cord). The results are illustrated in Fig. 25-2. Furthermore, CSF was also analyzed in the same manner.
Fig. 26 illustrates the ratio of the amount of α-synuclein protein at 14 days, 28 days, 42 days, and 56 days after the start of administration, when the amount of α-synuclein protein in CSF before the start of administration was taken as 100. The test samples (ds2 single administration and ds2 double administration) showed a significant decrease in the amount of α-synuclein protein in each region of the brain, spinal cord, and CSF.

### [Example 11]

In Example 11, using male rats (slc:SD rats, 13 weeks of age, manufactured by Japan SLC, Inc.) as subjects, intranasal administration into a cribriform foramen of a cribriform plate was performed, and after survival for 7 days following administration, the transferred amount of the administered substance and the intensity of knockdown in each region of the brain of each specimen were evaluated.

In Example 11, intranasal administration was performed in the same manner as in Example 8. For intranasal administration, each of the oligonucleotides of antisense oligonucleotides (sequences 1346 to 1348) was dissolved in aCSF to a concentration of 60 mg/mL, respectively, and 30 µL thereof was administered.

7 days after administration, each region of the brain was collected, and thereafter cDNA preparation was performed by the same method as in Example 8.

Using this cDNA as a template for PCR reaction, the SNCA gene, SOD1 gene, MALAT1 gene, and, as a control, the ACTB gene were subjected to PCR reaction by the TaqMan (registered trademark) probe method using a QuantStudio 12K Flex real-time PCR system, the amounts of mRNA amplification thereof were measured, respectively, and semiquantitative values of mRNA of each target gene were calculated using the amount of ACTB mRNA amplification as an internal control. Further, the amounts of mRNA amplification of each target gene and ACTB in the negative control group were measured in the same manner, respectively, and semiquantitative values of mRNA of each target gene were calculated.

TaqMan probe Rn01425143_m1 (manufactured by Applied Biosystems) was used for measurement of the SNCA gene, TaqMan probe Rn00566938_m1 (manufactured by Applied Biosystems) was used for measurement of the SOD1 gene, TaqMan MGB probe 3 (SEQ ID NO: 1755) (manufactured by Applied Biosystems), primer 6 (SEQ ID NO: 1753), and primer 7 (SEQ ID NO: 1754) were used for measurement of the MALAT gene, Rn00667869_m1 (manufactured by Applied Biosystems) was used for measurement of the ACTB gene, and TaqMan (registered trademark) Gene Expression Master Mix was used as the reaction reagent, according to the attached protocol. It should be noted that the base sequences of the respective primers and probes are shown in Table 13.

The amount of target mRNA in the nucleic acid-administered individuals was calculated as a relative ratio when the amount of mRNA of each target gene in the negative control group (non-administered group) was taken as 1. The results expressed as mean ± standard error of the relative ratio of the amount of mRNA are illustrated in Fig. 27. The data for each region in Fig. 27 represent, from left to right, the results for negative control (non-administration) and antisense oligonucleotide.

The test samples (SEQ ID NO: 1346 to 1348) showed a significant improvement in knockdown activity against each target gene as compared with the non-administered group.

The present application is based on Japanese Patent Application No. 2024-017851 filed on February 8, 2024, the entire content of which is incorporated herein by reference.

### Reference Signs List

- 1: Administration device
- 10: Needle portion (lumen 10a)
- 11: Needle shaft portion
- 12: Puncture portion (needle tip portion 12a)
- 13: Blade surface
- 14: Distal end opening portion
- 15: Proximal end opening portion
- 20: Hub portion
- 21: Main body portion (lumen 21a)
- 22: Connection portion
- 30: Cannula portion
- 31: Main body portion (lumen 31a)
- 32: Stopper portion (abutment portion 32a)
- 40: Accommodation portion
- 41: Accommodation space
- 42: Liquid feeding portion (distal end opening portion 42a)
- 50: Connection member
- 100: Guide catheter
- 110: Catheter main body
- 111: Lumen
- 200: Administration system
- A: Oligonucleotide
- B: Brain tissue
- C: Cerebrospinal fluid (CSF)
- X1: Ethmoid bone
- X2: Cribriform plate
- X3: Cribriform foramen
- Y1: Olfactory bulb
- Y2: Olfactory mucosa
- Y3: Olfactory epithelium
- Y4: Lamina propria
- Y5: Olfactory nerve
- Z1: Nasal cavity

## Claims

1. An oligonucleotide that is administered intranasally into the brain of a mammal by being injected from within a cribriform foramen of a cribriform plate.

2. The oligonucleotide according to claim 1, wherein
the oligonucleotide is administered intranasally into the brain of a mammal using an administration device including a needle portion having a puncture portion, and
is injected through an opening portion of the puncture portion in a state where the puncture portion is disposed within a cribriform foramen of a cribriform plate.

3. The oligonucleotide according to claim 1, wherein the oligonucleotide is any one selected from the group consisting of an aptamer, an antisense oligonucleotide, a decoy nucleic acid, a ribozyme, siRNA, miRNA, and mRNA.

4. The oligonucleotide according to claim 1, wherein the oligonucleotide is siRNA or an antisense oligonucleotide.

5. The oligonucleotide according to claim 1, wherein the oligonucleotide is siRNA.

6. The oligonucleotide according to claim 5, wherein the proportion of 2'-modified nucleotides among nucleotides constituting the siRNA is 80% or more.

7. The oligonucleotide according to claim 5, wherein all nucleotides constituting the siRNA are 2'-modified nucleotides.

8. The oligonucleotide according to claim 5, wherein the siRNA has one or more lipophilic moieties.

9. The oligonucleotide according to claim 8, wherein the lipophilic moiety is at least one selected from the group consisting of a substituted or unsubstituted alkyl chain having 14 to 24 carbon atoms, eicosapentaenoic acid (EPA), arachidonic acid (ARA), and cholic acid.

10. The oligonucleotide according to claim 5, wherein the target gene of the siRNA is SNCA or HTT.

11. The oligonucleotide according to claim 5, wherein all phosphodiester bonds linking the first and second nucleotides counted from both ends of the sense strand and antisense strand of the siRNA are replaced with phosphorothioate bonds.

12. The oligonucleotide according to claim 5, wherein the siRNA includes any one pair of nucleic acid sequences selected from the group consisting of (S1) to (S11) below:
(S1) SEQ ID NO: 46 and SEQ ID NO: 213;
(S2) SEQ ID NO: 83 and SEQ ID NO: 250;
(S3) SEQ ID NO: 93 and SEQ ID NO: 260;
(S4) SEQ ID NO: 169 and SEQ ID NO: 336;
(S5) SEQ ID NO: 180 and SEQ ID NO: 347;
(S6) SEQ ID NO: 1360 and SEQ ID NO: 1389;
(S7) SEQ ID NO: 1366 and SEQ ID NO: 1395;
(S8) SEQ ID NO: 1368 and SEQ ID NO: 1397;
(S9) SEQ ID NO: 1372 and SEQ ID NO: 1401;
(S10) SEQ ID NO: 1374 and SEQ ID NO: 1403; and
(S11) SEQ ID NO: 1756 and SEQ ID NO: 1759.

13. The oligonucleotide according to claim 5, wherein
the siRNA includes a sequence of any one pair of oligonucleotides selected from the group consisting of ds2, ds9, ds17 to ds249, and ds254 to ds273 described in any of Tables 1-1 to 1-6,
note that, in the Tables, "[L1]" represents a compound represented by Formula (1) below, "^" represents a phosphorothioate bond, "mA", "mU", "mC", and "mG" represent 2'-O-methyl-RNA, "fA", "fU", "fC", and "fG" represent 2'-fluoro-DNA, "dA", "dT", "dC", and "dG" represent DNA, " (vmU) " represents 5'-vinylphosphate-2'-O-methyl-uridine, and "p" represents 5'-phosphate.

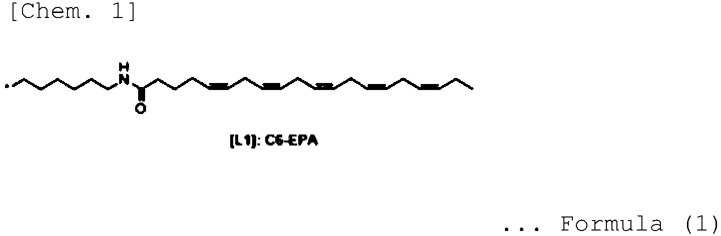

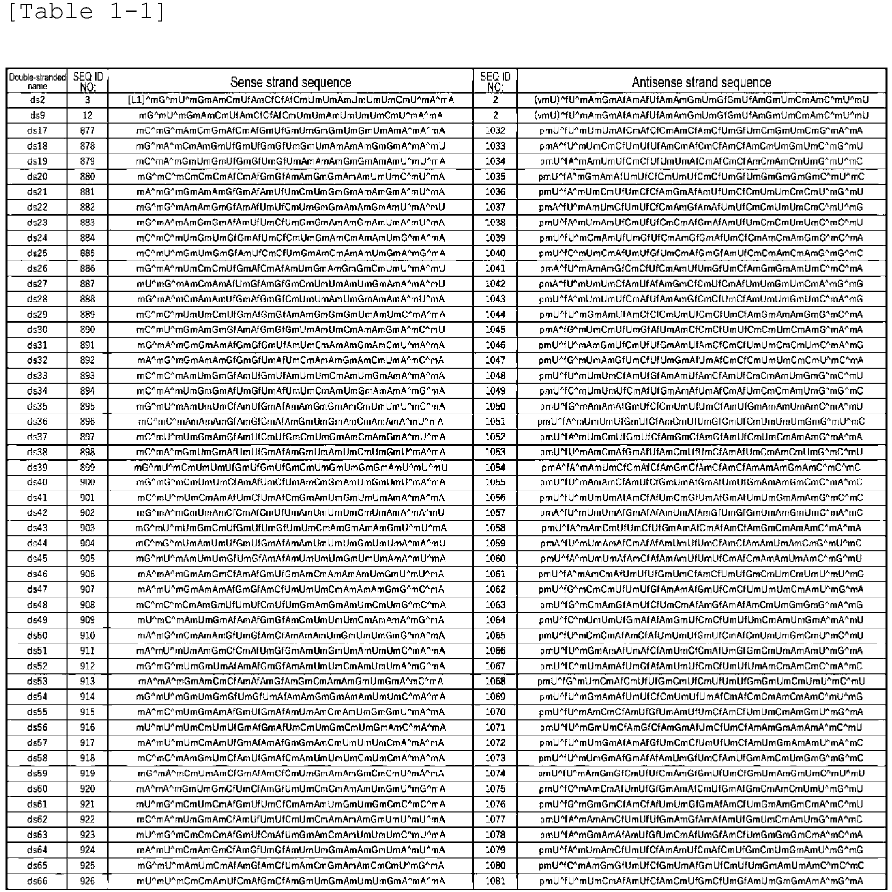

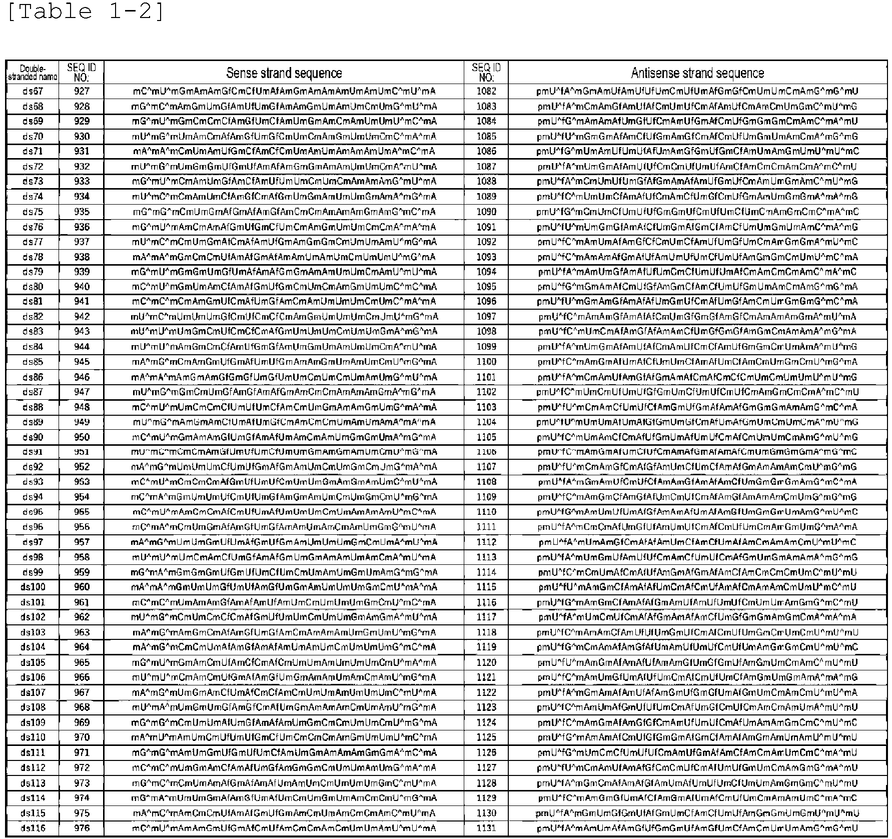

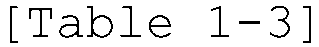

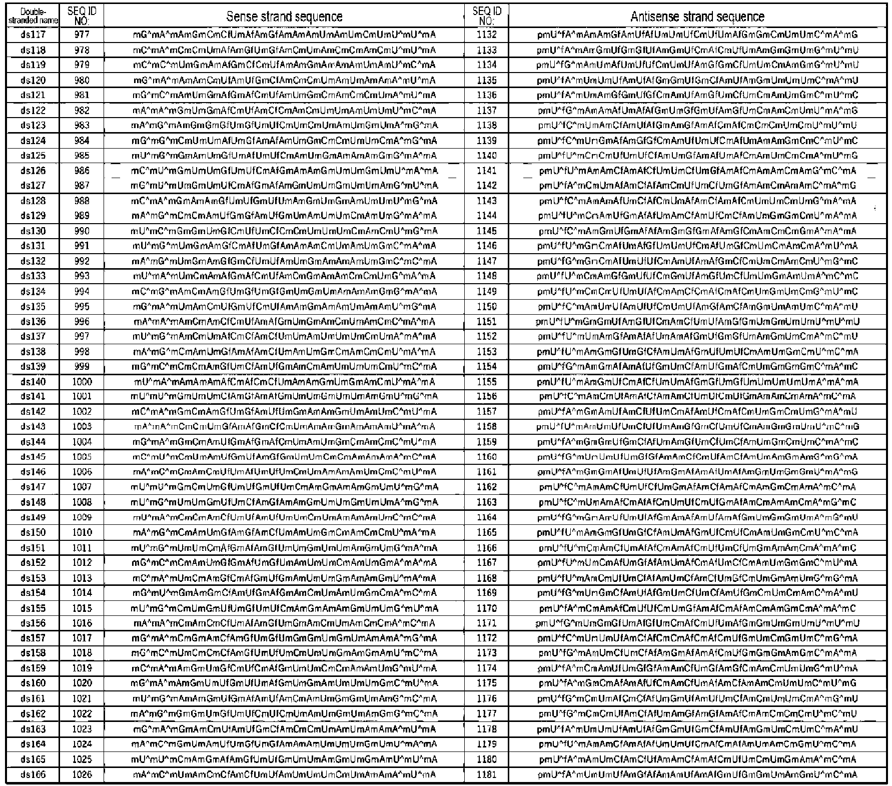

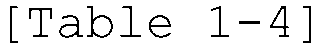

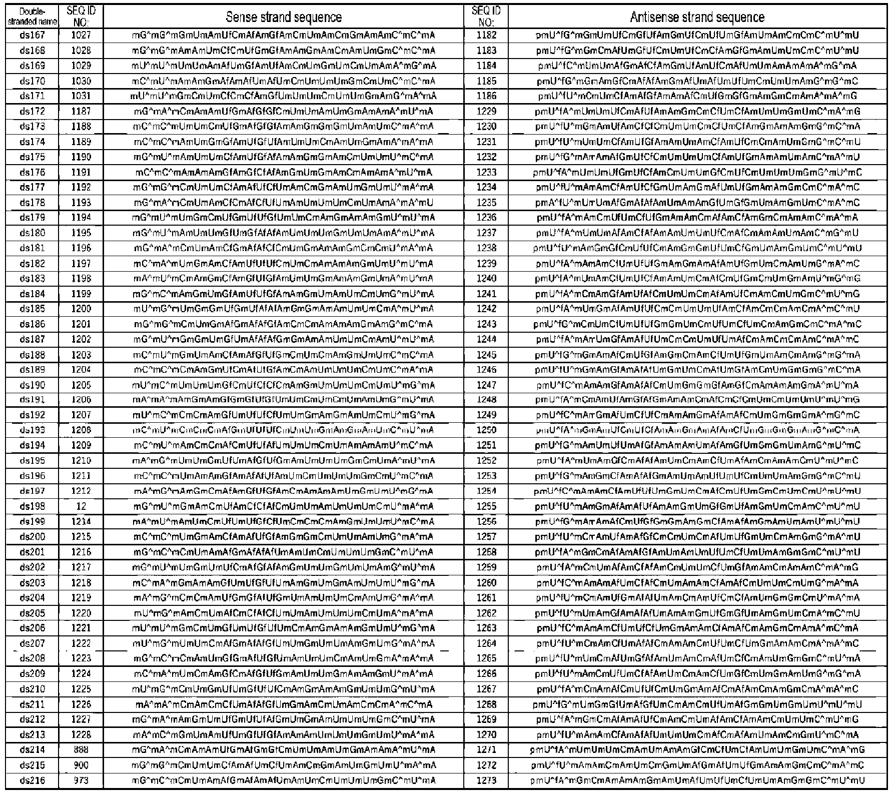

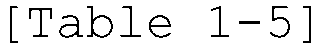

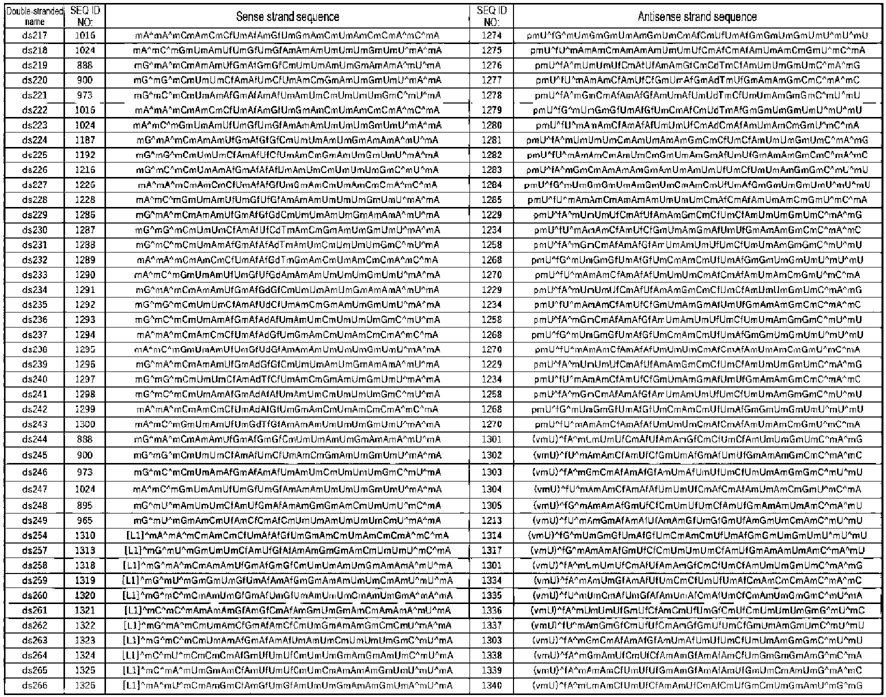

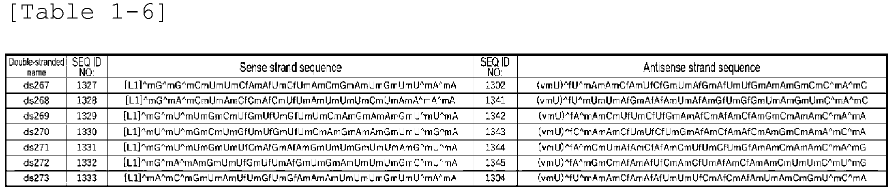

14. The oligonucleotide according to claim 13, wherein the siRNA includes a sequence of any one pair of oligonucleotides selected from the group consisting of the ds9, the ds28, the ds35, the ds36, the ds40, the ds42, the ds79, the ds105, the ds113, the ds156, the ds164, the ds172, the ds175, the ds176, the ds177, the ds178, the ds187, the ds198, the ds201, the ds211, and the ds213 to the ds246 described in any of the Tables 1-1 to 1-6.

15. The oligonucleotide according to claim 13, wherein the siRNA is any one pair of oligonucleotides selected from the group consisting of the ds2, the ds254, the ds257, the ds258, the ds259, the ds261, the ds263, the ds267, the ds268, and the ds273 described in any of Tables 1-1 to 1-6.

16. The oligonucleotide according to claim 5, wherein the siRNA includes a sequence of any one pair of oligonucleotides selected from the group consisting of ds274 to ds461 described in any of Tables 2-1 to 2-4,
note that, in the Tables, "^" represents a phosphorothioate bond, "mA", "mU", "mC", and "mG" represent 2'-O-methyl-RNA, "fA", "fU", "fC", and "fG" represent 2'-fluoro-DNA, "dA", "dT", "dC", and "dG" represent DNA, and "p" represents 5'-phosphate.

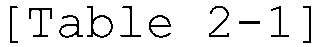

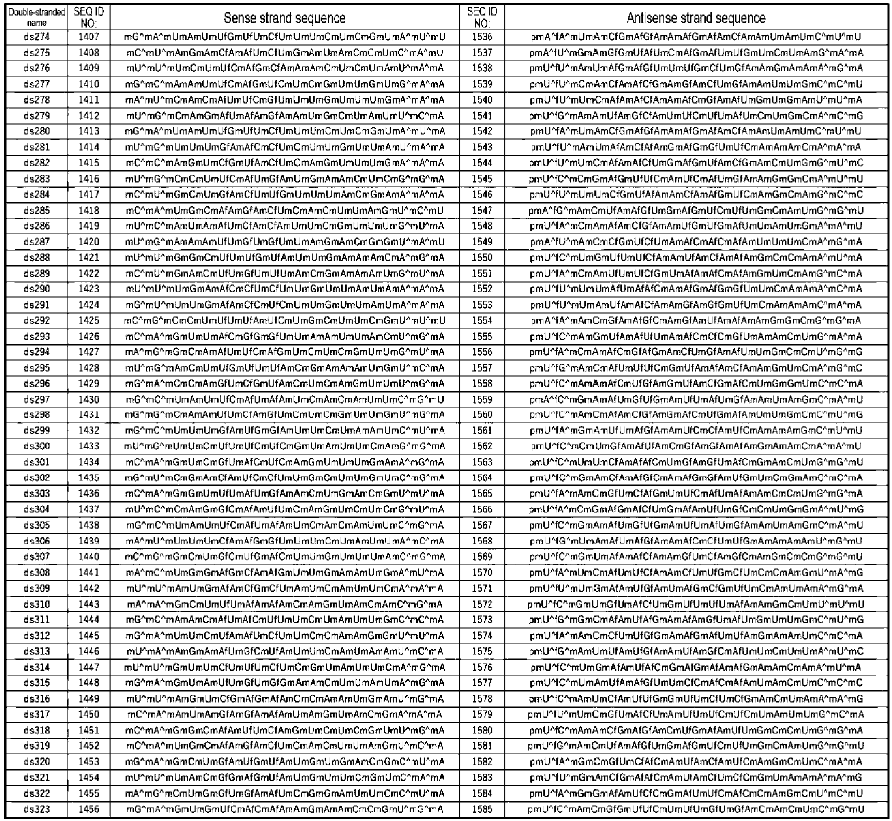

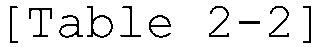

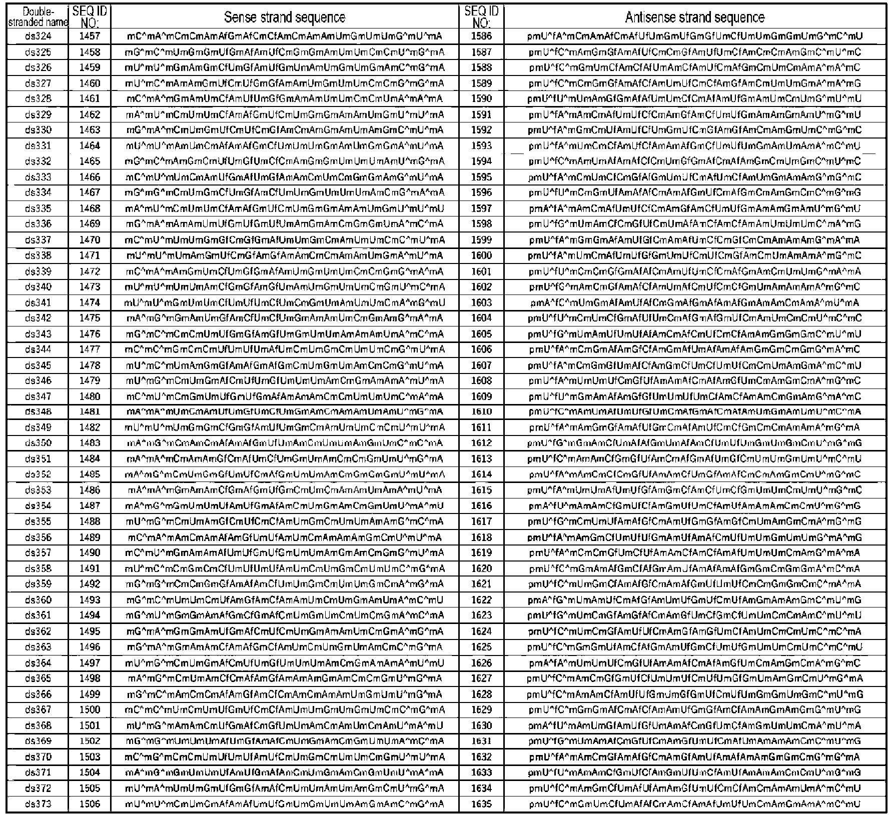

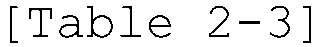

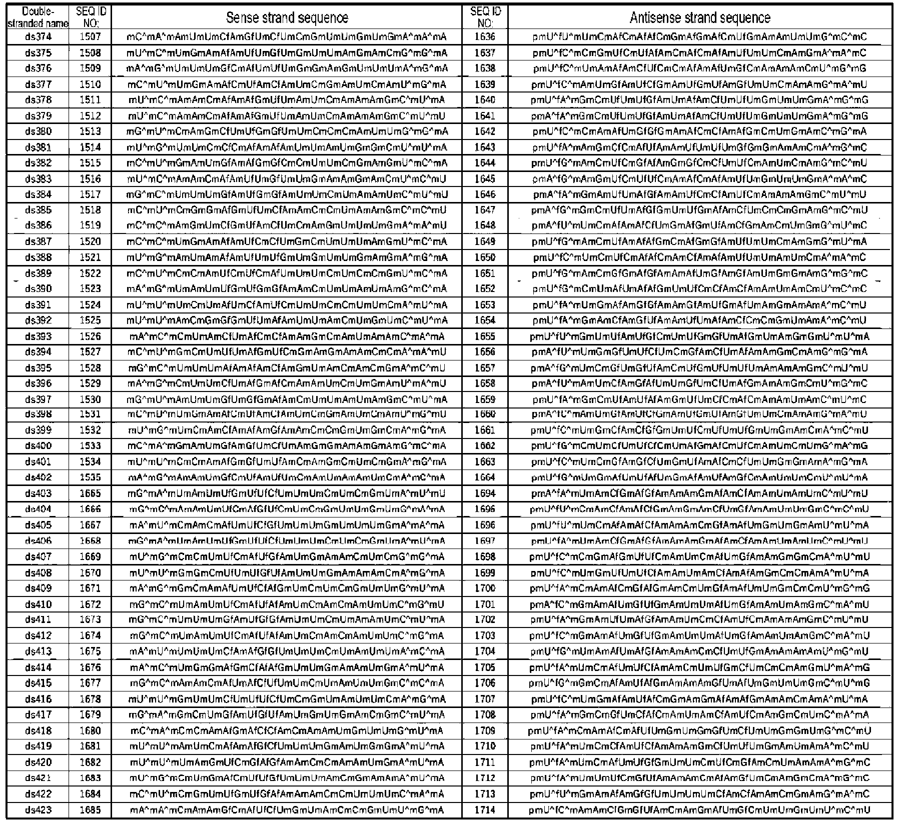

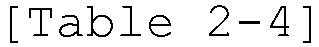

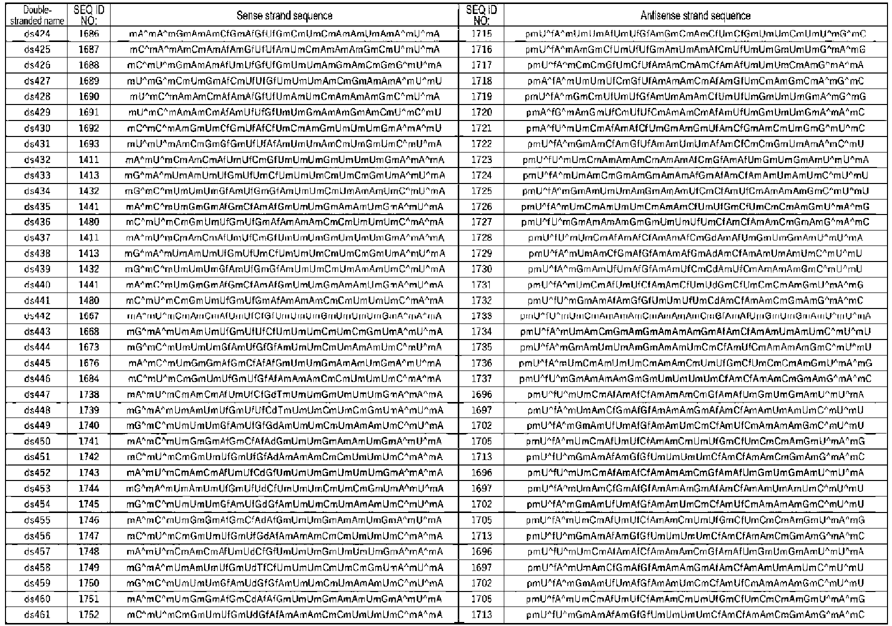

17. The oligonucleotide according to claim 16, wherein the oligonucleotide includes a sequence of any one pair of oligonucleotides selected from the group consisting of the ds278, the ds280, the ds299, the ds308, the ds347, the ds405, the ds406, the ds411, the ds414, the ds422, and the ds432 to ds461 described in any of the Tables 2-1 to 2-4.

18. A pharmaceutical composition comprising the oligonucleotide according to any one of claims 1 to 17.

19. A therapeutic agent for a central nervous system disease, comprising the oligonucleotide according to any one of claims 1 to 17.

20. The oligonucleotide according to claim 2, wherein
the administration device has a cannula portion that is formed of a tubular member and is disposed to cover the needle portion such that the puncture portion is exposed,
the opening portion is provided on a distal end side of the puncture portion,
a stopper portion having an abutment portion that abuts against an olfactory epithelium of an olfactory mucosa is formed at a distal end portion of the cannula portion, and
the opening portion is disposed within the cribriform plate in a state where the abutment portion of the stopper portion is brought into contact with the olfactory epithelium.

21. The oligonucleotide according to claim 20, wherein
the administration device has a hub portion to which an accommodation portion accommodating the oligonucleotide is attachable, and
the hub portion holds a proximal end of the cannula portion and/or a proximal end of a needle shaft portion of the needle portion inserted through a lumen of the cannula portion.

22. The oligonucleotide according to claim 20, wherein a cross-sectional shape of the abutment portion of the stopper portion is circular or elliptical.

23. An administration system comprising an accommodation portion accommodating the oligonucleotide according to any one of claims 1 to 17 and an administration device including a needle portion having a puncture portion, wherein the administration system is configured such that the oligonucleotide accommodated in the accommodation portion is dischargeable through an opening portion of the puncture portion.

24. The administration system according to claim 23, wherein
the administration device has a cannula portion that is formed of a tubular member and is disposed to cover the needle portion such that the puncture portion is exposed,
the opening portion is provided on a distal end side of the puncture portion,
a stopper portion having an abutment portion that abuts against an olfactory epithelium of an olfactory mucosa is formed at a distal end portion of the cannula portion, and
the opening portion is disposed within the cribriform plate in a state where the abutment portion of the stopper portion is brought into contact with the olfactory epithelium.

25. The administration system according to claim 24, wherein
the administration device has a hub portion to which an accommodation portion accommodating the oligonucleotide is attachable, and
the hub portion holds a proximal end of the cannula portion and/or a proximal end of a needle shaft portion of the needle portion inserted through a lumen of the cannula portion.

26. The administration system according to claim 24,
wherein a cross-sectional shape of the abutment portion of the stopper portion is circular or elliptical.
